(19) Europäisches Patentamt / European Patent Office / Office européen des brevets

(11) EP 4 624 475 A1

(12) EUROPEAN PATENT APPLICATION
published in accordance with Art. 153(4) EPC

(43) Date of publication:
01.10.2025 Bulletin 2025/40

(21) Application number: 23893998.7

(22) Date of filing: 24.11.2023

(51) International Patent Classification (IPC):
*C07D 519/00* (2006.01) *A61K 31/444* (2006.01)
*A61P 35/00* (2006.01)

(52) Cooperative Patent Classification (CPC):
A61K 31/444; A61P 35/00; C07D 519/00

(86) International application number:
PCT/CN2023/133860

(87) International publication number:
WO 2024/109909 (30.05.2024 Gazette 2024/22)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 25.11.2022 CN 202211494114

(71) Applicant: Applied Pharmaceutical Science, Inc.
Beijing 100176 (CN)

(72) Inventors:
- ZHONG, Jun
Beijing 100176 (CN)
- GAO, Qin
Beijing 100176 (CN)
- LIU, Yongbo
Beijing 100176 (CN)
- WANG, Hao
Beijing 100176 (CN)
- CHEN, Xiaohu
Beijing 100176 (CN)

(74) Representative: Gille Hrabal
Partnerschaftsgesellschaft mbB
Patentanwälte
Brucknerstraße 20
40593 Düsseldorf (DE)

(54) **SOLID FORM OF FUSED RING COMPOUND, AND PREPARATION METHOD THEREFOR AND USE THEREOF**

(57) The present disclosure relates to a solid form of a fused ring compound, and a preparation method therefor and a use thereof. A more advantageous pharmaceutically acceptable form is provided for a compound of formula (I). The solid form provided by the present disclosure has advantageous physicochemical and pharmacokinetic properties.

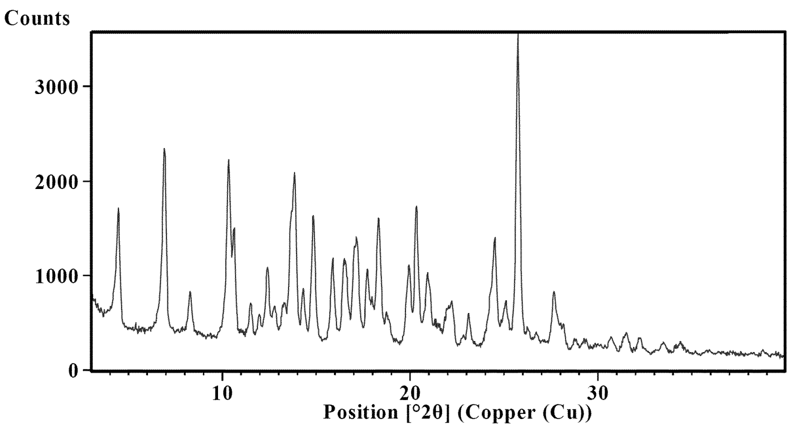

FIG. 1A



Processed by Luminess, 75001 PARIS (FR)

## Description

The present application claims priority to the prior Chinese Patent Application for Invention with the application No. 202211494114.7 and entitled "SOLID FORM OF FUSED RING COMPOUND, AND PREPARATION METHOD THEREFOR AND USE THEREOF" filed with the China National Intellectual Property Administration on November 25, 2022, which is incorporated herein by reference in its entirety.

TECHNICAL FIELD

The present disclosure relates to a solid form of a fused ring compound, a preparation method therefor, and use thereof, and pertains to the field of chemical medicine.

BACKGROUND

RET (rearranged during transfection) proto-oncogene was first demonstrated in 1985 by transfection of NIH3T3 cells (mouse embryonic fibroblast cell line) with human lymphoma DNA (Cell, 1985, 42(2): 581-588). RET proto-oncogene, located on chromosome 10q11.2, has a DNA of 60 kb in length, contains 21 exons, and encodes an RET protein consisting of 1100 amino acids. The RET protein is a tyrosine kinase receptor containing an extracellular region composed of cysteine, a transmembrane region, and an intracellular region capable of catalyzing tyrosine kinase (Mol Cell Endocrinol, 2010, 322(1-2): 2-7). RET is involved in cell proliferation, nerve conduction, cell migration, and cell differentiation, and activates various downstream pathways, such as RAS/RAF/MEK/ERK, PI3K/AKT, and STAT pathways, through the signal from a ligand/complex receptor/RET multi-protein complex to induce cell proliferation (J Clin Oncol, 2012, 30(2): 200-202).

The discovery that RET fusions are drivers in some cancers facilitates the use of multi-kinase inhibitors, which have RET inhibitory activity, in the treatment of tumor patients loaded with RET fusion proteins. The RET inhibitor selpercatinib (LOXO-292) has been approved for the treatment of tumor patients loaded with RET fusion proteins. However, one of the biggest challenges in cancer treatment is that tumor cells may become resistant to treatment after a certain duration. Generally, such resistance greatly limits the treatment options for patients, and in most cases, the cancer remains progressive and uncontrolled. It has been reported in the document (RET Solvent Front Mutations Mediate Acquired Resistance to Selective RET Inhibition in RET-Driven Malignancies, Journal of Thoracic Oncology, 2020, Vol. 15, No 4, 541-549) that non-small cell lung cancer patients with RET fusions exhibited RET G810 solvent-front mutations, such as G810R, G810S, and G801C mutations, after using the RET inhibitor selpercatinib (LOXO-292), resulting in a decrease in the binding of LOXO-292 to the ATP binding site, thereby causing drug resistance and cancer progression. Therefore, there is a need to develop compounds with good RET mutation inhibitory activity.

The PCT patent application with the application No. PCT/CN2020/107049 discloses a class of compounds as RET inhibitors, which have relatively strong inhibitory effects on the RET gatekeeper residue mutant RET V804M, RET solvent-front residue mutant G810R, and other clinically relevant RET mutants, as well as wt-RET. These compounds can also significantly inhibit the growth of TT cell lines derived from thyroid cancer and Ba/F3 cells derived from various RET mutants, block the RET autophosphorylation and downstream pathways in cells, and significantly induce the death of TT cells. Therefore, further research is needed into solid forms of such compounds, especially the development of crystal forms that are more suitable as active pharmaceutical ingredients.

SUMMARY

In order to achieve the object described above, the present disclosure provides a solid form of a compound of formula (I) below or a pharmaceutically acceptable salt thereof:

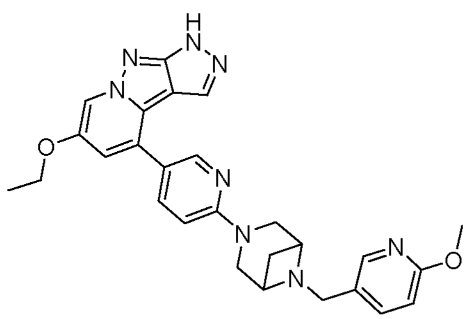

(I)

The solid form is selected from solid forms of an anhydrate, a hydrate, an organic solvate, and a co-solvate of water and an organic solvent.

According to an embodiment of the present disclosure, the organic solvent is selected from one of alcohols (such as methanol and ethanol), nitriles (such as acetonitrile), haloalkanes (such as dichloromethane), and ethers (such as 1,4-dioxane).

According to an embodiment of the present disclosure, the solid form is selected from one, two, three, or more of the crystal forms described below.

The present disclosure further provides a crystal form A of the compound of formula (I), wherein an X-ray powder diffraction pattern of the crystal form A has one or two characteristic peaks at 2θ values selected from the following: 6.91°±0.20° and 10.32°±0.20°.

According to an embodiment of the present disclosure, the X-ray powder diffraction pattern of the crystal form A has one, two, or all characteristic peaks at 2θ values selected from the following: 6.91°±0.20°, 10.32°±0.20°, and 25.72°±0.20°.

Preferably, the X-ray powder diffraction pattern of the crystal form A has one, two, more, or all characteristic peaks at 2θ values selected from the following: 6.91°±0.20°, 10.32°±0.20°, 13.88°±0.20°, and 25.72°±0.20°.

According to an embodiment of the present disclosure, the X-ray powder diffraction pattern of the crystal form A has one, two, more, or all characteristic peaks at 2θ values selected from the following: 4.46°±0.20°, 6.91°±0.20°, 10.32°±0.20°, 13.88°±0.20°, 14.84°±0.20°, 18.33°±0.20°, 20.32°±0.20°, and 25.72°±0.20°.

According to an embodiment of the present disclosure, the X-ray powder diffraction pattern of the crystal form A has one, two, more, or all characteristic peaks at 2θ values selected from the following: 4.46°±0.20°, 6.91°±0.20°, 10.32°±0.20°, 10.63°±0.20°, 13.61°±0.20°, 13.88°±0.20°, 14.84°±0.20°, 18.33°±0.20°, 20.32°±0.20°, and 25.72°±0.20°.

According to an embodiment of the present disclosure, the X-ray powder diffraction pattern of the crystal form A further has one, two, more, or all characteristic peaks at 2θ values selected from the following: 15.86°±0.20°, 17.00°±0.20°, 17.18°±0.20°, and 24.51°±0.20°.

According to an embodiment of the present disclosure, the X-ray powder diffraction pattern of the crystal form A has one, two, more, or all characteristic peaks at 2θ values selected from the following:

| 2θ (°±0.20°) | 2θ (°±0.20°) |
|---|---|
| 4.46 | 18.33 |
| 6.91 | 18.80 |
| 8.28 | 19.95 |
| 10.32 | 20.32 |
| 10.63 | 20.91 |
| 11.51 | 22.25 |
| 12.40 | 23.10 |
| 13.24 | 24.51 |
| 13.61 | 25.08 |
| 13.88 | 25.72 |
| 14.30 | 27.65 |
| 14.84 | 28.79 |
| 15.86 | 30.68 |
| 16.44 | 31.51 |
| 17.00 | 32.22 |
| 17.18 | 33.49 |
| 17.70 | 34.34 |

According to an embodiment of the present disclosure, when the X-ray powder diffraction pattern of the crystal form A has one, two, more, or all characteristic peaks at 2θ values selected from the following, the crystal form A optionally further has relative intensities selected from those corresponding to the characteristic peaks.

| 2θ (°±0.20°) | Relative intensity (%) | 2θ (°±0.20°) | Relative intensity (%) |
|---|---|---|---|
| 4.46 | 46.10 | 18.33 | 42.83 |
| 6.91 | 64.70 | 18.80 | 11.57 |
| 8.28 | 19.59 | 19.95 | 28.12 |
| 10.32 | 62.05 | 20.32 | 47.34 |
| 10.63 | 40.28 | 20.91 | 25.51 |
| 11.51 | 16.14 | 22.25 | 15.67 |
| 12.40 | 27.64 | 23.10 | 12.70 |
| 13.24 | 15.67 | 24.51 | 37.40 |
| 13.61 | 40.01 | 25.08 | 16.32 |
| 13.88 | 56.36 | 25.72 | 100.00 |
| 14.30 | 20.88 | 27.65 | 19.74 |
| 14.84 | 44.19 | 28.79 | 4.28 |
| 15.86 | 30.08 | 30.68 | 5.08 |
| 16.44 | 28.52 | 31.51 | 6.29 |
| 17.00 | 30.69 | 32.22 | 4.79 |
| 17.18 | 35.62 | 33.49 | 3.33 |
| 17.70 | 26.86 | 34.34 | 2.90 |

According to an embodiment of the present disclosure, the crystal form A has an X-ray powder diffraction pattern substantially as shown in FIG. 1A.

According to an embodiment of the present disclosure, a differential scanning calorimetry (DSC) profile of the crystal form A has an endothermic peak at an onset temperature of about 175.03 °C and/or a peak temperature of about 180.61 °C.

According to an embodiment of the present disclosure, the crystal form A has a differential scanning calorimetry (DSC) profile substantially as shown in FIG. 1B.

According to an embodiment of the present disclosure, thermogravimetric analysis (TGA) of the crystal form A shows a weight loss of about 0.45% to about 0.55% (e.g., about 0.499%) upon heating from room temperature to 190 °C.

According to an embodiment of the present disclosure, the crystal form A has a thermogravimetric analysis (TGA) profile substantially as shown in FIG. 1C.

According to an embodiment of the present disclosure, the crystal form A is an anhydrous crystal form.

According to an embodiment of the present disclosure, the crystal form A has a $^1$H NMR spectrum substantially as shown in FIG. 1D.

The present disclosure further provides a crystal form B of the compound of formula (I), wherein an X-ray powder diffraction pattern of the crystal form B has one, two, or all characteristic peaks at 2θ values selected from the following: 18.19°±0.20°, 25.74°±0.20°, and 26.95°±0.20°.

According to an embodiment of the present disclosure, the X-ray powder diffraction pattern of the crystal form B has one, two, more, or all characteristic peaks at 2θ values selected from the following: 11.04°±0.20°, 12.78°±0.20°, 16.12°±0.20°, 16.74°±0.20°, 18.19°±0.20°, 25.22°±0.20°, 25.74°±0.20°, and 26.95°±0.20°.

According to an embodiment of the present disclosure, the X-ray powder diffraction pattern of the crystal form B has one, two, more, or all characteristic peaks at 2θ values selected from the following: 4.26°±0.20°, 11.04°±0.20°, 12.78°±0.20°, 16.12°±0.20°, 16.74°±0.20°, 18.19°±0.20°, 19.96°±0.20°, 24.33°±0.20°, 25.22°±0.20°, 25.74°±0.20°, and 26.95°±0.20°.

According to an embodiment of the present disclosure, the X-ray powder diffraction pattern of the crystal form B has one, two, more, or all characteristic peaks at 2θ values selected from the following:

| 2θ (±0.20°) | Relative intensity (%) | 2θ (°±0.20°) | Relative intensity (%) |
|---|---|---|---|
| 4.26 | 11.84 | 17.82 | 7.35 |
| 6.81 | 5.23 | 18.19 | 19.85 |

(continued)

| 2θ (±0.20°) | Relative intensity (%) | 2θ (°±0.20°) | Relative intensity (%) |
|---|---|---|---|
| 7.87 | 9.12 | 18.66 | 6.67 |
| 9.93 | 5.91 | 19.59 | 6.85 |
| 10.63 | 7.60 | 19.96 | 11.54 |
| 11.04 | 12.13 | 21.50 | 6.62 |
| 12.78 | 15.79 | 22.65 | 8.40 |
| 13.56 | 8.20 | 23.21 | 6.61 |
| 14.30 | 6.16 | 24.33 | 11.21 |
| 14.72 | 8.84 | 25.22 | 14.89 |
| 16.12 | 12.47 | 25.74 | 100.00 |
| 16.74 | 12.62 | 26.95 | 20.78 |
| 16.94 | 6.83 | | |

According to an embodiment of the present disclosure, when the X-ray powder diffraction pattern of the crystal form B has one, two, more, or all characteristic peaks at 2θ values selected from those described above, the crystal form B optionally further has relative intensities corresponding to the characteristic peaks.

According to an embodiment of the present disclosure, the crystal form B has an X-ray powder diffraction pattern substantially as shown in FIG. 2A.

According to an embodiment of the present disclosure, a differential scanning calorimetry (DSC) profile of the crystal form B has an endothermic peak at an onset temperature of about 186.7 °C and/or a peak temperature of about 189.8 °C.

According to an embodiment of the present disclosure, the crystal form B has a differential scanning calorimetry (DSC) profile substantially as shown in FIG. 2B.

According to an embodiment of the present disclosure, thermogravimetric analysis (TGA) of the crystal form B shows a weight loss of about 0.5% to about 0.6% (e.g., about 0.54%) upon heating from room temperature to 150 °C.

According to an embodiment of the present disclosure, the crystal form B has a thermogravimetric analysis (TGA) profile substantially as shown in FIG. 2B.

According to an embodiment of the present disclosure, the crystal form B is an anhydrous crystal form.

According to an embodiment of the present disclosure, the crystal form B has a $^1$H NMR spectrum substantially as shown in FIG. 2C.

The present disclosure further provides a crystal form C of the compound of formula (I), wherein an X-ray powder diffraction pattern of the crystal form C has one, two, or all characteristic peaks at 2θ values selected from the following: 7.25°±0.20°, 16.53°±0.20°, and 18.84°±0.20°.

According to an embodiment of the present disclosure, the X-ray powder diffraction pattern of the crystal form C has one, two, more, or all characteristic peaks at 2θ values selected from the following: 7.25°±0.20°, 14.48°±0.20°, 16.53°±0.20°, and 18.84°±0.20°.

According to an embodiment of the present disclosure, the X-ray powder diffraction pattern of the crystal form C has one, two, more, or all characteristic peaks at 2θ values selected from the following: 7.25°±0.20°, 12.54°±0.20°, 13.04°±0.20°, 14.48°±0.20°, 16.53°±0.20°, 18.84°±0.20°, 19.18°±0.20°, and 25.02°±0.20°.

According to an embodiment of the present disclosure, the X-ray powder diffraction pattern of the crystal form C has one, two, more, or all characteristic peaks at 2θ values selected from the following:

| 2θ (°±0.20°) | Relative intensity (%) | 2θ (°±0.20°) | Relative intensity (%) |
|---|---|---|---|
| 6.28 | 5.65 | 18.32 | 16.19 |
| 7.25 | 100.00 | 18.84 | 33.82 |
| 12.54 | 21.85 | 19.18 | 23.06 |
| 13.04 | 24.98 | 20.28 | 7.63 |
| 14.48 | 30.59 | 21.84 | 8.66 |

(continued)

| 2θ (°±0.20°) | Relative intensity (%) | 2θ (°±0.20°) | Relative intensity (%) |
|---|---|---|---|
| 14.94 | 11.57 | 23.86 | 6.21 |
| 16.53 | 39.55 | 25.02 | 24.96 |
| 17.87 | 13.40 | 26.98 | 9.95 |

According to an embodiment of the present disclosure, when the X-ray powder diffraction pattern of the crystal form C has one, two, more, or all characteristic peaks at 2θ values selected from those described above, the crystal form C optionally further has relative intensities corresponding to the characteristic peaks.

According to an embodiment of the present disclosure, the crystal form C has an X-ray powder diffraction pattern substantially as shown in FIG. 3A.

According to an embodiment of the present disclosure, a differential scanning calorimetry (DSC) profile of the crystal form C has endothermic peaks at a peak temperature of about 109.1 °C, as well as an onset temperature of about 123.4 °C and/or a peak temperature of about 132.5 °C.

According to an embodiment of the present disclosure, the crystal form C has a differential scanning calorimetry (DSC) profile substantially as shown in FIG. 3B.

According to an embodiment of the present disclosure, thermogravimetric analysis (TGA) of the crystal form C shows a weight loss of about 0.2% to about 0.3% (e.g., about 0.27%) upon heating from room temperature to 200 °C.

According to an embodiment of the present disclosure, the crystal form C has a thermogravimetric analysis (TGA) profile substantially as shown in FIG. 3B.

According to an embodiment of the present disclosure, the crystal form C is an anhydrous crystal form.

According to an embodiment of the present disclosure, the crystal form C has a $^{1}H$ NMR spectrum substantially as shown in FIG. 3C.

The present disclosure further provides a crystal form D of the compound of formula (I), wherein an X-ray powder diffraction pattern of the crystal form D has one, two, or all characteristic peaks at 2θ values selected from the following: 11.01°±0.20°, 25.01°±0.20°, and 26.42°±0.20°.

According to an embodiment of the present disclosure, the X-ray powder diffraction pattern of the crystal form D has one, two, more, or all characteristic peaks at 2θ values selected from the following: 4.26°±0.20°, 11.01°±0.20°, 15.61°±0.20°, 18.53°±0.20°, 22.14°±0.20°, 25.01°±0.20°, and 26.42°±0.20°.

According to an embodiment of the present disclosure, the X-ray powder diffraction pattern of the crystal form D has one, two, more, or all characteristic peaks at 2θ values selected from the following: 4.26°±0.20°, 9.01°±0.20°, 11.01°±0.20°, 15.61°±0.20°, 18.53°±0.20°, 22.14°±0.20°, 25.01°±0.20°, and 26.42°±0.20°.

According to an embodiment of the present disclosure, the X-ray powder diffraction pattern of the crystal form D has one, two, more, or all characteristic peaks at 2θ values selected from the following:

| 2θ (±0.20°) | Relative intensity (%) | 2θ (°±0.20°) | Relative intensity (%) |
|---|---|---|---|
| 4.26 | 54.79 | 18.53 | 47.38 |
| 9.01 | 35.44 | 19.56 | 31.98 |
| 9.21 | 32.59 | 20.02 | 32.53 |
| 11.01 | 70.99 | 20.88 | 27.96 |
| 13.37 | 24.29 | 21.22 | 25.10 |
| 15.61 | 42.44 | 22.14 | 44.28 |
| 17.00 | 18.01 | 25.01 | 100.00 |
| 18.03 | 13.52 | 26.42 | 97.56 |

According to an embodiment of the present disclosure, when the X-ray powder diffraction pattern of the crystal form D has one, two, more, or all characteristic peaks at 2θ values selected from those described above, the crystal form D optionally further has relative intensities corresponding to the characteristic peaks.

According to an embodiment of the present disclosure, the crystal form D has an X-ray powder diffraction pattern substantially as shown in FIG. 4A.

According to an embodiment of the present disclosure, a differential scanning calorimetry (DSC) profile of the

crystal form D has endothermic peaks at a peak temperature of about 63.7 °C, as well as an onset temperature of about 181.7 °C and/or a peak temperature of about 184.3 °C.

According to an embodiment of the present disclosure, the crystal form D has a differential scanning calorimetry (DSC) profile substantially as shown in FIG. 4B.

According to an embodiment of the present disclosure, thermogravimetric analysis (TGA) of the crystal form D shows a weight loss of about 3.3% to about 3.4% (e.g., about 3.35%) upon heating from room temperature to 200 °C.

According to an embodiment of the present disclosure, the crystal form D has a thermogravimetric analysis (TGA) profile substantially as shown in FIG. 4B.

According to an embodiment of the present disclosure, the crystal form D is a hydrate.

According to an embodiment of the present disclosure, the crystal form D has a $^1H$ NMR spectrum substantially as shown in FIG. 4C.

According to an embodiment of the present disclosure, the crystal form D has a variable-temperature XRPD pattern substantially as shown in FIG. 4D.

The present disclosure further provides a crystal form E of the compound of formula (I), wherein an X-ray powder diffraction pattern of the crystal form E has one, two, or all characteristic peaks at 2θ values selected from the following: 5.98°±0.20°, 12.73°±0.20°, and 14.53°±0.20°.

According to an embodiment of the present disclosure, the X-ray powder diffraction pattern of the crystal form E has one, two, more, or all characteristic peaks at 2θ values selected from the following: 5.98°±0.20°, 10.66°±0.20°, 11.95°±0.20°, 12.73°±0.20°, 14.53°±0.20°, 17.67°±0.20°, and 22.91°±0.20°.

According to an embodiment of the present disclosure, the X-ray powder diffraction pattern of the crystal form E has one, two, more, or all characteristic peaks at 2θ values selected from the following:

| 2θ (°±0.20°) | Relative intensity (%) | 2θ (°±0.20°) | Relative intensity (%) |
|---|---|---|---|
| 5.98 | 100.00 | 17.67 | 14.92 |
| 10.66 | 12.05 | 20.03 | 5.80 |
| 11.95 | 24.23 | 20.82 | 5.18 |
| 12.73 | 26.01 | 21.56 | 9.91 |
| 14.53 | 25.99 | 22.91 | 10.11 |
| 16.73 | 8.10 | | |

According to an embodiment of the present disclosure, when the X-ray powder diffraction pattern of the crystal form E has one, two, more, or all characteristic peaks at 2θ values selected from those described above, the crystal form E optionally further has relative intensities corresponding to the characteristic peaks.

According to an embodiment of the present disclosure, the crystal form E has an X-ray powder diffraction pattern substantially as shown in FIG. 5A.

According to an embodiment of the present disclosure, a differential scanning calorimetry (DSC) profile of the crystal form E has endothermic peaks at a peak temperature of about 114.9 °C and a peak temperature of about 127.9 °C.

According to an embodiment of the present disclosure, the crystal form E has a differential scanning calorimetry (DSC) profile substantially as shown in FIG. 5B.

According to an embodiment of the present disclosure, thermogravimetric analysis (TGA) of the crystal form E shows a weight loss of about 1.9% to about 2.0% (e.g., about 1.95%) upon heating from room temperature to 50 °C, and a weight loss of about 3.0% to about 3.1% (e.g., about 3.02%) upon heating from 50 °C to 150 °C.

According to an embodiment of the present disclosure, the crystal form E has a thermogravimetric analysis (TGA) profile substantially as shown in FIG. 5B.

According to an embodiment of the present disclosure, the crystal form E is a methanol solvate.

According to an embodiment of the present disclosure, the crystal form E has a $^1H$ NMR spectrum substantially as shown in FIG. 5C.

According to an embodiment of the present disclosure, the crystal form E has a variable-temperature XRPD pattern substantially as shown in FIG. 5D.

The present disclosure further provides a crystal form F of the compound of formula (I), wherein an X-ray powder diffraction pattern of the crystal form F has one, two, or all characteristic peaks at 2θ values selected from the following: 11.07°±0.20°, 24.79°±0.20°, and 26.42°±0.20°.

According to an embodiment of the present disclosure, the X-ray powder diffraction pattern of the crystal form F has one, two, or all characteristic peaks at 2θ values selected from the following: 11.07°±0.20°, 17.83°±0.20°,

22.44°±0.20°, 24.79°±0.20°, and 26.42°±0.20°. Preferably, the X-ray powder diffraction pattern of the crystal form F has one, two, or all characteristic peaks at 2θ values selected from the following: 11.07°±0.20°, 17.83°±0.20°, 22.44°±0.20°, 24.79°±0.20°, and 26.42°±0.20°.

According to an embodiment of the present disclosure, the X-ray powder diffraction pattern of the crystal form F has one, two, or all characteristic peaks at 2θ values selected from the following: 11.07°±0.20°, 14.99°±0.20°, 17.83°±0.20°, 19.93°±0.20°, 20.64°±0.20°, 22.44°±0.20°, 24.79°±0.20°, and 26.42°±0.20°.

According to an embodiment of the present disclosure, the X-ray powder diffraction pattern of the crystal form F has one, two, or all characteristic peaks at 2θ values selected from the following:

| 2θ (°±0.20°) | Relative intensity (%) | 2θ (°±0.20°) | Relative intensity (%) |
|---|---|---|---|
| 4.31 | 5.14 | 18.47 | 23.64 |
| 8.85 | 17.25 | 18.97 | 12.19 |
| 9.32 | 13.12 | 19.31 | 20.59 |
| 10.16 | 5.36 | 19.93 | 26.78 |
| 11.07 | 44.18 | 20.64 | 24.94 |
| 12.32 | 11.28 | 21.10 | 10.52 |
| 12.89 | 6.48 | 21.54 | 7.13 |
| 13.25 | 11.77 | 22.44 | 34.23 |
| 14.26 | 20.06 | 23.60 | 7.72 |
| 14.99 | 25.31 | 24.79 | 100.00 |
| 15.74 | 21.04 | 25.24 | 6.60 |
| 16.07 | 11.10 | 26.42 | 65.83 |
| 17.22 | 17.53 | 27.45 | 6.87 |
| 17.83 | 43.01 | 28.63 | 7.68 |

According to an embodiment of the present disclosure, when the X-ray powder diffraction pattern of the crystal form F has one, two, more, or all characteristic peaks at 2θ values selected from those described above, the crystal form F optionally further has relative intensities corresponding to the characteristic peaks.

According to an embodiment of the present disclosure, the crystal form F has an X-ray powder diffraction pattern substantially as shown in FIG. 6A.

According to an embodiment of the present disclosure, a differential scanning calorimetry (DSC) profile of the crystal form F has endothermic peaks at a peak temperature of about 69.9 °C, as well as an onset temperature of about 182.1 °C and/or a peak temperature of about 184.5 °C.

According to an embodiment of the present disclosure, the crystal form F has a differential scanning calorimetry (DSC) profile substantially as shown in FIG. 6B.

According to an embodiment of the present disclosure, thermogravimetric analysis (TGA) of the crystal form F shows a weight loss of about 2.2% to about 2.3% (e.g., about 2.25%) upon heating from room temperature to 100 °C, and a weight loss of about 2.7% to about 2.8% (e.g., about 2.79%) upon heating from 100 °C to 200 °C.

According to an embodiment of the present disclosure, the crystal form F has a thermogravimetric analysis (TGA) profile substantially as shown in FIG. 6B.

According to an embodiment of the present disclosure, the crystal form F is an ethanol solvate.

According to an embodiment of the present disclosure, the crystal form F has a $^{1}H$ NMR spectrum substantially as shown in FIG. 6C.

According to an embodiment of the present disclosure, the crystal form F has a variable-temperature XRPD pattern substantially as shown in FIG. 6D.

The present disclosure further provides a crystal form G of the compound of formula (I), wherein an X-ray powder diffraction pattern of the crystal form G has one, two, or all characteristic peaks at 2θ values selected from the following: 6.97°±0.20°, 13.39°±0.20°, and 25.81°±0.20°.

According to an embodiment of the present disclosure, the X-ray powder diffraction pattern of the crystal form G has one, two, more, or all characteristic peaks at 2θ values selected from the following: 4.50°±0.20°, 6.97°±0.20°, 13.39°±0.20°, 14.40°±0.20°, 17.11°±0.20°, 17.81°±0.20°, 23.94°±0.20°, and 25.81°±0.20°.

According to an embodiment of the present disclosure, the X-ray powder diffraction pattern of the crystal form G has one, two, more, or all characteristic peaks at 2θ values selected from the following:

| 2θ (°±0.20°) | Relative intensity (%) | 2θ (°±0.20°) | Relative intensity (%) |
|---|---|---|---|
| 4.50 | 68.36 | 17.11 | 67.74 |
| 6.97 | 100.00 | 17.81 | 52.92 |
| 8.12 | 10.53 | 18.32 | 19.72 |
| 10.11 | 31.07 | 18.59 | 10.38 |
| 10.38 | 33.69 | 19.10 | 15.02 |
| 10.67 | 31.00 | 20.17 | 30.64 |
| 11.57 | 14.89 | 20.44 | 33.33 |
| 11.93 | 25.80 | 21.42 | 19.08 |
| 12.22 | 29.54 | 21.67 | 7.29 |
| 13.39 | 69.03 | 23.25 | 25.32 |
| 13.86 | 38.14 | 23.94 | 67.70 |
| 14.40 | 49.78 | 24.56 | 14.71 |
| 14.59 | 37.62 | 25.81 | 70.39 |
| 15.06 | 46.53 | 26.28 | 17.76 |
| 16.26 | 38.00 | 28.29 | 13.66 |
| 16.53 | 19.39 | 29.80 | 5.96 |

According to an embodiment of the present disclosure, when the X-ray powder diffraction pattern of the crystal form G has one, two, more, or all characteristic peaks at 2θ values selected from those described above, the crystal form G optionally further has relative intensities corresponding to the characteristic peaks.

According to an embodiment of the present disclosure, the crystal form G has an X-ray powder diffraction pattern substantially as shown in FIG. 7A.

According to an embodiment of the present disclosure, a differential scanning calorimetry (DSC) profile of the crystal form G has endothermic peaks at a peak temperature of about 122.6 °C, as well as an onset temperature of about 168.5 °C and/or a peak temperature of about 173.1 °C.

According to an embodiment of the present disclosure, the crystal form G has a differential scanning calorimetry (DSC) profile substantially as shown in FIG. 7B.

According to an embodiment of the present disclosure, thermogravimetric analysis (TGA) of the crystal form G shows a weight loss of about 1.2% to about 1.4% (e.g., about 1.3%) upon heating from room temperature to 75 °C, and a weight loss of about 2.6% to about 2.7% (e.g., about 2.69%) upon heating from 75 °C to 150 °C.

According to an embodiment of the present disclosure, the crystal form G has a thermogravimetric analysis (TGA) profile substantially as shown in FIG. 7B.

According to an embodiment of the present disclosure, the crystal form G is an acetonitrile solvate.

According to an embodiment of the present disclosure, the crystal form G has a $^1$H NMR spectrum substantially as shown in FIG. 7C.

The present disclosure further provides a crystal form H of the compound of formula (I), wherein an X-ray powder diffraction pattern of the crystal form H has one, two, or all characteristic peaks at 2θ values selected from the following: 12.92°±0.20°, 15.82°±0.20°, and 17.25°±0.20°.

According to an embodiment of the present disclosure, the X-ray powder diffraction pattern of the crystal form H has one, two, more, or all characteristic peaks at 2θ values selected from the following: 7.89°±0.20°, 10.51°±0.20°, 12.92°±0.20°, 15.82°±0.20°, 16.69°±0.20°, 17.25°±0.20°, 19.12°±0.20°, and 25.39°±0.20°.

According to an embodiment of the present disclosure, the X-ray powder diffraction pattern of the crystal form H has one, two, more, or all characteristic peaks at 2θ values selected from the following:

| 2θ (°±0.20°) | Relative intensity (%) | 2θ (°±0.20°) | Relative intensity (%) |
|---|---|---|---|
| 4.31 | 23.68 | 15.82 | 46.68 |
| 5.26 | 16.90 | 16.69 | 29.20 |
| 7.89 | 33.13 | 17.25 | 100.00 |
| 8.62 | 12.44 | 17.89 | 14.51 |
| 9.81 | 6.36 | 19.12 | 31.51 |
| 10.51 | 29.88 | 21.60 | 22.40 |
| 10.88 | 15.01 | 24.72 | 16.92 |
| 12.00 | 14.59 | 25.39 | 43.61 |
| 12.92 | 76.77 | 27.32 | 15.48 |
| 14.18 | 8.13 | | |

According to an embodiment of the present disclosure, when the X-ray powder diffraction pattern of the crystal form H has one, two, more, or all characteristic peaks at 2θ values selected from those described above, the crystal form H optionally further has relative intensities corresponding to the characteristic peaks.

According to an embodiment of the present disclosure, the crystal form H has an X-ray powder diffraction pattern substantially as shown in FIG. 8A.

According to an embodiment of the present disclosure, a differential scanning calorimetry (DSC) profile of the crystal form H has endothermic peaks at peak temperatures of about 121.7 °C, about 138.7 °C, about 170.2 °C, and about 184.7 °C.

According to an embodiment of the present disclosure, the crystal form H has a differential scanning calorimetry (DSC) profile substantially as shown in FIG. 8B.

According to an embodiment of the present disclosure, thermogravimetric analysis (TGA) of the crystal form H shows a weight loss of about 1.7% to about 1.8% (e.g., about 1.73%) upon heating from room temperature to 70 °C, and a weight loss of about 7.9% to about 8.0% (e.g., about 7.98%) upon heating from 70 °C to 150 °C.

According to an embodiment of the present disclosure, the crystal form H has a thermogravimetric analysis (TGA) profile substantially as shown in FIG. 8B.

According to an embodiment of the present disclosure, the crystal form H is a dichloromethane solvate.

According to an embodiment of the present disclosure, the crystal form H has a $^1$H NMR spectrum substantially as shown in FIG. 8C.

According to an embodiment of the present disclosure, the crystal form H has a variable-temperature XRPD pattern substantially as shown in FIG. 8D.

The present disclosure further provides a crystal form I of the compound of formula (I), wherein an X-ray powder diffraction pattern of the crystal form I has one, two, more, or all characteristic peaks at 2θ values selected from the following: 4.45°±0.20°, 13.35°±0.20°, and 17.82°±0.20°.

According to an embodiment of the present disclosure, the X-ray powder diffraction pattern of the crystal form I has one, two, more, or all characteristic peaks at 2θ values selected from the following: 4.45°±0.20°, 13.35°±0.20°, 17.15°±0.20°, 17.82°±0.20°, 22.32°±0.20°, 23.82°±0.20°, and 25.70°±0.20°.

According to an embodiment of the present disclosure, the X-ray powder diffraction pattern of the crystal form I has one, two, more, or all characteristic peaks at 2θ values selected from the following:

| 2θ (°±0.20°) | Relative intensity (%) | 2θ (°±0.20°) | Relative intensity (%) |
|---|---|---|---|
| 4.45 | 59.28 | 21.42 | 5.47 |
| 6.94 | 4.68 | 22.32 | 15.20 |
| 10.43 | 3.20 | 23.00 | 5.45 |
| 11.88 | 4.62 | 23.37 | 4.12 |
| 12.21 | 3.57 | 23.82 | 22.31 |
| 13.35 | 60.14 | 24.44 | 5.75 |
| 14.39 | 11.28 | 25.70 | 18.30 |

(continued)

| 2θ (°±0.20°) | Relative intensity (%) | 2θ (°±0.20°) | Relative intensity (%) |
|---|---|---|---|
| 15.09 | 5.78 | 26.19 | 7.94 |
| 16.36 | 3.84 | 26.85 | 1.62 |
| 16.59 | 2.39 | 27.62 | 1.40 |
| 17.15 | 24.86 | 28.21 | 2.80 |
| 17.82 | 100.00 | 29.62 | 6.73 |
| 18.69 | 6.19 | 30.07 | 1.01 |
| 19.03 | 2.40 | 31.46 | 0.63 |
| 19.85 | 1.16 | 33.15 | 1.16 |
| 20.51 | 2.75 | 33.86 | 0.29 |
| 20.73 | 2.86 | 34.31 | 1.59 |
| | | 36.08 | 0.87 |

According to an embodiment of the present disclosure, when the X-ray powder diffraction pattern of the crystal form I has one, two, more, or all characteristic peaks at 2θ values selected from those described above, the crystal form I optionally further has relative intensities corresponding to the characteristic peaks.

According to an embodiment of the present disclosure, the crystal form I has an X-ray powder diffraction pattern substantially as shown in FIG. 9.

The present disclosure further provides a crystal form J of the compound of formula (I), wherein an X-ray powder diffraction pattern of the crystal form J has one, two, or all characteristic peaks at 2θ values selected from the following: 4.22°±0.20°, 7.15°±0.20°, and 25.24°±0.20°.

According to an embodiment of the present disclosure, the X-ray powder diffraction pattern of the crystal form J has one, two, more, or all characteristic peaks at 2θ values selected from the following: 4.22°±0.20°, 6.74°±0.20°, 7.15°±0.20°, 11.04°±0.20°, 12.62°±0.20°, 13.48°±0.20°, 16.86°±0.20°, and 25.24°±0.20°.

According to an embodiment of the present disclosure, the X-ray powder diffraction pattern of the crystal form J has one, two, more, or all characteristic peaks at 2θ values selected from the following:

| 2θ (°±0.20°) | Relative intensity (%) | 2θ (°±0.20°) | Relative intensity (%) |
|---|---|---|---|
| 4.22 | 100.00 | 14.77 | 14.97 |
| 6.74 | 42.15 | 15.52 | 7.86 |
| 7.15 | 71.32 | 16.32 | 18.04 |
| 9.71 | 11.37 | 16.86 | 44.59 |
| 11.04 | 22.41 | 18.32 | 15.86 |
| 12.62 | 42.93 | 19.79 | 19.53 |
| 13.48 | 37.00 | 21.08 | 17.26 |
| 14.09 | 16.97 | 25.24 | 47.53 |

According to an embodiment of the present disclosure, when the X-ray powder diffraction pattern of the crystal form J has one, two, more, or all characteristic peaks at 2θ values selected from those described above, the crystal form J optionally further has relative intensities corresponding to the characteristic peaks.

According to an embodiment of the present disclosure, the crystal form J has an X-ray powder diffraction pattern substantially as shown in FIG. 10A.

According to an embodiment of the present disclosure, a differential scanning calorimetry (DSC) profile of the crystal form J has endothermic peaks at an onset temperature of about 178.6 °C and/or a peak temperature of about 182.4 °C, as well as a peak temperature of about 188.9 °C.

According to an embodiment of the present disclosure, the crystal form J has a differential scanning calorimetry (DSC) profile substantially as shown in FIG. 10B.

According to an embodiment of the present disclosure, thermogravimetric analysis (TGA) of the crystal form J shows a weight loss of about 1.4% to about 1.5% (e.g., about 1.41%) upon heating from room temperature to 100 °C, and a weight loss of about 3.3% to about 3.4% (e.g., about 3.38%) upon heating from 100 °C to 200 °C.

According to an embodiment of the present disclosure, the crystal form J has a thermogravimetric analysis (TGA) profile substantially as shown in FIG. 10B.

According to an embodiment of the present disclosure, the crystal form J is a 1,4-dioxane solvate.

According to an embodiment of the present disclosure, the crystal form J has a $^1H$ NMR spectrum substantially as shown in FIG. 10C.

According to an embodiment of the present disclosure, the crystal form J has a variable-temperature XRPD pattern substantially as shown in FIG. 10D.

The present disclosure further provides a crystal form K of the compound of formula (I), wherein an X-ray powder diffraction pattern of the crystal form K has one, two, or all characteristic peaks at 2θ values selected from the following: 4.19°±0.20°, 6.48°±0.20°, 6.95°±0.20°, and 19.54°±0.20°.

According to an embodiment of the present disclosure, the X-ray powder diffraction pattern of the crystal form K has one, two, more, or all characteristic peaks at 2θ values selected from the following: 4.19°±0.20°, 5.13°±0.20°, 6.48°±0.20°, 6.95°±0.20°, 9.75°±0.20°, 11.06°±0.20°, 17.13°±0.20°, and 19.54°±0.20°.

According to an embodiment of the present disclosure, the X-ray powder diffraction pattern of the crystal form K has one, two, more, or all characteristic peaks at 2θ values selected from the following:

| 2θ (°±0.20°) | Relative intensity (%) | 2θ (°±0.20°) | Relative intensity (%) |
|---|---|---|---|
| 4.19 | 33.89 | 13.06 | 6.44 |
| 5.13 | 13.99 | 15.11 | 5.81 |
| 6.48 | 30.66 | 17.13 | 14.65 |
| 6.95 | 38.26 | 17.94 | 7.16 |
| 9.75 | 17.21 | 19.54 | 100.00 |
| 11.06 | 15.81 | 24.85 | 8.49 |

According to an embodiment of the present disclosure, when the X-ray powder diffraction pattern of the crystal form K has one, two, more, or all characteristic peaks at 2θ values selected from those described above, the crystal form K optionally further has relative intensities corresponding to the characteristic peaks.

According to an embodiment of the present disclosure, the crystal form K has an X-ray powder diffraction pattern substantially as shown in FIG. 11A.

According to an embodiment of the present disclosure, the crystal form K has a differential scanning calorimetry (DSC) profile substantially as shown in FIG. 11B.

According to an embodiment of the present disclosure, the crystal form K has a thermogravimetric analysis (TGA) profile substantially as shown in FIG. 11B.

According to an embodiment of the present disclosure, the crystal form K has a $^1H$ NMR spectrum substantially as shown in FIG. 11C.

The present disclosure further provides a crystal form L of the compound of formula (I), wherein an X-ray powder diffraction pattern of the crystal form L has one, two, or all characteristic peaks at 2θ values selected from the following: 18.85°±0.20°, 22.30°±0.20°, and 29.35°±0.20°.

According to an embodiment of the present disclosure, the X-ray powder diffraction pattern of the crystal form L has one, two, more, or all characteristic peaks at 2θ values selected from the following: 9.23°±0.20°, 18.85°±0.20°, 22.30°±0.20°, 22.69°±0.20°, 23.22°±0.20°, 23.82°±0.20°, 26.58°±0.20°, and 29.35°±0.20°.

According to an embodiment of the present disclosure, the X-ray powder diffraction pattern of the crystal form L has one, two, more, or all characteristic peaks at 2θ values selected from the following:

| 2θ (°±0.20°) | Relative intensity (%) | 2θ (°±0.20°) | Relative intensity (%) |
|---|---|---|---|
| 9.23 | 16.99 | 23.22 | 6.91 |
| 16.46 | 6.68 | 23.82 | 14.93 |
| 18.85 | 32.50 | 26.58 | 7.24 |
| 21.00 | 5.41 | 27.56 | 6.83 |

(continued)

| 2θ (°±0.20°) | Relative intensity (%) | 2θ (°±0.20°) | Relative intensity (%) |
|---|---|---|---|
| 22.30 | 100.00 | 29.35 | 18.50 |
| 22.69 | 8.25 | | |

According to an embodiment of the present disclosure, when the X-ray powder diffraction pattern of the crystal form L has one, two, more, or all characteristic peaks at 2θ values selected from those described above, the crystal form L optionally further has relative intensities corresponding to the characteristic peaks.

According to an embodiment of the present disclosure, the crystal form L has an X-ray powder diffraction pattern substantially as shown in FIG. 12A.

According to an embodiment of the present disclosure, a differential scanning calorimetry (DSC) profile of the crystal form L has endothermic peaks at an onset temperature of about 95.4 °C and/or a peak temperature of about 100.0 °C, as well as a peak temperature of about 154.5 °C.

According to an embodiment of the present disclosure, the crystal form L has a differential scanning calorimetry (DSC) profile substantially as shown in FIG. 12B.

According to an embodiment of the present disclosure, thermogravimetric analysis (TGA) of the crystal form L has a weight loss of about 8.9% to about 9.0% (e.g., about 8.98%) upon heating from room temperature to 100 °C, a weight loss of about 2.5% to about 2.6% (e.g., about 2.56%) upon heating from 100 °C to 125 °C, and a weight loss of about 1.8% to about 1.9% (e.g., about 1.81%) upon heating from 125 °C to 150 °C.

According to an embodiment of the present disclosure, the crystal form L has a thermogravimetric analysis (TGA) profile substantially as shown in FIG. 12B.

According to an embodiment of the present disclosure, the crystal form L is a methanol-water co-solvate.

According to an embodiment of the present disclosure, the crystal form L has a $^1$H NMR spectrum substantially as shown in FIG. 12C.

According to an embodiment of the present disclosure, a monocrystal of the crystal form L has a schematic asymmetric unit diagram of a monocrystal structure as shown in FIG. 12D.

According to an embodiment of the present disclosure, the monocrystal of the crystal form L has the following structural parameters:

| | |
|---|---|
| Empirical formula | $C_{27}H_{28}N_8O_2 \cdot CH_3OH \cdot 2H_2O$ |
| Formula weight | 564.65 |
| Temperature | 119.99(10) K |
| Wavelength | Cu/$K_\alpha$ ($\lambda$ = 1.54184 Å) |
| Crystal system, space group | Triclinic, $P\bar{1}$ |
| Unit cell dimensions | $a$ = 10.86800(10) Å<br>$b$ = 11.09330(10) Å<br>$c$ = 12.30730(10) Å<br>$\alpha$ = 110.4970(10)°<br>$\beta$ = 96.2040(10)°<br>$\gamma$ = 92.5880(10)° |
| Unit cell volume | 1376.22(2) Å$^3$ |
| Z, calculated density | 2, 1.363 g/cm$^3$ |
| Absorption coefficient | 0.791 mm$^{-1}$ |
| F(000) | 600.0 |
| Crystal size | 0.172 × 0.156 × 0.145 mm$^3$ |
| 2θ range for data collection | 7.738 to 152.61 |
| Limiting indices | $-13 \le h \le 13$<br>$-13 \le k \le 13$<br>$-15 \le l \le 15$ |
| Reflections collected/unique | 59379/5497 [$R_{int}$=0.0150, $R_{sigma}$=0.0058] |

(continued)

| | |
|---|---|
| Refinement method | Full-matrix least-squares on $F^2$ |
| Completeness | 99.46 % |
| Data/restraints/parameters | 5497/0/380 |
| Goodness-of-fit on F2 | 1.044 |
| Final R indices [I ≥ 2sigma(I)] | $R_1$ = 0.0425, $wR_2$ = 0.1147 |
| Final R indices [all data] | $R_1$ = 0.0427, $wR_2$ = 0.1149 |
| Largest diff. peak and hole | 0.45/-0.40 e.Å$^{-3}$ |

The present disclosure further provides a crystal form M of the compound of formula (I), wherein an X-ray powder diffraction pattern of the crystal form M has one, two, or all characteristic peaks at 2θ values selected from the following: 4.22°±0.20°, 18.59°±0.20°, and 25.31°±0.20°.

According to an embodiment of the present disclosure, the X-ray powder diffraction pattern of the crystal form M has one, two, more, or all characteristic peaks at 2θ values selected from the following: 4.22°±0.20°, 9.05°±0.20°, 10.94°±0.20°, 15.48°±0.20°, 18.59°±0.20°, 21.81°±0.20°, 25.31°±0.20°, and 26.48°±0.20°.

According to an embodiment of the present disclosure, the X-ray powder diffraction pattern of the crystal form M has the following characteristic peaks:

| 2θ (°±0.20°) | Relative intensity (%) | 2θ (°±0.20°) | Relative intensity (%) |
|---|---|---|---|
| 4.22 | 72.08 | 16.77 | 27.47 |
| 9.05 | 41.37 | 18.59 | 74.74 |
| 10.94 | 64.25 | 19.51 | 16.93 |
| 12.59 | 23.51 | 20.19 | 18.81 |
| 13.38 | 26.12 | 21.12 | 9.22 |
| 14.05 | 9.52 | 21.81 | 47.54 |
| 15.48 | 33.17 | 25.31 | 100.00 |
| 15.99 | 21.65 | 26.48 | 70.66 |

According to an embodiment of the present disclosure, when the X-ray powder diffraction pattern of the crystal form M has one, two, more, or all characteristic peaks at 2θ values selected from those described above, the crystal form M optionally further has relative intensities corresponding to the characteristic peaks.

According to an embodiment of the present disclosure, the crystal form M has an X-ray powder diffraction pattern substantially as shown in FIG. 13A.

According to an embodiment of the present disclosure, a differential scanning calorimetry (DSC) profile of the crystal form M has endothermic peaks at a peak temperature of about 61.3 °C, as well as an onset temperature of about 181.4 °C and/or a peak temperature of about 184.2 °C.

According to an embodiment of the present disclosure, the crystal form M has a differential scanning calorimetry (DSC) profile substantially as shown in FIG. 13B.

According to an embodiment of the present disclosure, thermogravimetric analysis (TGA) of the crystal form M shows a weight loss of about 2.0% to about 2.1% (e.g., about 2.05%) upon heating from room temperature to 200 °C.

According to an embodiment of the present disclosure, the crystal form M has a thermogravimetric analysis (TGA) profile substantially as shown in FIG. 13B.

According to an embodiment of the present disclosure, the crystal form M is a hydrate.

The present disclosure further provides a crystal form N of the compound of formula (I), wherein an X-ray powder diffraction pattern of the crystal form N has one, two, or all characteristic peaks at 2θ values selected from the following: 5.82°±0.20°, 13.96°±0.20°, and 20.24°±0.20°.

According to an embodiment of the present disclosure, the X-ray powder diffraction pattern of the crystal form N has one, two, more, or all characteristic peaks at 2θ values selected from the following: 5.82°±0.20°, 7.18°±0.20°, 12.35°±0.20°, 13.96°±0.20°, 15.77°±0.20°, 17.49°±0.20°, 18.40°±0.20°, and 20.24°±0.20°.

According to an embodiment of the present disclosure, the X-ray powder diffraction pattern of the crystal form N

has one, two, more, or all characteristic peaks at 2θ values selected from the following:

| 2θ (°±0.20°) | Relative intensity (%) | 2θ (°±0.20°) | Relative intensity (%) |
|---|---|---|---|
| 5.82 | 100.00 | 17.49 | 12.11 |
| 7.18 | 8.24 | 18.40 | 9.06 |
| 12.35 | 12.08 | 20.24 | 15.65 |
| 13.96 | 19.56 | 24.74 | 5.95 |
| 15.77 | 6.54 | | |

According to an embodiment of the present disclosure, when the X-ray powder diffraction pattern of the crystal form N has one, two, more, or all characteristic peaks at 2θ values selected from those described above, the crystal form N optionally further has relative intensities corresponding to the characteristic peaks.

According to an embodiment of the present disclosure, the crystal form N has an X-ray powder diffraction pattern substantially as shown in FIG. 14A.

According to an embodiment of the present disclosure, a differential scanning calorimetry (DSC) profile of the crystal form N has an endothermic peak at an onset temperature of about 118.0 °C and/or a peak temperature of about 125.9 °C.

According to an embodiment of the present disclosure, the crystal form N has a differential scanning calorimetry (DSC) profile substantially as shown in FIG. 14B.

According to an embodiment of the present disclosure, thermogravimetric analysis (TGA) of the crystal form N shows a weight loss of about 0.1% to about 0.2% (e.g., about 0.19%) upon heating from room temperature to 200 °C.

According to an embodiment of the present disclosure, the crystal form N has a thermogravimetric analysis (TGA) profile substantially as shown in FIG. 14B.

According to an embodiment of the present disclosure, the crystal form N is an anhydrous crystal form.

According to an embodiment of the present disclosure, the crystal form N has a $^1$H NMR spectrum substantially as shown in FIG. 14C.

According to an embodiment of the present disclosure, the crystal form N has a variable-temperature XRPD pattern substantially as shown in FIG. 14D.

The present disclosure further provides a crystal form O of the compound of formula (I), wherein an X-ray powder diffraction pattern of the crystal form O has one, two, or all characteristic peaks at 2θ values selected from the following: 11.06°±0.20°, 24.85°±0.20°, and 26.44°±0.20°.

According to an embodiment of the present disclosure, the X-ray powder diffraction pattern of the crystal form O has one, two, more, or all characteristic peaks at 2θ values selected from the following: 4.32°±0.20°, 11.06°±0.20°, 15.11°±0.20°, 15.76°±0.20°, 17.22°±0.20°, 17.93°±0.20°, 24.85°±0.20°, and 26.44°±0.20°.

According to an embodiment of the present disclosure, the X-ray powder diffraction pattern of the crystal form O has one, two, more, or all characteristic peaks at 2θ values selected from the following:

| 2θ (°±0.20°) | Relative intensity (%) | 2θ (°±0.20°) | Relative intensity (%) |
|---|---|---|---|
| 4.32 | 44.06 | 17.22 | 34.55 |
| 6.94 | 17.36 | 17.93 | 37.32 |
| 8.95 | 25.93 | 18.40 | 22.98 |
| 9.23 | 21.87 | 19.18 | 23.32 |
| 10.37 | 8.14 | 19.53 | 27.12 |
| 11.06 | 70.26 | 19.92 | 21.69 |
| 12.34 | 11.27 | 20.74 | 20.04 |
| 12.90 | 15.97 | 21.57 | 12.40 |
| 13.28 | 16.53 | 22.36 | 33.36 |
| 13.78 | 7.55 | 22.97 | 6.51 |
| 14.18 | 27.87 | 23.85 | 9.78 |

(continued)

| 2θ (°±0.20°) | Relative intensity (%) | 2θ (°±0.20°) | Relative intensity (%) |
|---|---|---|---|
| 15.11 | 39.89 | 24.85 | 100.00 |
| 15.76 | 33.72 | 25.37 | 20.79 |
| 16.14 | 13.71 | 26.44 | 55.28 |

According to an embodiment of the present disclosure, when the X-ray powder diffraction pattern of the crystal form O has one, two, more, or all characteristic peaks at 2θ values selected from those described above, the crystal form O optionally further has relative intensities corresponding to the characteristic peaks.

According to an embodiment of the present disclosure, the crystal form O has an X-ray powder diffraction pattern substantially as shown in FIG. 15A.

According to an embodiment of the present disclosure, a differential scanning calorimetry (DSC) profile of the crystal form O has endothermic peaks at peak temperatures of about 174.7 °C and about 187.1 °C.

According to an embodiment of the present disclosure, the crystal form O has a differential scanning calorimetry (DSC) profile substantially as shown in FIG. 15B.

According to an embodiment of the present disclosure, thermogravimetric analysis (TGA) of the crystal form O shows a weight loss of about 4.0% to about 4.1% (e.g., about 4.03%) upon heating from room temperature to 150 °C, and a weight loss of about 1.5% to about 1.7% (e.g., about 1.60%) upon heating from 150 °C to 200 °C.

According to an embodiment of the present disclosure, the crystal form O has a thermogravimetric analysis (TGA) profile substantially as shown in FIG. 15B.

According to an embodiment of the present disclosure, the crystal form O is a methanol solvate. According to an embodiment of the present disclosure, the crystal form O has a $^1$H NMR spectrum substantially as shown in FIG. 15C.

According to an embodiment of the present disclosure, the crystal form O has a variable-temperature XRPD pattern substantially as shown in FIG. 15D.

The present disclosure further provides a crystal form P of the compound of formula (I), wherein an X-ray powder diffraction pattern of the crystal form P has one, two, or all characteristic peaks at 2θ values selected from the following: 6.36°±0.20°, 16.15°±0.20°, and 21.05°±0.20°.

According to an embodiment of the present disclosure, the X-ray powder diffraction pattern of the crystal form P has one, two, more, or all characteristic peaks at 2θ values selected from the following: 6.36°±0.20°, 11.09°±0.20°, 16.15°±0.20°, 17.27°±0.20°, 21.05°±0.20°, 26.38°±0.20°, 26.62°±0.20°, and 34.88°±0.20°.

According to an embodiment of the present disclosure, the X-ray powder diffraction pattern of the crystal form P has one, two, more, or all characteristic peaks at 2θ values selected from the following:

| 2θ (°±0.20°) | Relative intensity (%) | 2θ (°±0.20°) | Relative intensity (%) |
|---|---|---|---|
| 6.36 | 32.98 | 21.05 | 100.00 |
| 11.09 | 22.80 | 26.38 | *5.50* |
| 16.15 | 28.39 | 26.62 | 15.20 |
| 17.27 | 14.85 | 34.88 | 7.45 |

According to an embodiment of the present disclosure, when the X-ray powder diffraction pattern of the crystal form P has one, two, more, or all characteristic peaks at 2θ values selected from those described above, the crystal form P optionally further has relative intensities corresponding to the characteristic peaks.

According to an embodiment of the present disclosure, the crystal form P has an X-ray powder diffraction pattern substantially as shown in FIG. 16A.

According to an embodiment of the present disclosure, the crystal form P is a hydrate.

The present disclosure further provides a crystal form Q of the compound of formula (I), wherein an X-ray powder diffraction pattern of the crystal form Q has one, two, or all characteristic peaks at 2θ values selected from the following: 18.41°±0.20°, 25.19°±0.20°, and 26.36°±0.20°.

According to an embodiment of the present disclosure, the X-ray powder diffraction pattern of the crystal form Q has one, two, more, or all characteristic peaks at 2θ values selected from the following: 4.15°±0.20°, 10.85°±0.20°, 13.28°±0.20°, 18.41°±0.20°, 20.03°±0.20°, 21.69°±0.20°, 25.19°±0.20°, and 26.36°±0.20°.

According to an embodiment of the present disclosure, the X-ray powder diffraction pattern of the crystal form Q has one, two, more, or all characteristic peaks at 2θ values selected from the following:

| 2θ (°±0.20°) | Relative intensity (%) | 2θ (°±0.20°) | Relative intensity (%) |
|---|---|---|---|
| 4.15 | 51.68 | 17.48 | 14.29 |
| 7.51 | 5.15 | 18.41 | 66.25 |
| 9.03 | 24.82 | 19.34 | 19.72 |
| 10.49 | 8.43 | 20.03 | 30.92 |
| 10.85 | 47.93 | 20.88 | 26.11 |
| 12.48 | 15.35 | 21.03 | 29.01 |
| 13.28 | 32.28 | 21.69 | 41.97 |
| 14.07 | 17.54 | 22.90 | 7.26 |
| 15.38 | 29.38 | 25.19 | 100.00 |
| 15.83 | 26.54 | 26.36 | 66.69 |
| 16.28 | 25.34 | 28.17 | 8.48 |
| 16.66 | 27.68 | 29.19 | 6.84 |

According to an embodiment of the present disclosure, when the X-ray powder diffraction pattern of the crystal form Q has one, two, more, or all characteristic peaks at 2θ values selected from those described above, the crystal form Q optionally further has relative intensities corresponding to the characteristic peaks.

According to an embodiment of the present disclosure, the crystal form Q has an X-ray powder diffraction pattern substantially as shown in FIG. 17.

According to an embodiment of the present disclosure, the crystal form Q is an anhydrous crystal form.

The present disclosure further provides a crystal form R of the compound of formula (I), wherein an X-ray powder diffraction pattern of the crystal form R has one, two, or all characteristic peaks at 2θ values selected from the following: 7.22°±0.20°, 13.03°±0.20°, and 18.81°±0.20°.

According to an embodiment of the present disclosure, the X-ray powder diffraction pattern of the crystal form R has one, two, more, or all characteristic peaks at 2θ values selected from the following: 7.22°±0.20°, 13.03°±0.20°, 18.81°±0.20°, and 19.15°±0.20°.

According to an embodiment of the present disclosure, the X-ray powder diffraction pattern of the crystal form R has one, two, more, or all characteristic peaks at 2θ values selected from the following:

| 2θ (°±0.20°) | Relative intensity (%) | 2θ (°±0.20°) | Relative intensity (%) |
|---|---|---|---|
| 6.24 | 12 | 19.15 | 47.4 |
| 7.22 | 93.3 | 20.17 | 5.9 |
| 12.52 | 11.8 | 21.79 | 3 |
| 13.03 | 37.1 | 23.79 | 3.7 |
| 14.46 | 18.5 | 24.43 | 2.9 |
| 16.49 | 6.8 | 25.11 | 7.7 |
| 17.81 | 0.8 | 26.92 | 3.6 |
| 18.81 | 100 | 31.84 | 2 |
| | | 33.44 | 5.3 |

According to an embodiment of the present disclosure, when the X-ray powder diffraction pattern of the crystal form R has one, two, more, or all characteristic peaks at 2θ values selected from those described above, the crystal form R optionally further has relative intensities corresponding to the characteristic peaks.

According to an embodiment of the present disclosure, the crystal form R has an X-ray powder diffraction pattern substantially as shown in FIG. 22A.

According to an embodiment of the present disclosure, the crystal form R is an anhydrous crystal form.

The present disclosure further provides a crystal form S of the compound of formula (I), wherein an X-ray powder

diffraction pattern of the crystal form S has one, two, or all characteristic peaks at 2θ values selected from the following: 6.30°±0.20°, 16.11°±0.20°, and 21.01°±0.20°.

According to an embodiment of the present disclosure, the X-ray powder diffraction pattern of the crystal form S has one, two, more, or all characteristic peaks at 2θ values selected from the following: 6.30°±0.20°, 11.06°±0.20°, 16.11°±0.20°, and 21.01°±0.20°.

According to an embodiment of the present disclosure, the X-ray powder diffraction pattern of the crystal form S has one, two, more, or all characteristic peaks at **2θ** values selected from the following:

| 2θ (°±0.20°) | Relative intensity (%) | 2θ (°±0.20°) | Relative intensity (%) |
|---|---|---|---|
| 6.30 | 98.9 | 19.08 | 3.9 |
| 7.06 | 5.7 | 19.79 | 2.7 |
| 8.79 | 3.5 | 21.01 | 100 |
| 11.06 | 32 | 22.89 | 10 |
| 11.8 | 4.6 | 25.39 | 16.7 |
| 14.09 | 4.8 | 25.73 | 22.5 |
| 14.85 | 8.7 | 26.57 | 14.1 |
| 15.41 | 2.5 | 28.25 | 10.8 |
| 16.11 | 35.8 | 28.45 | 7.9 |
| 16.61 | 25 | 31.91 | 9.3 |
| 17.25 | 16.8 | 33.53 | 2.7 |
| 18.38 | 8 | 34.87 | 14.4 |

According to an embodiment of the present disclosure, when the X-ray powder diffraction pattern of the crystal form S has one, two, more, or all characteristic peaks at 2θ values selected from those described above, the crystal form S optionally further has relative intensities corresponding to the characteristic peaks.

**According** to an embodiment of the present disclosure, the crystal form S has an X-ray powder diffraction pattern substantially as shown in FIG. 23.

According to an embodiment of the present disclosure, the crystal form S is a hydrate.

The present disclosure further provides a crystal form T of maleate of the compound of formula (I), wherein the crystal form T has an X-ray powder diffraction pattern substantially as shown in FIG. 24A.

The present disclosure further provides a crystal form U of methanesulfonate of the compound of formula (I), wherein the crystal form U has an X-ray powder diffraction pattern substantially as shown in FIG. 25A.

According to the context of the specification in the present disclosure, data such as X-ray powder diffraction patterns or 2θ values of the crystal forms are obtained by using Cu target radiation.

The present disclosure further provides a preparation method for the crystal form A, wherein the preparation method for the crystal form A comprises dissolving the compound of formula (I) (such as an amorphous form thereof, e.g., amorphous form Z) in a mixed solvent of ethyl acetate and dichloromethane, and volatilizing the resulting solution at room temperature to obtain the crystal form A. Preferably, in the preparation method for the crystal form A, ethyl acetate and dichloromethane may be in a volume ratio of 1:1 to 20:1, e.g., 9:1.

The present disclosure further provides a preparation method for the crystal form A, wherein the preparation method for the crystal form A comprises heating the crystal form G to 150 °C, cooling to room temperature, and exposing to air to obtain the crystal form A.

The present disclosure further provides a preparation method for the crystal form A, wherein the preparation method for the crystal form A comprises drying the crystal form I at room temperature to obtain the crystal form A.

The present disclosure further provides a preparation method for the crystal form L, wherein the preparation method for the crystal form L comprises mixing the compound of formula (I) (such as an amorphous form thereof) with a mixed solvent of methanol and water to obtain a solution, and volatilizing the resulting solution at room temperature to obtain the crystal form L. Preferably, in the preparation method for the crystal form L, methanol and water may be in a volume ratio of 1:1 to 20:1, e.g., 4:1.

The present disclosure further provides a preparation method for the crystal form B, wherein the preparation method for the crystal form B comprises stirring the crystal form L in acetone to obtain the crystal form B. Preferably, the crystal form L and acetone may be in a mass-to-volume ratio of 20 mg:(0.1-1 mL), e.g., 20 mg:0.5 mL. Preferably, the

stirring is performed at 50-70 °C, e.g., 60 °C.

The present disclosure further provides a preparation method for the crystal form C, wherein the preparation method for the crystal form C comprises stirring the crystal form L in acetone at room temperature first, heating to 60 °C and stirring, cooling to 0-5 °C and stirring, and then mixing the separated solid with water and stirring to obtain the crystal form C. Preferably, the crystal form L and acetone may be in a mass-to-volume ratio of 20 mg:(0.1-1 mL), e.g., 20 mg:0.5 mL; the crystal form L and water may be in a mass-to-volume ratio of 20 mg:(0.1-1 mL), e.g., 20 mg:0.5 mL.

Preferably, the stirring of the crystal form L in acetone is performed first at 50-70 °C, e.g., 60 °C, and then at 0-5 °C. The separated solid is stirred in water at room temperature.

Alternatively, the crystal form L is stirred in a mixed solvent of acetone and water, wherein acetone and water may be in a volume ratio of (1-2):1, e.g., 1.5:1.

Alternatively, a wet sample of the crystal form P is dried in the air to obtain the crystal form C. The present disclosure further provides a preparation method for the crystal form D, wherein the preparation method for the crystal form D comprises stirring the crystal form L in acetone at room temperature first, heating to 60 °C and stirring, cooling to 0-5 °C and stirring, and then stirring the separated solid in a mixed solvent of acetone and water to obtain the crystal form D. Preferably, the crystal form L and acetone may be in a mass-to-volume ratio of 20 mg:(0.1-1 mL), e.g., 20 mg:0.5 mL; the crystal form L and the mixed solvent of acetone and water may be in a mass-to-volume ratio of 20 mg:(0.1-1 mL), e.g., 20 mg:0.5 mL. Preferably, in the mixed solvent of acetone and water, acetone and water may be in a volume ratio of (5-7):1, e.g., 1.5:1. Preferably, the stirring of the crystal form L in acetone is performed first at 50-70 °C, e.g., 60 °C, and then at 0-5 °C. The separated solid is stirred in the mixed solvent of acetone and water at room temperature.

Alternatively, the crystal form L is stirred in a mixed solvent of acetone and water, wherein acetone and water may be in a volume ratio of (3-6):1, e.g., 4:1.

The present disclosure further provides a preparation method for the crystal form E, wherein the preparation method for the crystal form E comprises stirring the crystal form L in methanol to obtain the crystal form E. Preferably, the stirring is performed at room temperature. Preferably, the crystal form L and methanol may be in a mass-to-volume ratio of 20 mg:(0.1-1 mL), e.g., 20 mg:0.5 mL.

The present disclosure further provides a preparation method for the crystal form F, wherein the preparation method for the crystal form F comprises stirring the compound of formula (I) (such as an amorphous form thereof) in a mixed solvent of acetone and ethanol to obtain the crystal form F. Preferably, in the preparation method for the crystal form L, acetone and ethanol may be in a volume ratio of 1:1.

The present disclosure further provides a preparation method for the crystal form G, wherein the preparation method for the crystal form G comprises stirring the crystal form L in acetone first, and then stirring the separated solid in acetonitrile to obtain the crystal form G. Preferably, the crystal form L and acetone may be in a mass-to-volume ratio of 20 mg:(0.1-1 mL), e.g., 20 mg:0.5 mL; the crystal form L and acetonitrile may be in a mass-to-volume ratio of 20 mg:(0.1-1 mL), e.g., 20 mg:0.5 mL.

The present disclosure further provides a preparation method for the crystal form H, wherein the preparation method for the crystal form H comprises stirring the crystal form L in acetone at room temperature first, heating to 60 °C and stirring, cooling to 0-5 °C and stirring, and then performing gas-solid diffusion on the separated solid in a dichloromethane atmosphere to obtain the crystal form H. Preferably, the crystal form L and acetone may be in a mass-to-volume ratio of 20 mg:(0.1-1 mL), e.g., 20 mg:0.5 mL.

The present disclosure further provides a preparation method for the crystal form I, wherein the preparation method for the crystal form I comprises dissolving the compound of formula (I) (such as an amorphous form thereof) in N-methylpyrrolidone, then adding an anti-solvent such as acetonitrile, and allowing to stand at room temperature to precipitate a solid, thereby obtaining the crystal form I. Preferably, the compound of formula (I) and *N*-methylpyrrolidone may be in a mass-to-volume ratio of 20 mg:(1.0-1.5 mL). The compound of formula (I) and *N*-methylpyrrolidone may be in a mass-to-volume ratio of 20 mg:(3-5 mL), e.g., 20 mg:4 mL.

The present disclosure further provides a preparation method for the crystal form J, wherein the preparation method for the crystal form J comprises stirring the crystal form L in acetone at room temperature first, heating to 60 °C and stirring, cooling to 0-5 °C and stirring, mixing the separated solid with 1,4-dioxane and stirring at 50 °C, and then stirring at room temperature to obtain the crystal form J. Preferably, the crystal form L and acetone may be in a mass-to-volume ratio of 20 mg:(0.1-1 mL), e.g., 20 mg:0.5 mL. The crystal form L and 1,4-dioxane may be in a mass-to-volume ratio of 20 mg:(0.1-1 mL), e.g., 20 mg:0.5 mL.

The present disclosure further provides a preparation method for the crystal form M, wherein the preparation method for the crystal form M comprises heating the crystal form D to 150 °C, then cooling to room temperature, and exposing to air for about 10 min to obtain the crystal form M.

The present disclosure further provides a preparation method for the crystal form M, wherein the preparation method for the crystal form M comprises heating the crystal form F to 160 °C, cooling to room temperature, and exposing to air to obtain the crystal form M.

The present disclosure further provides a preparation method for the crystal form M, wherein the preparation

method for the crystal form M comprises heating a crystal form O sample to 175 °C to obtain the crystal form M.

The present disclosure further provides a preparation method for the crystal form M, wherein the preparation method for the crystal form M comprises placing the crystal form Q at room temperature and room humidity to obtain the crystal form M.

The present disclosure further provides a preparation method for the crystal form N, wherein the preparation method for the crystal form N comprises heating the crystal form E to 100 °C, then cooling to room temperature, and exposing to air to obtain the crystal form N.

The present disclosure further provides a preparation method for the crystal form O, wherein the preparation method for the crystal form O comprises dissolving the compound of formula (I) (such as an amorphous form thereof, e.g., amorphous form Z) in a mixed solvent of methanol and water, and volatilizing at room temperature to obtain the crystal form O. Preferably, in the mixed solvent of methanol and water, methanol and water may be in a volume ratio of (3-5):1, e.g., 4:1.

The present disclosure further provides a preparation method for the crystal form P, wherein the preparation method for the crystal form P comprises stirring the crystal form L in acetone at room temperature first, heating to 60 °C and stirring, cooling to 0-5 °C and stirring, and then slurring the separated solid in a mixed solvent of MEK, THF, and $H_2O$ to obtain the crystal form P. Preferably, in the mixed solvent of MEK, THF, and $H_2O$, MEK, THF, and $H_2O$ may be in a volume ratio of 1:0.1:2.

The present disclosure further provides a preparation method for the crystal form Q, wherein the preparation method for the crystal form Q comprises purging the crystal form D under $N_2$ atmosphere for 20 min, heating the sample to 150 °C, and cooling to 30 °C to obtain the crystal form Q.

The present disclosure further provides a preparation method for the crystal form R, wherein the preparation method for the crystal form R comprises mixing the compound of formula (I) (such as an amorphous form, e.g., amorphous form Z) with a mixed solvent of dichloromethane and acetone, dissolving completely, concentrating to dryness under reduced pressure, adding acetone, heating for complete dissolution, adding water, naturally cooling to room temperature, stirring, and filtering to obtain the crystal form R. Preferably, in the mixed solvent of dichloromethane and acetone, dichloromethane and acetone may be in a volume ratio of 3:1 to 9:1, e.g., 6:1; preferably, acetone added after concentrating to dryness under reduced pressure and acetone in the mixed solvent of dichloromethane and acetone may be in a volume ratio of 1:1 to 1:1.5, e.g., 4:5; preferably, water added after heating for complete dissolution and acetone added after concentrating to dryness under reduced pressure may be in a volume ratio of 0.5:1 to 1:1, e.g., 0.75:1. Preferably, the compound of formula (I) and the mixed solvent of dichloromethane and acetone may be in a mass-to-volume ratio of 1 mg:(0.3-0.5) mL, e.g., 1 mg:0.35 mL.

The present disclosure further provides a preparation method for the crystal form S, wherein the preparation method for the crystal form S comprises mixing the compound of formula (I) (such as an amorphous form, e.g., amorphous form Z) with 1,4-dioxane, dissolving completely, adding water, precipitating a solid, filtering, and drying to obtain the crystal form S. Preferably, 1,4-dioxane and water are in a volume ratio of 1:2 to 1:3, e.g., 1:2.2. Preferably, the compound of formula (I) and 1,4-dioxane may be in a mass-to-volume ratio of 1 mg:(0.08-0.15) mL, e.g., 1 mg:0.11 mL.

The present disclosure further provides a preparation method for the crystal form T, wherein the preparation method for the crystal form T comprises mixing the compound of formula (I) (such as an amorphous form, e.g., amorphous form Z) with trichloromethane to obtain a solution 1; mixing maleic acid with ethanol to obtain a solution 2; and mixing the solution 2 with the solution 1, adding methyl *tert-butyl* ether, stirring, centrifuging, and drying to obtain the crystal form T. Preferably, the compound of formula (I) and trichloromethane may be in a mass-to-volume ratio of 1 g:(12-20) mL, e.g., 1 g:(14-15) mL. Preferably, the compound of formula (I) and maleic acid may be in a mass ratio of (9-10):1. Preferably, the compound of formula (I) and ethanol may be in a mass-to-volume ratio of 1 g:(1-4) mL, e.g., 1 g:2 mL. Preferably, the solution 2 and the solution 1 may be in a volume ratio of 1:(1.2-1.3). Preferably, trichloromethane and the total amount of methyl *tert-butyl* ether may be in a volume ratio of (11-12):1.

The present disclosure further provides a preparation method for the crystal form U, wherein the preparation method for the crystal form U comprises mixing the compound of formula (I) (such as an amorphous form, e.g., amorphous form Z) with trichloromethane to obtain a solution 1; mixing methanesulfonic acid with trichloromethane to obtain a solution 2; and mixing the solution 2 with the solution 1, stirring, and drying to obtain the crystal form U. Preferably, the compound of formula (I) and trichloromethane in the solution 1 may be in a mass-to-volume ratio of 1 g:(12-20) mL, e.g., 1 g:(14-15) mL. Preferably, the compound of formula (I) and methanesulfonic acid may be in a mass ratio of (9-10):1. Preferably, the compound of formula (I) and trichloromethane in the solution 2 may be in a mass-to-volume ratio of 1 g:(1-4) mL, e.g., 1 g:2 mL. Preferably, the solution 2 and the solution 1 may be in a volume ratio of 1:(1.3-1.4).

The present disclosure further provides a mixture, wherein the mixture comprises at least one, such as one, two, three, or more, selected from the solid forms of the compound of formula (I) or the pharmaceutically acceptable salt thereof, preferably one, two, three, or more selected from the crystal forms A to U (i.e., crystal forms A, B, C, D, E, F, G, H, I, J, K, L, M, N, O, P, Q, R, S, T, and U).

According to an embodiment of the present disclosure, the mixture comprises the crystal form A and one, two, three, or more selected from the crystal forms B to U, preferably the crystal form A and one, two, three, or more selected

from the crystal forms B to U.

According to an embodiment of the present disclosure, the content of the crystal form A in percentages by weight is 80% or more, such as 90% or more, preferably 95% or more, such as 98% or more, and further preferably 99% or more, such as 99.5% or more, 99.8% or more, or 99.9% or more, based on the total weight of the compound of formula (I) in the mixture.

It will be appreciated by those skilled in the art that any one of the mixtures described above can be obtained by means of physical blending.

The present disclosure further provides a pharmaceutical composition, wherein the pharmaceutical composition comprises at least one selected from the solid forms of the compound of formula (I) or the pharmaceutically acceptable salt thereof, preferably one, two, three, or more selected from the crystal forms A to U; or the pharmaceutical composition comprises the mixture.

Alternatively, the pharmaceutical composition comprises the mixture described above. According to an embodiment of the present disclosure, the pharmaceutical composition may further comprise a pharmaceutically acceptable auxiliary material.

According to an embodiment of the present disclosure, the pharmaceutical composition further comprises at least one, such as one, two, three, or more, additional therapeutic agents.

The present disclosure further provides use of the solid form of the compound of formula (I) or the pharmaceutically acceptable salt thereof, wherein the use is selected from at least one, such as one, two, three, or more of the following:

use in inhibiting cell proliferation *in vitro* or *in vivo*; and
use in treating an RET kinase-mediated disease; and
use in inhibiting RET kinase activity; and
use in treating a cancer and/or inhibiting metastasis associated with a specific cancer; and
use in treating irritable bowel syndrome (IBS) or pain associated with IBS; and
use in providing supportive care, including preventing or minimizing a gastrointestinal condition, such as diarrhea, associated with a treatment (including chemotherapy), for a cancer patient; and
use in treating a disease or condition associated with RET; and
use in reversing or preventing acquired resistance to an anti-cancer drug; and
use in delaying and/or preventing the development of resistance to an anti-cancer drug in an individual; and
use in treating an individual suffering from a cancer and having an increased possibility of developing resistance to an anti-cancer drug.

The present disclosure further provides a method for inhibiting cell proliferation *in vitro* or *in vivo,* the method comprising contacting a cell with at least one selected from the solid forms, preferably at least one selected from the crystal forms A to U, e.g., the crystal form A and one, two, three, or more selected from the crystal forms B to U.

The present disclosure further provides a method for treating an RET kinase-mediated disease, comprising administering to a patient at least one selected from the solid forms, preferably at least one selected from the crystal forms A to U, e.g., the crystal form A and one, two, three, or more selected from the crystal forms B to U.

The present disclosure further provides a method for treating a disease or condition associated with RET in a patient in need of treatment, the method comprising administering to the patient at least one selected from the solid forms, preferably at least one selected from the crystal forms A to U, e.g., the crystal form A and one, two, three, or more selected from the crystal forms B to U.

The present disclosure further provides a method for treating a cancer and/or inhibiting metastasis associated with a specific cancer in a patient in need of treatment, the method comprising administering to the patient at least one selected from the solid forms, preferably at least one selected from the crystal forms A to U, e.g., the crystal form A and one, two, three, or more selected from the crystal forms B to U.

The present disclosure further provides a method for treating irritable bowel syndrome (IBS) and/or pain associated with IBS in a patient in need of treatment, the method comprising administering to the patient at least one selected from the solid forms, preferably at least one selected from the crystal forms A to U, e.g., the crystal form A and one, two, three, or more selected from the crystal forms B to U.

The present disclosure further provides a method for providing supportive care, including preventing or minimizing a gastrointestinal disease (such as diarrhea) associated with a treatment (including chemotherapy), for a cancer patient, the method comprising administering to the patient at least one selected from the solid forms, preferably at least one selected from the crystal forms A to U, e.g., the crystal form A and one, two, three, or more selected from the crystal forms B to U.

The present disclosure further provides use of at least one selected from the solid forms, preferably at least one selected from the crystal forms A to U, e.g., the crystal form A and one, two, three, or more selected from the crystal forms B

to U, for manufacturing a medicament for the treatment of an RET kinase-mediated disease.

The present disclosure further provides use of at least one selected from the solid forms, preferably at least one selected from the crystal forms A to U, e.g., the crystal form A and one, two, three, or more selected from the crystal forms B to U, for manufacturing a medicament for the treatment of a cancer and/or the inhibition of metastasis associated with a specific cancer.

The present disclosure further provides use of at least one selected from the solid forms, preferably at least one selected from the crystal forms A to U, e.g., the crystal form A and one, two, three, or more selected from the crystal forms B to U, for manufacturing a medicament for the treatment of irritable bowel syndrome (IBS) or pain associated with IBS.

The present disclosure further provides use of at least one selected from the solid forms, preferably at least one selected from the crystal forms A to U, e.g., the crystal form A and one, two, three, or more selected from the crystal forms B to U, for manufacturing a medicament for providing supportive care, including preventing or minimizing a gastrointestinal condition, such as diarrhea, associated with a treatment (including chemotherapy), for a cancer patient.

The present disclosure further provides use of at least one selected from the solid forms, preferably at least one selected from the crystal forms A to U, e.g., the crystal form A and one, two, three, or more selected from the crystal forms B to U, for manufacturing a medicament for the inhibition of RET kinase activity.

The present disclosure further provides use of at least one selected from the solid forms, preferably at least one selected from the crystal forms A to U, e.g., the crystal form A and one, two, three, or more selected from the crystal forms B to U, for manufacturing a medicament for the treatment of a disease or condition associated with RET.

The present disclosure further provides a method for treating a cancer in a patient in need, the method comprising: (a) determining whether the cancer is associated with the dysregulation of: RET gene, RET kinase, or the expression, activity, or level of either (such as a cancer associated with RET); and (b) if it is determined that the cancer is associated with the dysregulation of: RET gene, RET kinase, or the expression, activity, or level of either (such as a cancer associated with RET), administering to the patient at least one selected from the solid forms, preferably at least one selected from the crystal forms A to U, e.g., the crystal form A and one, two, three, or more selected from the crystal forms B to U.

The present disclosure further provides a method for reversing or preventing acquired resistance to an anti-cancer drug, the method comprising administering to a patient at risk of developing or having acquired resistance to other anti-cancer drugs at least one selected from the solid forms, preferably at least one selected from the crystal forms A to U, e.g., the crystal form A and one, two, three, or more selected from the crystal forms B to U.

The present disclosure further provides a method for delaying and/or preventing the development of resistance to an anti-cancer drug in an individual, the method comprising administering to the individual at least one selected from the solid forms, preferably at least one selected from the crystal forms A to U, e.g., the crystal form A and one, two, three, or more selected from the crystal forms B to U, and administering other anti-cancer drugs before, during, or after the administration.

The present disclosure further provides a method for treating an individual suffering from a cancer and having an increased possibility of developing resistance to an anti-cancer drug, the method comprising co-administering to the individual (a) at least one selected from the solid forms, preferably at least one selected from the crystal forms A to U, e.g., the crystal form A and one, two, three, or more selected from the crystal forms B to U; and (b) other anti-cancer drugs.

The present disclosure further provides a method for treating an individual suffering from a cancer associated with RET, wherein the cancer has one or more RET inhibitor resistance mutations that increase the resistance of the cancer to an RET inhibitor other than at least one of the compound represented by formula I or the pharmaceutically acceptable salt thereof (such as substitutions at amino acid positions 804, 810, and 904, e.g., V804M, V804L, V804E, G810R, G810S, G810C, G810V, and S904F), and the method comprises administering at least one selected from the solid forms, preferably at least one selected from the crystal forms A to U, e.g., the crystal form A and one, two, three, or more selected from the crystal forms B to U, before, during, or after administering an additional anti-cancer drug.

The present disclosure further provides a method for treating an individual suffering from a cancer associated with RET, the method comprising administering at least one selected from the solid forms, preferably at least one selected from the crystal forms A to U, e.g., the crystal form A and one, two, three, or more selected from the crystal forms B to U, before, during, or after administering an additional anti-cancer drug.

The present disclosure provides a method for treating a cancer (e.g., a cancer associated with RET) in a patient in need, the method comprising administering to the patient at least one selected from the solid forms, preferably at least one selected from the crystal forms A to U, e.g., the crystal form A and one, two, three, or more selected from the crystal forms B to U. According to an embodiment of the present disclosure, when at least one selected from the solid forms, preferably at least one selected from the crystal forms A to U, e.g., the crystal form A and one, two, three, or more selected from the crystal forms B to U, is used for the treatment, administration, or manufacture of a medicament, the amount of the solid form (e.g., at least one selected from the crystal forms A to U, e.g., the crystal form A and one, two, three, or more selected from the crystal forms B to U) is preferably a therapeutically effective amount.

## Beneficial Effects

The solid forms of the fused ring compound provided in the present disclosure are not disclosed or taught in the prior art, providing the compound of formula (I) with a more advantageous pharmaceutically acceptable form. The solid forms provided in the present disclosure, particularly the crystal form A, have excellent physicochemical and pharmacokinetic properties, are beneficial for improving formulation properties and shelf-life storage, and bring more beneficial therapeutic effects to patients.

## BRIEF DESCRIPTION OF THE DRAWINGS

FIG. 1A shows an X-ray powder diffraction (XRPD) pattern of the crystal form A of the present disclosure.
FIG. 1B shows a differential scanning calorimetry (DSC) profile of the crystal form A of the present disclosure.
FIG. 1C shows a thermogravimetric analysis (TGA) profile of the crystal form A of the present disclosure.
FIG. 1D shows a $^1$H NMR spectrum of the crystal form A of the present disclosure.
FIG. 2A shows an X-ray powder diffraction (XRPD) pattern of the crystal form B of the present disclosure.
FIG. 2B shows a differential scanning calorimetry (DSC) profile and a thermogravimetric analysis (TGA) profile of the crystal form B of the present disclosure.
FIG. 2C shows a $^1$H NMR spectrum of the crystal form B of the present disclosure.
FIG. 3A shows an X-ray powder diffraction (XRPD) pattern of the crystal form C of the present disclosure.
FIG. 3B shows a differential scanning calorimetry (DSC) profile and a thermogravimetric analysis (TGA) profile of the crystal form C of the present disclosure.
FIG. 3C shows a $^1$H NMR spectrum of the crystal form C of the present disclosure.
FIG. 4A shows an X-ray powder diffraction (XRPD) pattern of the crystal form D of the present disclosure.
FIG. 4B shows a differential scanning calorimetry (DSC) profile and a thermogravimetric analysis (TGA) profile of the crystal form D of the present disclosure.
FIG. 4C shows a $^1$H NMR spectrum of the crystal form D of the present disclosure.
FIG. 4D shows a variable-temperature XRPD overlay of the crystal form D of the present disclosure.
FIG. 5A shows an X-ray powder diffraction (XRPD) pattern of the crystal form E of the present disclosure.
FIG. 5B shows a differential scanning calorimetry (DSC) profile and a thermogravimetric analysis (TGA) profile of the crystal form E of the present disclosure.
FIG. 5C shows a $^1$H NMR spectrum of the crystal form E of the present disclosure.
FIG. 5D shows a variable-temperature XRPD overlay of the crystal form E of the present disclosure.
FIG. 5E shows a $^1$H NMR spectrum of the crystal form E of the present disclosure after heating.
FIG. 6A shows an X-ray powder diffraction (XRPD) pattern of the crystal form F of the present disclosure.
FIG. 6B shows a differential scanning calorimetry (DSC) profile and a thermogravimetric analysis (TGA) profile of the crystal form F of the present disclosure.
FIG. 6C shows a $^1$H NMR spectrum of the crystal form F of the present disclosure.
FIG. 6D shows a variable-temperature XRPD overlay of the crystal form F of the present disclosure.
FIG. 6E shows a $^1$H NMR spectrum of the crystal form F of the present disclosure after heating.
FIG. 7A shows an X-ray powder diffraction (XRPD) pattern of the crystal form G of the present disclosure.
FIG. 7B shows a differential scanning calorimetry (DSC) profile and a thermogravimetric analysis (TGA) profile of the crystal form G of the present disclosure.
FIG. 7C shows a $^1$H NMR spectrum of the crystal form G of the present disclosure.
FIG. 7D shows a $^1$H NMR spectrum of the crystal form G of the present disclosure after heating.
FIG. 8A shows an X-ray powder diffraction (XRPD) pattern of the crystal form H of the present disclosure.
FIG. 8B shows a differential scanning calorimetry (DSC) profile and a thermogravimetric analysis (TGA) profile of the crystal form H of the present disclosure.
FIG. 8C shows a $^1$H NMR spectrum of the crystal form H of the present disclosure.
FIG. 8D shows a variable-temperature XRPD overlay of the crystal form H of the present disclosure.
FIG. 9 shows an X-ray powder diffraction (XRPD) pattern of the crystal form I of the present disclosure.
FIG. 10A shows an X-ray powder diffraction (XRPD) pattern of the crystal form J of the present disclosure.
FIG. 10B shows a differential scanning calorimetry (DSC) profile and a thermogravimetric analysis (TGA) profile of the crystal form J of the present disclosure.
FIG. 10C shows a $^1$H NMR spectrum of the crystal form J of the present disclosure.
FIG. 10D shows a variable-temperature XRPD overlay of the crystal form J of the present disclosure.
FIG. 10E shows a $^1$H NMR spectrum of the crystal form J of the present disclosure after heating.

FIG. 11A shows an X-ray powder diffraction (XRPD) pattern of the crystal form K of the present disclosure.
FIG. 11B shows a differential scanning calorimetry (DSC) profile and a thermogravimetric analysis (TGA) profile of the crystal form K of the present disclosure.
FIG. 11C shows a $^1$H NMR spectrum of the crystal form K of the present disclosure.
FIG. 11D shows an XRPD overlay of the crystal form obtained by repeating the method in Example 12.
FIG. 12A shows an X-ray powder diffraction (XRPD) pattern of the crystal form L of the present disclosure.
FIG. 12B shows a differential scanning calorimetry (DSC) profile and a thermogravimetric analysis (TGA) profile of the crystal form L of the present disclosure.
FIG. 12C shows a $^1$H NMR spectrum of the crystal form L of the present disclosure.
FIG. 12D shows a schematic asymmetric unit diagram of a monocrystal structure of the crystal form L of the present disclosure.
FIG. 13A shows an X-ray powder diffraction (XRPD) pattern of the crystal form M of the present disclosure.
FIG. 13B shows a differential scanning calorimetry (DSC) profile and a thermogravimetric analysis (TGA) profile of the crystal form M of the present disclosure.
FIG. 14A shows an X-ray powder diffraction (XRPD) pattern of the crystal form N of the present disclosure.
FIG. 14B shows a differential scanning calorimetry (DSC) profile and a thermogravimetric analysis (TGA) profile of the crystal form N of the present disclosure.
FIG. 14C shows a $^1$H NMR spectrum of the crystal form N of the present disclosure.
FIG. 14D shows a variable-temperature XRPD overlay of the crystal form N of the present disclosure.
FIG. 15A shows an X-ray powder diffraction (XRPD) pattern of the crystal form O of the present disclosure.
FIG. 15B shows a differential scanning calorimetry (DSC) profile and a thermogravimetric analysis (TGA) profile of the crystal form O of the present disclosure.
FIG. 15C shows a $^1$H NMR spectrum of the crystal form O of the present disclosure.
FIG. 15D shows a variable-temperature XRPD overlay of the crystal form O of the present disclosure.
FIG. 15E shows a $^1$H NMR spectrum of the crystal form O of the present disclosure after heating.
FIG. 16A shows an X-ray powder diffraction (XRPD) pattern of the crystal form P of the present disclosure.
FIG. 16B shows an XRPD overlay of the crystal form P of the present disclosure before and after drying.
FIG. 17 shows an X-ray powder diffraction (XRPD) pattern of the crystal form Q of the present disclosure.
FIG. 18 shows a DVS plot of the crystal form A in the hygroscopicity test of Test Example 1.
FIG. 19 shows a DVS plot of the crystal form B in the hygroscopicity test of Test Example 1.
FIG. 20A shows an XRPD overlay of the solid after suspension competition in Test Example 4.
FIG. 20B shows an XRPD overlay of the solid after suspension competition in Test Example 4.
FIG. 21A shows an X-ray powder diffraction (XRPD) pattern of the amorphous form Z of the compound of formula (I) in Example 1.
FIG. 21B shows a differential scanning calorimetry (DSC) profile of the amorphous form Z of the compound of formula (I) in Example 1.
FIG. 21C shows a thermogravimetric analysis (TGA) profile of the amorphous form Z of the compound of formula (I) in Example 1.
FIG. 22A shows an X-ray powder diffraction (XRPD) pattern of the crystal form R of the present disclosure.
FIG. 22B shows a DVS test plot of the crystal form R in Test Example 1.
FIG. 22C shows an isothermal adsorption curve of the crystal form R in Test Example 1.
FIG. 23 shows an X-ray powder diffraction (XRPD) pattern of the crystal form S of the present disclosure.
FIG. 24A shows an X-ray powder diffraction (XRPD) pattern of the crystal form T of the present disclosure.
FIG. 24B shows a $^1$H NMR spectrum of the crystal form T of the present disclosure.
FIG. 25A shows an X-ray powder diffraction (XRPD) pattern of the crystal form U of the present disclosure.
FIG. 25B shows a $^1$H NMR spectrum of the crystal form U of the present disclosure.

## DETAILED DESCRIPTION

The technical solutions of the present disclosure will be further explained in detail by the description of the following specific examples. The following examples are merely exemplary illustrations and explanations of the present disclosure, and should not be construed as limiting the protection scope of the present disclosure. All techniques implemented based on the content of the present disclosure described above are encompassed within the protection scope of the present disclosure.

Unless otherwise stated, the starting materials and reagents used in the following examples are all commercially available products or can be prepared by using known methods.

Unless otherwise stated, the instruments and detection methods described below are adopted in the following examples.

I. Detection instruments and methods

X-ray powder diffraction (XRPD)

XRPD results were acquired on a PANalytical Empyrean and X'Pert$^3$ X-ray powder diffraction analyzer, and the scanning parameters are shown in the table below:

| Model of instrument | X' Pert$^3$ (reflection) | Empyrean (variable-temperature) |
|---|---|---|
| X-ray | Cu, kα; Kα1 (Å): 1.540598, Kα2 (Å): 1.544426<br>Intensity ratio Kα2/Kα1: 0.50 | Cu, kα; Kα1 (Å): 1.540598, Kα2 (Å): 1.544426<br>Intensity ratio Kα2/Kα1: 0.50 |
| Settings of X-ray tube | 45 kV, 40 mA | 45 kV, 40 mA |
| Divergence slit | 1/8° | Automatic |
| Scanning mode | Continuous | Continuous |
| Scanning range (°2TH) | 3° ~ 40° | 3° ~ 40° |
| Scanning step length (°2TH) | 0.0263 | 0.0167 |
| Scanning time per step (s) | 46.665 | 17.780/33.020 |
| Scanning time (s) | 5 min 03 s | 10 min 13 s |

Thermogravimetric analysis (TGA) and differential scanning calorimetry (DSC)

TGA and DSC profiles were acquired on a TA Discovery 5500 thermogravimetric analyzer and a TA Discovery 2500 differential scanning calorimeter, respectively, and the test parameters are shown in the table below:

| Parameter | TGA | DSC |
|---|---|---|
| Method | Linear heating | Linear heating |
| Sample tray | Aluminum tray, uncovered | Aluminum tray, with cover |
| Temperature range | RT-350 °C | RT-target temperature |
| Rate of heating | 10 °C/min | 10 °C/min |
| Protective gas | Nitrogen | Nitrogen |

Dynamic vapor sorption (DVS)

Dynamic vapor sorption (DVS) curves were acquired on a DVS Intrinsic in SMS (Surface Measurement Systems). The relative humidity at 25 °C was corrected with the deliquescence points of LiCl, $Mg(NO_3)_2$, and KCl. The DVS test parameters are shown in the table below:

| Parameter | DVS |
|---|---|
| Temperature | 25 °C |
| Sample amount | 20 ~ 40 mg |
| Protective gas and flow rate | $N_2$, 200 mL/min |
| dm/dt | 0.002%/min |
| Minimum dm/dt equilibration time | 10 min |
| Maximum equilibration time | 180 min |
| RH range | 0%RH ~ 95%RH<br>0%RH ~ 95%RH~0%RH |
| RH gradient | 10%RH (0%RH ~ 90%RH & 90%RH ~ 0%RH)<br>5%RH (90%RH ~ 95%RH & 95%RH ~ 90%RH) |

$^1H$ solution nuclear magnetic resonance ($^1H$ Solution NMR)

The $^1H$ solution nuclear magnetic resonance spectra were acquired on a Bruker 400M nuclear magnetic resonance spectrometer using DMSO-$d_6$ as a solvent.

II. Correspondence table of solvent names

| In English | In Chinese | In English | In Chinese |
|---|---|---|---|
| MeOH | Methanol | MTBE | Methyl *tert*-butyl ether |
| EtOH | Ethanol | THF | Tetrahydrofuran |
| IPA | Isopropanol | 2-MeTHF | 2-Methyltetrahydrofuran |
| Acetone | Acetone | CPME | Cyclopentyl methyl ether |
| $CHCl_3$ | Chloroform | ACN | Acetonitrile |
| MIBK | Methyl isobutyl ketone | n-Heptane | *n*-Heptane |
| EtOAc | Ethyl acetate | Toluene | Toluene |
| IPAc | Isopropyl acetate | $H_2O$ | Water |
| DMSO | Dimethylsulfoxide | DCM | Dichloromethane |
| Anisole | Anisole | 1,4-Dioxane | 1,4-Dioxane |
| MEK | Methyl ethyl ketone | DMF | Dimethylformamide |
| Cumene | Cumene | n-Hexane | *n*-Hexane |
| NMP | *N*-Methylpyrrolidone | | |

Example 1: Preparation of Amorphous Form of Compound of Formula (I)

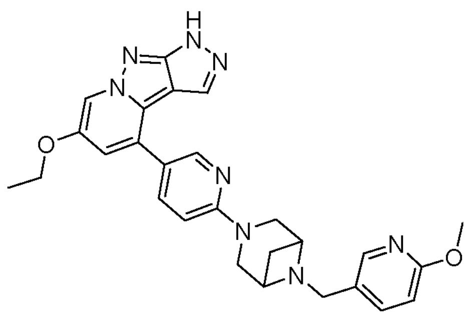

**(I)**

**According** to the method in Example 9 of the PCT patent application with the application No. PCT/CN2020/107049, 2-iodoethane (47 mg, 0.3 mmol) and potassium carbonate (83 mg, 0.6 mmol) were added to a solution of 4-(6-(6-((6-methoxypyridin-3-)methylene)3,6-diazabicyclo[3.1.1]heptan-3-yl)pyridin-3-yl)-1*H*-pyrazolo [3',4':3,4]pyrazolo[1,5-a]pyridin-6-ol (150 mg, 0.3 mmol) in DMF (10.0 mL), and the mixture was heated to 60 °C and reacted for 12 h. Water was added, and the resulting solution was extracted with ethyl acetate. The organic phases were combined and washed with water, dried over anhydrous sodium sulfate, filtered, concentrated under reduced pressure, and separated by column chromatography. The solvent was removed by rotary evaporation to obtain the compound of formula (I) described herein as amorphous form Z. The amorphous form Z has an X-ray powder diffraction (XRPD) pattern shown in FIG. 21A, a differential scanning calorimetry (DSC) profile shown in FIG. 21B, and a thermogravimetric analysis (TGA) profile shown in FIG. 21C.

Example 2: Preparation of Anhydrous Crystal Form A

20 mg of the amorphous form Z of the compound of formula (I) prepared in Example 1 was added to a 20-mL vial, and a solvent of a mixed solvent of ethyl acetate and dichloromethane (9:1, v:v) was added to dissolve the solid. After filtration through a filter membrane, the resulting clear filtrate was sealed with a sealing film, and 5 small holes were made on the film. The vial was then placed at room temperature for slow volatilization. The solid obtained by volatilization was collected to obtain the crystal form A. This crystal form has an X-ray powder diffraction pattern shown in FIG. 1A, a

differential scanning calorimetry (DSC) profile, which was tested after the sample was heated in advance to remove the adsorbed solvent on the surface, shown in FIG. 1B, a thermogravimetric analysis (TGA) profile shown in FIG. 1C, and a $^1$H NMR spectrum shown in FIG. 1D.

The crystal form A sample was purged under $N_2$ atmosphere for 20 min, heated to 150 °C, and cooled to 30 °C, with no crystal form changes observed during the process. Combined with the TGA/DSC and $^1$H NMR results of the sample, the crystal form A was determined to be an anhydrous crystal form.

Example 3: Preparation of Anhydrous Crystal Form B

20 mg of the crystal form L prepared according to the method in Example 13 was weighed into an HPLC vial, and 0.5 mL of solvent acetone was added. The resulting suspension was magnetically stirred (at ~750 rpm) at 60 °C for about 6 days, and a solid was separated out by centrifugation to obtain the crystal form B. This crystal form has an X-ray powder diffraction pattern shown in FIG. 2A, a differential scanning calorimetry (DSC) profile and a thermogravimetric analysis (TGA) profile shown in FIG. 2B, and a $^1$H NMR spectrum shown in FIG. 2C.

The $^1$H NMR results showed that a small amount (0.2 wt%) of acetone residue was observed in this sample. Based on the relatively small weight loss (0.5 wt%) of the sample before decomposition in TGA and the absence of distinct endothermic peaks in DSC before the sample was melted, the crystal form B was determined to be an anhydrous crystal form.

Example 4: Preparation of Anhydrous Crystal Form C

20 mg of the crystal form L prepared according to the method in Example 13 was weighed into an HPLC vial, and 0.5 mL of solvent acetone was added. The resulting mixture was stirred at room temperature for 30 min, heated to 60 °C and stirred for 30 min, and then cooled to 0-5 °C and stirred for 6 h, and a solid was separated out by centrifugation. 0.5 mL of solvent water was added, and the resulting suspension was magnetically stirred (at ~750 rpm) at room temperature for about 2 weeks. A solid was separated out by centrifugation to obtain the crystal form C. This crystal form has an X-ray powder diffraction pattern shown in FIG. 3A, a differential scanning calorimetry (DSC) profile and a thermogravimetric analysis (TGA) profile, which were tested after the sample was heated in advance to remove the adsorbed solvent/water on the surface, shown in FIG. 3B, and a $^1$H NMR spectrum shown in FIG. 3C.

The crystal form C sample was purged under $N_2$ atmosphere for 20 min, heated to 100 °C, and cooled to 30 °C, with no crystal form changes observed during the process. Combined with the TGA/DSC and $^1$H NMR results of the sample, the crystal form C was determined to be an anhydrous crystal form.

In addition, the solvent in the method described above was replaced with a mixed solvent of acetone and water (6:4, v:v), and the crystal form C was also obtained.

Example 5: Preparation of Hydrate Crystal Form D

20 mg of the crystal form L prepared according to the method in Example 13 was weighed into an HPLC vial, and 0.5 mL of solvent acetone was added. The resulting mixture was stirred at room temperature for 30 min, heated to 60 °C and stirred for 30 min, and then cooled to 0-5 °C and stirred for 6 h, and a solid was separated out by centrifugation. 0.5 mL of a mixed solvent of acetone and water (86:14, v/v) was added, and the resulting suspension was magnetically stirred (at ~750 rpm) at room temperature for about 9 days. A solid was separated out by centrifugation to obtain the crystal form D. This crystal form has an X-ray powder diffraction pattern shown in FIG. 4A, a differential scanning calorimetry (DSC) profile and a thermogravimetric analysis (TGA) profile shown in FIG. 4B, and a $^1$H NMR spectrum shown in FIG. 4C. The $^1$H NMR results showed that the crystal form D sample contained very little (~0.5 wt%) acetone residue.

In addition, the solvent in the method described above was replaced with a mixed solvent of acetone and water (4:1, v/v), and the crystal form D was also obtained.

The variable-temperature XRPD results (FIG. 4D) showed that the crystal form D sample was purged under $N_2$ atmosphere for 20 min, and changes in diffraction peaks were observed; when the sample was heated to 150 °C and cooled to 30 °C, a crystal form change was observed, and the obtained new crystal form was a crystal form Q; after the sample was heated to 150 °C, cooled to room temperature, and exposed to air, changes in some diffraction peaks were observed, and the obtained new crystal form was a crystal form M. Combined with the crystal form change of the crystal form D sample observed after $N_2$ purging and heating, the DSC results showed a relatively wide endothermic signal (at a peak temperature of 63.7 °C) before the melting peak, and the $^1$H NMR results showed that the sample contained only very little acetone residue (far less than TGA weight loss). Thus, the crystal form D was determined to be a hydrate.

Example 6: Preparation of Methanol Solvate Crystal Form E

20 mg of the crystal form L prepared according to the method in Example 13 was weighed into an HPLC vial, and 0.5 mL of solvent MeOH was added. The resulting suspension was magnetically stirred at room temperature overnight, and a solid was separated out by centrifugation to obtain the crystal form E. This crystal form has an X-ray powder diffraction pattern shown in FIG. 5A, a differential scanning calorimetry (DSC) profile and a thermogravimetric analysis (TGA) profile shown in FIG. 5B, and a $^1$H NMR spectrum shown in FIG. 5C. The $^1$H NMR results (FIG. 5C) showed that 2.5 wt% MeOH was detected (the molar ratio of MeOH to the compound of formula (I) was 0.4:1).

The crystal form E was identified through a heating test, and the results are shown in FIG. 5D. When the crystal form E was heated to 48 °C, cooled to room temperature, and exposed to air, no crystal form changes were observed; when the sample was heated to 100 °C, cooled to room temperature, and exposed to air, the new crystal form observed was a crystal form N; when the sample was further heated to 115 °C, cooled to room temperature, and exposed to air, the formation of an amorphous form was observed. Combining the $^1$H NMR results of the sample after the crystal form E was heated to 48 °C (FIG. 5E, the solvent content was not significantly changed compared to the sample before heating) with the TGA/DSC results, this crystal form was determined to be a MeOH solvate.

Example 7: Preparation of Ethanol Solvate Crystal Form F

20 mg of the amorphous form Z prepared according to the method in Example 1 was weighed into an HPLC vial, and 0.5 mL of a mixed solvent of acetone and ethanol (1:1, v:v) was added. The resulting suspension was magnetically stirred (at ~750 rpm) at room temperature for about 4 days, and a solid was separated out by centrifugation to obtain the crystal form F. This crystal form has an X-ray powder diffraction pattern shown in FIG. 6A, a differential scanning calorimetry (DSC) profile and a thermogravimetric analysis (TGA) profile shown in FIG. 6B, and a $^1$H NMR spectrum shown in FIG. 6C.

The $^1$H NMR analysis detected about 2.1 wt% ethanol residue (the molar ratio of ethanol to the compound of formula (I) was 0.23:1) and about 0.2 wt% acetone residue. The crystal form F was identified through a heating test, and the results are shown in FIG. 6D. When the crystal form F was heated to 100 °C, cooled to room temperature, and exposed to air, no crystal form changes were observed; when the sample was heated to 160 °C, cooled to room temperature, and exposed to air, a transformation to the crystal form M was observed. Combining the $^1$H NMR results of the sample after the crystal form F was heated to 100 °C (FIG. 6E, the solvent content was not significantly changed compared to the sample before heating) with the TGA/DSC results, this crystal form was determined to be an ethanol solvate.

Example 8: Preparation of Acetonitrile Solvate Crystal Form G

20 mg of the crystal form L prepared according to the method in Example 13 was weighed into an HPLC vial, and 0.5 mL of solvent acetone was added. The resulting mixture was stirred at room temperature for 30 min, heated to 60 °C and stirred for 30 min, and then cooled to 0-5 °C and stirred for 6 h, and a solid was separated out by centrifugation. 0.5 mL of ACN was added, and the resulting suspension was magnetically stirred (at ~750 rpm) at 50 °C for about 4 days. A solid was separated out by centrifugation to obtain the crystal form G. This crystal form has an X-ray powder diffraction pattern shown in FIG. 7A, a differential scanning calorimetry (DSC) profile and a thermogravimetric analysis (TGA) profile shown in FIG. 7B, and a $^1$H NMR spectrum shown in FIG. 7C.

The crystal form G was identified through a heating test, and the results showed that when the free crystal form G sample was heated to 75 °C, cooled to room temperature, and exposed to air, no crystal form changes were observed; when the sample was further heated to 150 °C, cooled to room temperature, and exposed to air, a transformation to the crystal form A was observed. Combining the $^1$H NMR results of the sample after the crystal form G was heated to 75 °C (FIG. 7D, the solvent content was not changed compared to the sample before heating) with the TGA/DSC results, this crystal form was determined to be an acetonitrile solvate.

Example 9: Preparation of Dichloromethane Solvate Crystal Form H

20 mg of the crystal form L prepared according to the method in Example 13 was weighed into an HPLC vial, and 0.5 mL of solvent acetone was added. The resulting mixture was stirred at room temperature for 30 min, heated to 60 °C and stirred for 30 min, and then cooled to 0-5 °C and stirred for 6 h, and a solid was separated out by centrifugation and subjected to gas-solid diffusion in dichloromethane for ~12 days to obtain the crystal form H. This crystal form has an X-ray powder diffraction pattern shown in FIG. 8A, a differential scanning calorimetry (DSC) profile and a thermogravimetric analysis (TGA) profile shown in FIG. 8B, and a $^1$H NMR spectrum shown in FIG. 8C.

The $^1$H NMR results showed that 6.1 wt% dichloromethane residue was detected (the molar ratio of dichloromethane to the compound of formula (I) was 0.38:1). The free crystal form H was identified through a heating test, and the

results are shown in FIG. 8D. When the free crystal form H was heated to 125 °C, cooled to room temperature, and exposed to air, a sample with low crystallinity was observed; when the sample was further heated to 140 °C and 172 °C, the formation of an amorphous sample was observed. Combined with the TGA/DSC and $^1$H NMR results of this sample, this crystal form was determined to be a dichloromethane solvate.

Example 10: Preparation of Crystal Form I

20 mg of the amorphous form Z prepared according to the method in Example 1 was weighed into a 3-mL vial. The solid was dissolved in 1.0-1.5 mL of solvent NMP (N-methylpyrrolidone), and the resulting mixture was filtered through a filter membrane to obtain a clear solution. About 4 mL of anti-solvent ACN was added to a 20-mL vial. The uncovered 3-mL vial containing the filtrate was placed into the 20-mL vial, and then the 20-mL vial was sealed and allowed to stand at room temperature. The solid was precipitated and collected to obtain the crystal form I. The X-ray powder diffraction pattern of this crystal form is shown in FIG. 9. However, the crystal form I was transformed to the crystal form A after drying at room temperature.

Example 11: Preparation of 1,4-Dioxane Solvate Crystal Form J

20 mg of the crystal form L prepared according to the method in Example 13 was weighed into an HPLC vial, and 0.5 mL of solvent acetone was added. The resulting mixture was stirred at room temperature for 30 min, heated to 60 °C and stirred for 30 min, and then cooled to 0-5 °C and stirred for 6 h, and a solid was separated out by centrifugation. 0.5 mL of solvent 1,4-dioxane was added. The resulting suspension was magnetically stirred (at ~750 rpm) at 50 °C to obtain a clear solution, and then the clear solution was successively stirred at room temperature for about 4 days. A solid was separated out by centrifugation to obtain the crystal form J. This crystal form has an X-ray powder diffraction pattern shown in FIG. 10A, a differential scanning calorimetry (DSC) profile and a thermogravimetric analysis (TGA) profile shown in FIG. 10B, and a $^1$H NMR spectrum shown in FIG. 10C.

$^1$H NMR detected that the sample contained 3.0 wt% 1,4-dioxane (the molar ratio of 1,4-dioxane to the compound of formula (I) was 0.18:1). The crystal form J was identified through a heating test, and the results are shown in FIG. 10D. When the crystal form J sample was heated to 100 °C, cooled to room temperature, and exposed to air, no crystal form changes were observed; when the sample was further heated to 160 °C, the formation of a sample with low crystallinity was observed. Combining the $^1$H NMR results of the sample after the free crystal form J was heated to 100 °C (FIG. 10E, the solvent content was not changed compared to the sample before heating) with the TGA/DSC results, this crystal form was determined to be a 1,4-dioxane solvate.

Example 12: Preparation of Crystal Form K

20 mg of the crystal form L prepared according to the method in Example 13 was weighed into an HPLC vial, and 0.5 mL of solvent acetone was added. The resulting mixture was stirred at room temperature for 30 min, heated to 60 °C and stirred for 30 min, and then cooled to 0-5 °C and stirred for 6 h, and a solid was separated out by centrifugation. A solvent of a mixed solvent of MeOH and $H_2O$ (4:1, v:v) was added to dissolve the solid. After filtration through a filter membrane, the resulting clear filtrate was sealed with a sealing film, and 5 small holes were made on the film. The vial was then placed at room temperature for slow volatilization. The solid obtained by volatilization was collected to obtain the crystal form K. This crystal form has an X-ray powder diffraction pattern shown in FIG. 11A, a differential scanning calorimetry (DSC) profile and a thermogravimetric analysis (TGA) profile shown in FIG. 11B, and a $^1$H NMR spectrum shown in FIG. 11C.

The $^1$H NMR results showed that the sample contained 0.9 wt% MeOH. However, repeating the method described above did not reliably prepare the crystal form K and occasionally may result in the crystal form O or the crystal form L (FIG. 11D).

Example 13: Preparation of Methanol-Water Co-Solvate Crystal Form L

About 20 mg of the amorphous form Z prepared according to the method in Example 1 was weighed into a 20-mL vial, and a solvent of a mixed solvent of methanol and water (4:1, v:v) was added to dissolve the solid. After filtration through a filter membrane, the resulting clear filtrate was sealed with a sealing film, and 5 small holes were made on the film. The vial was then placed at room temperature for slow volatilization. The solid obtained by volatilization was collected to obtain the crystal form L. This crystal form has an X-ray powder diffraction pattern shown in FIG. 12A, a differential scanning calorimetry (DSC) profile and a thermogravimetric analysis (TGA) profile shown in FIG. 12B, and a $^1$H NMR spectrum shown in FIG. 12C.

The $^1$H NMR results showed that the sample contained 6.1 wt% methanol residue (the molar ratio of methanol to

the compound of formula (I) was 1:1). This sample was used for monocrystal structure analysis, and the analysis result showed that the free crystal form L was a methanol-water co-solvate (FIG. 12D).

Example 14: Preparation of Hydrate Crystal Form M

About 20 mg of the crystal form D prepared according to the method in Example 5 was weighed out, heated to 150 °C, then cooled to room temperature, and exposed to air to obtain the crystal form M. This crystal form has an X-ray powder diffraction pattern shown in FIG. 13A, as well as a differential scanning calorimetry (DSC) profile and a thermogravimetric analysis (TGA) profile shown in FIG. 13B.

Example 15: Preparation of Anhydrous Crystal Form N

About 20 mg of the crystal form E prepared according to the method in Example 6 was weighed out, heated to 100 °C, then cooled to room temperature, and exposed to air to obtain the crystal form N. This crystal form has an X-ray powder diffraction pattern shown in FIG. 14A, a differential scanning calorimetry (DSC) profile and a thermogravimetric analysis (TGA) profile, which were obtained after heating to 100 °C to remove the adsorbed water/solvent on the surface, shown in FIG. 14B, and a $^1H$ NMR spectrum shown in FIG. 14C.

The $^1H$ NMR results showed no methanol residue in the crystal form N sample. As shown in the variable-temperature XRPD overlay of the crystal form E in FIG. 14D, the crystal form N can be obtained by heating the crystal form E under $N_2$ atmosphere to remove methanol, and thus, the crystal form N was determined to be an anhydrous crystal form.

Example 16: Preparation of Methanol Solvate Crystal Form O

About 20 mg of the amorphous form Z prepared according to the method in Example 1 was weighed out, completely dissolved in MeOH/$H_2O$ (4:1, v/v), and placed at room temperature for slow volatilization to obtain the crystal form O. This crystal form has an X-ray powder diffraction pattern shown in FIG. 15A, a differential scanning calorimetry (DSC) profile and a thermogravimetric analysis (TGA) profile shown in FIG. 15B, and a $^1H$ NMR spectrum shown in FIG. 15C.

The $^1H$ NMR results showed that 1.8 wt% methanol was detected. The crystal form O was identified through a heating test, and the results are shown in FIG. 15D. When the crystal form O sample was heated to 145 °C, cooled to room temperature, and exposed to air, shifts in diffraction peaks were observed; when the sample was further heated to 175 °C, a transformation to the free crystal form M was observed. Combining the $^1H$ NMR results of the sample after the free crystal form O was heated to 145 °C (FIG. 15E, the solvent content was not significantly changed compared to the sample before heating) with the TGA/DSC results, this crystal form was determined to be a MeOH solvate.

Example 17: Preparation of Hydrate Crystal Form P

20 mg of the crystal form L prepared according to the method in Example 13 was weighed into an HPLC vial, and 0.5 mL of solvent acetone was added. The resulting mixture was stirred at room temperature for 30 min, heated to 60 °C and stirred for 30 min, and then cooled to 0-5 °C and stirred for 6 h, and a solid was separated out by centrifugation. A mixed solvent of MEK, THF, and $H_2O$ (1:0.1:2, v:v:v) was added, and the resulting mixture was slurried at 5 °C for 4 days to obtain the crystal form P. The X-ray powder diffraction pattern of this crystal form is **shown in FIG.** 16A.

The wet sample of the crystal form P was placed in the air for about 10 min, and then a transformation to the crystal form C was observed. After drying in the air, the crystal form P was transformed to the anhydrous crystal form C. Thus, the crystal form P was determined to be a hydrate.

Example 18: Preparation of Anhydrous Crystal Form Q

About 20 mg of the crystal form D prepared according to the method in Example 5 was weighed out and purged under $N_2$ atmosphere for 20 min, and then the sample was heated to 150 °C and cooled to 30 °C to obtain the crystal form Q. The X-ray powder diffraction pattern of this crystal form is shown in FIG. 17.

As can be seen from FIG. 4D, the crystal form Q was obtained by heating the crystal form D under $N_2$ purge, and thus, the crystal form Q was determined to be an anhydrous crystal form. Meanwhile, the crystal form Q was transferred to the hydrate crystal form M after being placed at room temperature and room humidity.

Example 18: Preparation of Crystal Form R

100 mg of the amorphous form Z prepared according to the method in Example 1 was weighed into a vial, and 35

mL of a mixed solvent of dichloromethane and acetone (6:1, v:v) was added. The mixture was stirred for complete dissolution and concentrated to dryness under reduced pressure again. 4 mL of acetone was added, and the mixture was heated to 60 °C for complete dissolution. About 3 mL of purified water was then added, and the mixture was naturally cooled to room temperature, successively stirred for 2 h, and filtered to obtain the crystal form R. The X-ray powder diffraction pattern of this crystal form is shown in FIG. 22A, and this crystal form is an anhydrate.

Example 19: Preparation of Crystal Form S

100 mg of the amorphous form Z prepared according to the method in Example 1 was weighed into a vial, and 5 mL of 1,4-dioxane was added. The mixture was stirred at 35 °C for complete dissolution. 11 mL of water was added at room temperature, and a solid was precipitated. The mixture was stirred for 1 h and then filtered. The filter cake was rinsed with about 5 mL of water and dried at 25 °C overnight to obtain the crystal form S. The X-ray powder diffraction pattern of this crystal form is shown in FIG. 23, and this crystal form is a hydrate.

Example 20: Preparation of Crystal Form T of Maleate

1 g of the amorphous form Z prepared according to the method in Example 1 was weighed into a vial, and 14 mL of trichloromethane was added; the mixture was stirred at 35 °C for complete dissolution to obtain a solution 1; 102.64 mg of maleic acid was completely dissolved in 2 mL of ethanol to obtain a solution 2; 551 μL of the solution 2 was added to 0.7 mL of the solution 1 with stirring at room temperature, and no solid was precipitated; the mixture was stirred for 5 h and then placed at 4-8 °C overnight, and no solid was precipitated; 0.8 mL of methyl *tert-butyl* ether was added to obtain a turbid solution, and the turbid solution was stirred to obtain an oil; 0.4 mL of methyl *tert-butyl* ether was supplemented; and the mixture was successively stirred overnight, centrifuged, and dried in vacuum at 25 °C to obtain the crystal form T. The X-ray powder diffraction pattern of this crystal form is shown in FIG. 24A. As shown in FIG. 24B, decomposition occurred in the $^{1}$H-NMR analysis of this crystal form.

Example 21. Preparation of Crystal form U of Methanesulfonate

1 g of the amorphous form Z prepared according to the method in Example 1 was weighed into a vial, and 14 mL of trichloromethane was added to obtain a solution 1; 107.35 mg of methanesulfonic acid was completely dissolved in 2 mL of trichloromethane to obtain a solution 2; 441 μL of the solution 2 was added to 0.7 mL of the solution 1 with stirring at room temperature, and a small number of particles were precipitated and then dissolved after 45 min; and the mixture was stirred for 5 h to obtain a turbid solution, and the turbid solution was successively stirred for one day and dried in vacuum at 25 °C to obtain the crystal form U. The X-ray powder diffraction pattern of this crystal form is shown in FIG.25A. As shown in FIG. 25B, decomposition occurred in the $^{1}$H-NMR analysis of this crystal form.

Test Example 1: Hygroscopicity Test

The hygroscopicity of the crystal form A and crystal form B was tested by DVS test at 25 °C and 0% RH-95% RH and evaluated according to *Chinese Pharmacopoeia* 2015 Edition.

The DVS test results of the crystal form A and crystal form B are shown in FIG. 18 and FIG. 19, respectively. Under conditions of 25 °C/80% RH, the crystal form A sample exhibited a hygroscopic weight gain of about 1.1%, and the crystal form B sample exhibited a hygroscopic weight gain of about 1.0%, indicating that the crystal form A and crystal form B are both slightly hygroscopic. In addition, the XRPD results showed the crystal forms of the crystal form A and crystal form B samples were not changed after the DVS test.

The DVS test profile of the crystal form R is shown in FIG. 22B and the isothermal adsorption curve is shown in FIG. 22C. The results showed that the crystal form R and the crystal form S were easy to undergo a polymorphic transition to each other under the influence of ambient humidity, that is, the crystal form R started to significantly absorb water at 50% RH and underwent a polymorphic transition to the hydrate crystal form S, and the crystal form S started to dehydrate at 40% RH and underwent a polymorphic transition to the anhydrous crystal form R.

Test Example 2: Solid-State Stability Test

An appropriate amount of the crystal form A and crystal form B samples were separately weighed out and placed in a sealed state at 60 °C for 24 h, and in an uncovered state for 2, 4, or 6 weeks under the conditions of 25 °C/60% RH and 40 °C/75% RH. The solid samples placed under different conditions were tested for crystal form changes by XRPD and for purity by HPLC to evaluate their chemical stability.

XRPD characterization results before and after the stability test are summarized in Table 2-1 and Table 2-2, and

the results showed that HPLC purity of the crystal form A and crystal form B samples was not significantly reduced under the test conditions, and these crystal forms were not changed.

Table 2-1. Solid-state stability evaluation results for crystal form A

| Sample | Conditions | Time | Purity (area%) | Relative to initial purity (%) | Crystal form change |
|---|---|---|---|---|---|
| Crystal form A | -- | Initial | 99.96 | -- | -- |
| | 25 °C/60% RH/uncovered | 2 weeks | 99.95 | 99.99 | No change |
| | 40 °C/75% RH/uncovered | 2 weeks | 99.96 | 100.00 | No change |
| | 25 °C/60% RH/uncovered | 4 weeks | 99.94 | 99.98 | No change |
| | 40 °C/75% RH/uncovered | 4 weeks | 99.96 | 100.00 | No change |
| | 25 °C/60% RH/uncovered | 6 weeks | 99.96 | 100.00 | No change |
| | 40 °C/75% RH/uncovered | 6 weeks | 99.95 | 99.99 | No change |
| | -- | Initial | 99.96 | -- | -- |
| | 60 °C/sealed | 24 h | 99.96 | 100.00 | No change |

Table 2-2. Solid-state stability evaluation results for crystal form B

| Sample | Conditions | Time | Purity (area%) | Relative to initial purity (%) | Crystal form change |
|---|---|---|---|---|---|
| Crystal form B | -- | Initial | 99.95 | -- | -- |
| | 25 °C/60% RH/uncovered | 2 weeks | 99.96 | 100.01 | No change |
| | 40 °C/75% RH/uncovered | 2 weeks | 99.96 | 100.01 | No change |
| | 25 °C/60% RH/uncovered | 4 weeks | 99.96 | 100.01 | No change |
| | 40 °C/75% RH/uncovered | 4 weeks | 99.96 | 100.01 | No change |
| | 25 °C/60% RH/uncovered | 6 weeks | 99.96 | 100.01 | No change |
| | 40 °C/75% RH/uncovered | 6 weeks | 99.96 | 100.01 | No change |
| | -- | Initial | 99.96 | -- | -- |
| | 60 °C/sealed | 24 h | 99.97 | 100.01 | No change |

Test Example 3: Solubility Test

Test I

About 2 mg of the crystal form A sample prepared according to Example 2 was weighed out and added to an HPLC vial, and the corresponding solvent was added stepwise (50/50/200/700 μL in sequence), followed by oscillation until the solid was completely dissolved. If the sample was still not completely dissolved after 1 mL of the solvent was added, no more solvent was added. The solubility range was calculated based on the mass of the solid sample, the volume of the solvent added, and the observed dissolution results, and the results are summarized in Table 3-1.

Table 3-1. Summary of solubility test results

| Solvent | Solubility (mg/mL) | Solvent | Solubility (mg/mL) |
|---|---|---|---|
| MeOH* | S<2.1 | MTBE | S<2.2 |
| EtOH* | S<1.9 | THF | 1.9<S<6.3 |
| IPA* | S<2.0 | 2-MeTHF | 2.4<S<8.0 |
| Acetone* | S<1.9 | CPME* | S<2.0 |
| $CHCl_3$ | 20.0<S<40.0 | ACN | S<2.2 |
| MIBK | S<2.2 | n-Heptane | S<2.0 |
| EtOAc* | S<2.2 | Toluene | S<2.0 |
| IPAc | S<2.2 | $H_2O$ | S<2.3 |
| DMSO | 21.0<S<42.0 | DCM | 7.7<S<23.0 |

(continued)

| Solvent | Solubility (mg/mL) | Solvent | Solubility (mg/mL) |
|---|---|---|---|
| Anisole* | S<2.2 | 1,4-Dioxane | 2.0<S<6.7 |

*: The sample that was not completely dissolved after the room-temperature solubility test was placed at 50 °C for ~ 1 h for complete dissolution.

Test II

(1) Chromatography conditions

Chromatographic column: Waters XTERRA RP18, 4.6 mm × 150 mm, 3.5 μm or an equivalent chromatographic column.

Mobile phase: phase A was 10 mmol/L dipotassium phosphate solution (the pH value was adjusted to 9.0±0.05 with phosphoric acid); phase B was acetonitrile-methanol (85:15).

The detection wavelength was 250 nm, and the column temperature was 40 °C; the flow rate was 1.0 mL/min; the injection volume was 10 μL.

Solvent: dimethylsulfoxide-methanol = 1:9
Concentration of control: 0.1 mg/mL
Medium for solubility: water, pH 4.5 acetate buffer, and pH 6.8 phosphate buffer.

The gradient elution conditions are shown in Table 3-2.

Table 3-2

| Time (min) | 0 | 40 | 45 | 50 | 50.1 | 55 |
|---|---|---|---|---|---|---|
| Mobile phase A (%) | 80 | 35 | 20 | 20 | 80 | 80 |
| Mobile phase B (%) | 20 | 65 | 80 | 80 | 20 | 20 |

(2) Preparation of media:

Water: distilled water
pH 4.5 acetate buffer: 0.59806 g of sodium acetate trihydrate was added to a 200-mL measuring flask and dissolved in an appropriate amount of water; 1.6 mL of glacial acetic acid was added; and the mixture was diluted to volume to obtain the pH 4.5 acetate buffer.
pH 6.8 phosphate buffer: 1.38501 g of potassium dihydrogen phosphate and 0.18086 g of sodium hydroxide were added to the same 200-mL measuring flask; and the mixture was dissolved in water and diluted to volume to obtain the pH 6.8 phosphate buffer.

(3) Preparation of solutions:

The supersaturated solutions of the crystal form A and crystal form B samples were prepared by the following method:

10.0 mL of each medium was precisely measured, and the media were placed into different 25-mL measuring flasks; an appropriate amount of each test crystal form was added; and the flasks were sealed and oscillated in an oscillator at 37 °C for 24 h to obtain supersaturated solutions.

(i) Preparation of crystal form B sample solution:

pH 6.8 phosphate buffer sample solution: the supersaturated solution prepared using the pH 6.8 phosphate buffer medium was filtered to obtain the pH 6.8 phosphate buffer sample solution.

Water sample solution: the supersaturated solution prepared using the water medium was filtered to obtain the water sample solution.

pH 4.5 acetate buffer sample solution: the supersaturated solution prepared using the pH 4.5 acetate buffer medium was filtered, and 1.0 mL of the filtrate was precisely measured and placed into a 10-mL measuring flask; the filtrate was diluted to volume with the pH 4.5 acetate buffer medium, and 1.0 mL of the solution was then precisely measured and placed into the 10-mL measuring flask; and the solution was diluted to volume with methanol to obtain the pH 4.5 acetate buffer sample solution.

(ii) Preparation of crystal form A sample solution:

pH 6.8 phosphate buffer sample solution: the supersaturated solution prepared using the pH 6.8 phosphate buffer medium was filtered to obtain the pH 6.8 phosphate buffer sample solution.
Water sample solution: the supersaturated solution prepared using the water medium was filtered to obtain the water sample solution.
pH 4.5 acetate buffer sample solution: the supersaturated solution prepared using the pH 4.5 acetate buffer medium was filtered, and 1.0 mL of the filtrate was precisely measured and placed into a 10-mL measuring flask; the filtrate was diluted to volume with the pH 4.5 acetate buffer medium, and 1.0 mL of the solution was then precisely measured and placed into a 25-mL measuring flask; and the solution was diluted to volume with methanol to obtain the pH 4.5 acetate buffer sample solution.

(4) Sample determination:

Each medium sample solution was taken, 10 μL of each test sample solution was precisely measured and injected into a liquid chromatograph according to the chromatography conditions described above, and the chromatogram was recorded. The saturated solution degree of each medium was calculated according to the formula below.

$$\textbf{Saturated solubility}(\mu g/mL) = \frac{W_R \times P_R \times A_S \times D_S}{A_R \times D_R} \times 1000$$

In the formula: $W_R$ = sample weight of control solution, mg; $P_R$ = purity factor of control;

$A_S$ = peak area of main peak in test solution; $D_S$ = dilution factor of test solution;
$A_R$ = peak area of main peak in control solution; $D_R$ = dilution factor of control solution;

(5) The test data are shown in Table 3-3

Table 3-3

| Medium | Saturated solubility of crystal form A (μg/mL) | Saturated solubility of crystal form B (μg/mL) |
|---|---|---|
| pH 6.8 phosphate buffer | 0.04 | 0.02 |
| Water | 0.61 | 0.34 |
| pH 4.5 acetate buffer | 14545.08 | 3107.54 |

Test Example 4: Suspension competition experiment

Suspension competition experiments of the crystal form B with the crystal forms D, M, N, and P in a solvent system of ethanol/water with different water activities ($a_w$ = 0, 0.2, 0.4, 0.6, 0.8, and 1.0) were performed at room temperature according to Table 4. The XRPD patterns of the solids obtained in the experiments are shown in FIG. 20A (Experiments 1-5) or FIG. 20B (Experiment 6), and the results are shown in Table 22-1. Among them, the initial crystal forms in Experiments 1-4 and 6 were B, D, M, and N, and the initial crystal form in Experiment 5 was B, D, M, N, and P.

Table 4. Suspension competition experiment

| Experiment No. | Initial crystal form | Solvent (v:v) | Temperature | Result |
|---|---|---|---|---|
| 1 | B/D/M/N | Acetone ($a_w$ = 0) | Room temperature | Crystal form B |
| 2 | | Acetone/$H_2O$ (986:14, $a_w$ = 0.2) | | Crystal form B |
| 3 | | Acetone/$H_2O$ (95:5, $a_w$ = 0.4) | | Crystal form D |
| 4 | | Acetone/$H_2O$ (86:14, $a_w$ = 0.6) | | Crystal form D |
| 5 | B/D/M/N/P | Acetone/$H_2O$ (6:4, $a_w$ = 0.8) | | Crystal form D |
| 6 | B/D/M/N | $H_2O$ ($a_w$ = 0) | | A mixture of crystal form P and crystal form C |

The results showed that the anhydrous crystal form B was obtained under the condition of $a_w$ = 0-0.2, the hydrate crystal form D was obtained under the condition of $a_w$ = 0.4-0.8, the mixture of the hydrate crystal form P and the anhydrous crystal form C was obtained under the condition of $a_w$ = 1.0 (purified water), and the signal of the crystal form C was possibly generated by the polymorphic transition of the crystal form P during the XRPD test.

Test Example 5: Pharmacokinetic Experiment

Six SPF-grade SD rats were prepared and weighed before administration. The dose of administration was calculated based on the weight. An appropriate amount of the crystal form A and crystal form B samples were accurately weighed out, a solution of 5% DMSO in normal saline was added, and the mixtures were mixed uniformly by vortex or ultrasonic to obtain 1 mg/mL administration solutions. These administration solutions were orally and intragastrically administered at 10 mg/kg. The rats were weighed before administration, and the dose of administration was calculated based on the weight. 3 rats were used for each crystal form. The administration solutions were orally and intragastrically administered, followed by collection of samples via the jugular vein or other suitable means at the following time points: 0.25 h, 0.5 h, 1 h, 2 h, 4 h, 8 h, and 24 h. About 0.20 mL of each sample was collected, and the plasma was separated out by centrifugation within 2 h. The compound I was detected, and plasma drug concentration-time curves were plotted. The pharmacokinetic parameters were calculated by utilizing WinNonlin according to the plasma concentration data at different time points. The results showed that the *in vivo* absorption of the crystal form A was significantly superior to that of the crystal form B.

Table 5. Pharmacokinetic results

| Crystal form | AUC(0-t) (h*ng/mL) |
|---|---|
| A | 19085 |
| B | 10424 |

In this specification, terms such as "embodiments", "examples", or the like mean that a particular feature, structure, material, or characteristic described in reference to the embodiment or example is included in at least one embodiment or example of the present disclosure. In this specification, the schematic descriptions of the terms described above do not necessarily refer to the same embodiment or example. Moreover, the specific features, materials, structures and other characteristics described may be combined in any one or more embodiments or examples in an appropriate manner. Moreover, although the embodiments of the present disclosure have been shown and described above, it will be understood that the embodiments described above are exemplary and are not to be construed as limiting the present disclosure, and that those of ordinary skill in the art can make changes, modifications, replacements, and alterations to the embodiments described above within the scope of the present disclosure without departing from the principles and spirit of the present disclosure.

**Claims**

1. A solid form of a compound of formula (I) or a pharmaceutically acceptable salt thereof:

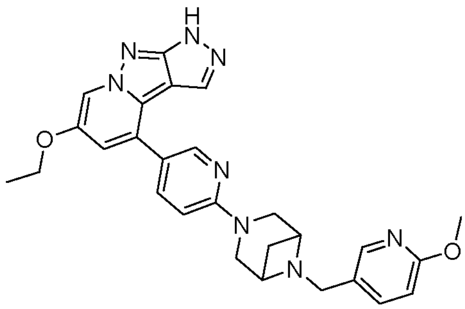

**(I)**

wherein the solid form is selected from solid forms of an anhydrate, a hydrate, an organic solvate, and a co-solvate of water and an organic solvent.

2. The solid form according to claim 1, wherein the solid form is selected from one, two, three, or more of the following crystal forms:
a crystal form A, wherein:

an X-ray powder diffraction pattern of the crystal form A has one or two characteristic peaks at 2θ values selected from the following: 6.91°±0.20° and 10.32°±0.20°;
preferably, the X-ray powder diffraction pattern of the crystal form A has one, two, or all characteristic peaks at 2θ values selected from the following: 6.91°±0.20°, 10.32°±0.20°, and 25.72°±0.20°;
preferably, the X-ray powder diffraction pattern of the crystal form A has one, two, more, or all characteristic peaks at 2θ values selected from the following: 6.91°±0.20°, 10.32°±0.20°, 13.88°±0.20°, and 25.72°±0.20°;
preferably, the X-ray powder diffraction pattern of the crystal form A has one, two, more, or all characteristic peaks at 2θ values selected from the following: 4.46°±0.20°, 6.91°±0.20°, 10.32°±0.20°, 13.88°±0.20°, 14.84°±0.20°, 18.33°±0.20°, 20.32°±0.20°, and 25.72°±0.20°;
preferably, the X-ray powder diffraction pattern of the crystal form A has one, two, more, or all characteristic peaks at 2θ values selected from the following: 4.46°±0.20°, 6.91°±0.20°, 10.32°±0.20°, 10.63°±0.20°, 13.61°±0.20°, 13.88°±0.20°, 14.84°±0.20°, 18.33°±0.20°, 20.32°±0.20°, and 25.72°±0.20°;
preferably, the X-ray powder diffraction pattern of the crystal form A further has one, two, more, or all characteristic peaks at 2θ values selected from the following: 15.86°±0.20°, 17.00°±0.20°, 17.18°±0.20°, and 24.51°±0.20°;
preferably, the X-ray powder diffraction pattern of the crystal form A has one, two, more, or all characteristic peaks at 2θ values selected from the following:

| 2θ (°±0.20°) | 2θ (°±0.20°) |
|---|---|
| 4.46 | 18.33 |
| 6.91 | 18.80 |
| 8.28 | 19.95 |
| 10.32 | 20.32 |
| 10.63 | 20.91 |
| 11.51 | 22.25 |
| 12.40 | 23.10 |
| 13.24 | 24.51 |
| 13.61 | 25.08 |
| 13.88 | 25.72 |
| 14.30 | 27.65 |
| 14.84 | 28.79 |
| 15.86 | 30.68 |
| 16.44 | 31.51 |
| 17.00 | 32.22 |

(continued)

| 2θ (°±0.20°) | 2θ (°±0.20°) |
|---|---|
| 17.18 | 33.49 |
| 17.70 | 34.34 |

preferably, the crystal form A has an X-ray powder diffraction pattern substantially as shown in FIG. 1A;
preferably, a differential scanning calorimetry (DSC) profile of the crystal form A has an endothermic peak at an onset temperature of about 175.03 °C and/or a peak temperature of about 180.61 °C;
preferably, the crystal form A has a differential scanning calorimetry (DSC) profile substantially as shown in FIG. 1B;
preferably, thermogravimetric analysis (TGA) of the crystal form A shows a weight loss of about 0.45% to about 0.55% upon heating from room temperature to 190 °C;
preferably, the crystal form A has a thermogravimetric analysis (TGA) profile substantially as shown in FIG. 1C;
preferably, the crystal form A is an anhydrous crystal form;
and
a crystal form B, wherein:

an X-ray powder diffraction pattern of the crystal form B has one, two, or all characteristic peaks at 2θ values selected from the following: 18.19°±0.20°, 25.74°±0.20°, and 26.95°±0.20°;
preferably, the X-ray powder diffraction pattern of the crystal form B has one, two, more, or all characteristic peaks at 2θ values selected from the following: 11.04°±0.20°, 12.78°±0.20°, 16.12°±0.20°, 16.74°±0.20°, 18.19°±0.20°, 25.22°±0.20°, 25.74°±0.20°, and 26.95°±0.20°;
preferably, the X-ray powder diffraction pattern of the crystal form B has one, two, more, or all characteristic peaks at 2θ values selected from the following: 4.26°±0.20°, 11.04°±0.20°, 12.78°±0.20°, 16.12°±0.20°, 16.74°±0.20°, 18.19°±0.20°, 19.96°±0.20°, 24.33°±0.20°, 25.22°±0.20°, 25.74°±0.20°, and 26.95°±0.20°;
preferably, the crystal form B has an X-ray powder diffraction pattern substantially as shown in FIG. 2A;
preferably, a differential scanning calorimetry (DSC) profile of the crystal form B has an endothermic peak at an onset temperature of about 186.7 °C and/or a peak temperature of about 189.8 °C;
preferably, the crystal form B has a differential scanning calorimetry (DSC) profile substantially as shown in FIG. 2B;
preferably, thermogravimetric analysis (TGA) of the crystal form B shows a weight loss of about 0.5% to about 0.6% (e.g., about 0.54%) upon heating from room temperature to 150 °C;
preferably, the crystal form B has a thermogravimetric analysis (TGA) profile substantially as shown in FIG. 2B;
preferably, the crystal form B is an anhydrous crystal form.

**3.** The solid form according to claim 1, wherein the solid form is selected from one, two, three, or more of the following crystal forms:
a crystal form C, wherein:

an X-ray powder diffraction pattern of the crystal form C has one, two, or all characteristic peaks at 2θ values selected from the following: 7.25°±0.20°, 16.53°±0.20°, and 18.84°±0.20°;
preferably, the X-ray powder diffraction pattern of the crystal form C has one, two, more, or all characteristic peaks at 2θ values selected from the following: 7.25°±0.20°, 14.48°±0.20°, 16.53°±0.20°, and 18.84°±0.20°;
preferably, the X-ray powder diffraction pattern of the crystal form C has one, two, more, or all characteristic peaks at 2θ values selected from the following: 7.25°±0.20°, 12.54°±0.20°, 13.04°±0.20°, 14.48°±0.20°, 16.53°±0.20°, 18.84°±0.20°, 19.18°±0.20°, and 25.02°±0.20°;
preferably, the crystal form C has an X-ray powder diffraction pattern substantially as shown in FIG. 3A;
preferably, a differential scanning calorimetry (DSC) profile of the crystal form C has endothermic peaks at a peak temperature of about 109.1 °C, as well as an onset temperature of about 123.4 °C and/or a peak temperature of about 132.5 °C;
preferably, the crystal form C has a differential scanning calorimetry (DSC) profile substantially as shown in FIG. 3B;
preferably, thermogravimetric analysis (TGA) of the crystal form C shows a weight loss of about 0.2% to about 0.3% (e.g., about 0.27%) upon heating from room temperature to 200 °C;
preferably, the crystal form C has a thermogravimetric analysis (TGA) profile substantially as shown in FIG. 3B;

preferably, the crystal form C is an anhydrous crystal form;
and
a crystal form D, wherein:

an X-ray powder diffraction pattern of the crystal form D has one, two, or all characteristic peaks at 2θ values selected from the following: 11.01°±0.20°, 25.01°±0.20°, and 26.42°±0.20°;
preferably, the X-ray powder diffraction pattern of the crystal form D has one, two, more, or all characteristic peaks at 2θ values selected from the following: 4.26°±0.20°, 11.01°±0.20°, 15.61°±0.20°, 18.53°±0.20°, 22.14°±0.20°, 25.01°±0.20°, and 26.42°±0.20°;
preferably, the X-ray powder diffraction pattern of the crystal form D has one, two, more, or all characteristic peaks at 2θ values selected from the following: 4.26°±0.20°, 9.01°±0.20°, 11.01°±0.20°, 15.61°±0.20°, 18.53°±0.20°, 22.14°±0.20°, 25.01°±0.20°, and 26.42°±0.20°;
preferably, the crystal form D has an X-ray powder diffraction pattern substantially as shown in FIG. 4A;
preferably, a differential scanning calorimetry (DSC) profile of the crystal form D has endothermic peaks at a peak temperature of about 63.7 °C, as well as an onset temperature of about 181.7 °C and/or a peak temperature of about 184.3 °C;
preferably, the crystal form D has a differential scanning calorimetry (DSC) profile substantially as shown in FIG. 4B;
preferably, thermogravimetric analysis (TGA) of the crystal form D shows a weight loss of about 3.3% to about 3.4% (e.g., about 3.35%) upon heating from room temperature to 200 °C;
preferably, the crystal form D has a thermogravimetric analysis (TGA) profile substantially as shown in FIG. 4B;
preferably, the crystal form D is a hydrate;
and
a crystal form E, wherein:

an X-ray powder diffraction pattern of the crystal form E has one, two, or all characteristic peaks at 2θ values selected from the following: 5.98°±0.20°, 12.73°±0.20°, and 14.53°±0.20°;
preferably, the X-ray powder diffraction pattern of the crystal form E has one, two, more, or all characteristic peaks at 2θ values selected from the following: 5.98°±0.20°, 10.66°±0.20°, 11.95°±0.20°, 12.73°±0.20°, 14.53°±0.20°, 17.67°±0.20°, and 22.91°±0.20°;
preferably, the crystal form E has an X-ray powder diffraction pattern substantially as shown in FIG. 5A;
preferably, a differential scanning calorimetry (DSC) profile of the crystal form E has endothermic peaks at a peak temperature of about 114.9 °C and a peak temperature of about 127.9 °C;
preferably, the crystal form E has a differential scanning calorimetry (DSC) profile substantially as shown in FIG. 5B;
preferably, thermogravimetric analysis (TGA) of the crystal form E shows a weight loss of about 1.9% to about 2.0% (e.g., about 1.95%) upon heating from room temperature to 50 °C,
and a weight loss of about 3.0% to about 3.1% (e.g., about 3.02%) upon heating from 50 °C to 150 °C;
preferably, the crystal form E has a thermogravimetric analysis (TGA) profile substantially as shown in FIG. 5B;
preferably, the crystal form E is a methanol solvate;
preferably, the crystal form E has a variable-temperature XRPD pattern substantially as shown in FIG. 5D.

**4.** The solid form according to claim 1, wherein the solid form is selected from one, two, three, or more of the following crystal forms:
a crystal form F, wherein:

an X-ray powder diffraction pattern of the crystal form F has one, two, or all characteristic peaks at 2θ values selected from the following: 11.07°±0.20°, 24.79°±0.20°, and 26.42°±0.20°;
preferably, the X-ray powder diffraction pattern of the crystal form F has one, two, or all characteristic peaks at 2θ values selected from the following: 11.07°±0.20°, 17.83°±0.20°, 22.44°±0.20°, 24.79°±0.20°, and 26.42°±0.20°;
preferably, the X-ray powder diffraction pattern of the crystal form F has one, two, or all characteristic peaks at 2θ values selected from the following: 11.07°±0.20°, 17.83°±0.20°, 22.44°±0.20°, 24.79°±0.20°, and 26.42°±0.20°;
preferably, the X-ray powder diffraction pattern of the crystal form F has one, two, or all characteristic peaks at 2θ

values selected from the following: 11.07°±0.20°, 14.99°±0.20°, 17.83°±0.20°, 19.93°±0.20°, 20.64°±0.20°, 22.44°±0.20°, 24.79°±0.20°, and 26.42°±0.20°;
preferably, the crystal form F has an X-ray powder diffraction pattern substantially as shown in FIG. 6A;
preferably, a differential scanning calorimetry (DSC) profile of the crystal form F has endothermic peaks at a peak temperature of about 69.9 °C, as well as an onset temperature of about 182.1 °C and/or a peak temperature of about 184.5 °C;
preferably, the crystal form F has a differential scanning calorimetry (DSC) profile substantially as shown in FIG. 6B;
preferably, thermogravimetric analysis (TGA) of the crystal form F shows a weight loss of about 2.2% to about 2.3% (e.g., about 2.25%) upon heating from room temperature to 100 °C, and a weight loss of about 2.7% to about 2.8% (e.g., about 2.79%) upon heating from 100 °C to 200 °C;
preferably, the crystal form F has a thermogravimetric analysis (TGA) profile substantially as shown in FIG. 6B;
preferably, the crystal form F is an ethanol solvate;
preferably, the crystal form F has a variable-temperature XRPD pattern substantially as shown in FIG. 6D; and
a crystal form G, wherein:

an X-ray powder diffraction pattern of the crystal form G has one, two, or all characteristic peaks at 2θ values selected from the following: 6.97°±0.20°, 13.39°±0.20°, and 25.81°±0.20°;
preferably, the X-ray powder diffraction pattern of the crystal form G has one, two, more, or all characteristic peaks at 2θ values selected from the following: 4.50°±0.20°, 6.97°±0.20°, 13.39°±0.20°, 14.40°±0.20°, 17.11°±0.20°, 17.81°±0.20°, 23.94°±0.20°, and 25.81°±0.20°;
preferably, the crystal form G has an X-ray powder diffraction pattern substantially as shown in FIG. 7A;
preferably, a differential scanning calorimetry (DSC) profile of the crystal form G has endothermic peaks at a peak temperature of about 122.6 °C, as well as an onset temperature of about 168.5 °C and/or a peak temperature of about 173.1 °C;
preferably, the crystal form G has a differential scanning calorimetry (DSC) profile substantially as shown in FIG. 7B;
preferably, thermogravimetric analysis (TGA) of the crystal form G shows a weight loss of about 1.2% to about 1.4% (e.g., about 1.3%) upon heating from room temperature to 75 °C,
and a weight loss of about 2.6% to about 2.7% (e.g., about 2.69%) upon heating from 75 °C to 150 °C;
preferably, the crystal form G has a thermogravimetric analysis (TGA) profile substantially as shown in FIG. 7B;
preferably, the crystal form G is an acetonitrile solvate;
and
a crystal form H, wherein:

an X-ray powder diffraction pattern of the crystal form H has one, two, or all characteristic peaks at 2θ values selected from the following: 12.92°±0.20°, 15.82°±0.20°, and 17.25°±0.20°;
preferably, the X-ray powder diffraction pattern of the crystal form H has one, two, more, or all characteristic peaks at 2θ values selected from the following: 7.89°±0.20°, 10.51°±0.20°, 12.92°±0.20°, 15.82°±0.20°, 16.69°±0.20°, 17.25°±0.20°, 19.12°±0.20°, and 25.39°±0.20°;
preferably, the crystal form H has an X-ray powder diffraction pattern substantially as shown in FIG. 8A;
preferably, a differential scanning calorimetry (DSC) profile of the crystal form H has endothermic peaks at peak temperatures of about 121.7 °C, about 138.7 °C, about 170.2 °C, and about 184.7 °C;
preferably, the crystal form H has a differential scanning calorimetry (DSC) profile substantially as shown in FIG. 8B;
preferably, thermogravimetric analysis (TGA) of the crystal form H shows a weight loss of about 1.7% to about 1.8% (e.g., about 1.73%) upon heating from room temperature to 70 °C, and a weight loss of about 7.9% to about 8.0% (e.g., about 7.98%) upon heating from 70 °C to 150 °C;
preferably, the crystal form H has a thermogravimetric analysis (TGA) profile substantially as shown in FIG. 8B;
preferably, the crystal form H is a dichloromethane solvate;
preferably, the crystal form H has a variable-temperature XRPD pattern substantially as shown in FIG. 8D.

**5.** The solid form according to claim 1, wherein the solid form is selected from one, two, three, or more of the following crystal forms:

a crystal form I, wherein:

an X-ray powder diffraction pattern of the crystal form I has one, two, more, or all characteristic peaks at 2θ values selected from the following: 4.45°±0.20°, 13.35°±0.20°, and 17.82°±0.20°;
preferably, the X-ray powder diffraction pattern of the crystal form I has one, two, more, or all characteristic peaks at 2θ values selected from the following: 4.45°±0.20°, 13.35°±0.20°, 17.15°±0.20°, 17.82°±0.20°, 22.32°±0.20°, 23.82°±0.20°, and 25.70°±0.20°;
preferably, the crystal form I has an X-ray powder diffraction pattern substantially as shown in FIG. 9;
and
a crystal form J, wherein:

an X-ray powder diffraction pattern of the crystal form J has one, two, or all characteristic peaks at 2θ values selected from the following: 4.22°±0.20°, 7.15°±0.20°, and 25.24°±0.20°;
preferably, the X-ray powder diffraction pattern of the crystal form J has one, two, more, or all characteristic peaks at 2θ values selected from the following: 4.22°±0.20°, 6.74°±0.20°, 7.15°±0.20°, 11.04°±0.20°, 12.62°±0.20°, 13.48°±0.20°, 16.86°±0.20°, and 25.24°±0.20°;
preferably, the crystal form J has an X-ray powder diffraction pattern substantially as shown in FIG. 10A;
preferably, a differential scanning calorimetry (DSC) profile of the crystal form J has endothermic peaks at an onset temperature of about 178.6 °C and/or a peak temperature of about 182.4 °C, as well as a peak temperature of about 188.9 °C;
preferably, the crystal form J has a differential scanning calorimetry (DSC) profile substantially as shown in FIG. 10B;
preferably, thermogravimetric analysis (TGA) of the crystal form J shows a weight loss of about 1.4% to about 1.5% (e.g., about 1.41%) upon heating from room temperature to 100 °C, and a weight loss of about 3.3% to about 3.4% (e.g., about 3.38%) upon heating from 100 °C to 200 °C;
preferably, the crystal form J has a thermogravimetric analysis (TGA) profile substantially as shown in FIG. 10B;
preferably, the crystal form J is a 1,4-dioxane solvate;
preferably, the crystal form J has a variable-temperature XRPD pattern substantially as shown in FIG. 10D;
and
a crystal form K, wherein:

an X-ray powder diffraction pattern of the crystal form K has one, two, or all characteristic peaks at 2θ values selected from the following: 4.19°±0.20°, 6.48°±0.20°, 6.95°±0.20°, and 19.54°±0.20°;
preferably, the X-ray powder diffraction pattern of the crystal form K has one, two, more, or all characteristic peaks at 2θ values selected from the following: 4.19°±0.20°, 5.13°±0.20°, 6.48°±0.20°, 6.95°±0.20°, 9.75°±0.20°, 11.06°±0.20°, 17.13°±0.20°, and 19.54°±0.20°;
preferably, the crystal form K has an X-ray powder diffraction pattern substantially as shown in FIG. 11A;
preferably, the crystal form K has a differential scanning calorimetry (DSC) profile substantially as shown in FIG. 11B;
preferably, the crystal form K has a thermogravimetric analysis (TGA) profile substantially as shown in FIG. 11B.

**6.** The solid form according to claim 1, wherein the solid form is selected from one, two, three, or more of the following crystal forms:
a crystal form L, wherein:

an X-ray powder diffraction pattern of the crystal form L has one, two, or all characteristic peaks at 2θ values selected from the following: 18.85°±0.20°, 22.30°±0.20°, and 29.35°±0.20°;
preferably, the X-ray powder diffraction pattern of the crystal form L has one, two, more, or all characteristic peaks at 2θ values selected from the following: 9.23°±0.20°, 18.85°±0.20°, 22.30°±0.20°, 22.69°±0.20°, 23.22°±0.20°, 23.82°±0.20°, 26.58°±0.20°, and 29.35°±0.20°;
preferably, the crystal form L has an X-ray powder diffraction pattern substantially as shown in 12A;
preferably, a differential scanning calorimetry (DSC) profile of the crystal form L has endothermic peaks at an onset temperature of about 95.4 °C and/or a peak temperature of about 100.0 °C, as well as a peak temperature of about 154.5 °C;
preferably, the crystal form L has a differential scanning calorimetry (DSC) profile substantially as shown in FIG. 12B;

preferably, thermogravimetric analysis (TGA) of the crystal form L has a weight loss of about 8.9% to about 9.0% (e.g., about 8.98%) upon heating from room temperature to 100 °C, a weight loss of about 2.5% to about 2.6% (e.g., about 2.56%) upon heating from 100 °C to 125 °C, and a weight loss of about 1.8% to about 1.9% (e.g., about 1.81%) upon heating from 125 °C to 150 °C;
preferably, the crystal form L has a thermogravimetric analysis (TGA) profile substantially as shown in FIG. 12B;
preferably, the crystal form L is a methanol-water co-solvate;
preferably, the crystal form L has a **$^1$H** NMR spectrum substantially as shown in FIG. 12C;
preferably, a monocrystal of the crystal form L has a schematic asymmetric unit diagram of a monocrystal structure as shown in FIG. 12D;
preferably, the monocrystal of the crystal form L has the following structural parameters:

| | |
|---|---|
| **Empirical formula** | **$C_{27}H_{28}N_3O_2 \cdot CH_3OH \cdot 2H_2O$** |
| **Formula weight** | **564.65** |
| **Temperature** | **119.99(10) K** |
| **Wavelength** | **Cu/$K_\alpha$ ($\lambda$ = 1.54184 Å)** |
| **Crystal system, space group** | **Triclinic, $P\bar{1}$** |
| **Unit cell dimensions** | **$a$ = 10.86800(10) Å**<br>**$b$ = 11.09330(10) Å**<br>**$c$ = 12.30730(10) Å**<br>**$\alpha$ = 110.4970(10)°**<br>**$\beta$ = 96.2040(10)°**<br>**$\gamma$ = 92.5880(10)°** |
| **Unit cell volume** | **1376.22(2) Å$^3$** |
| **Z, calculated density** | **2, 1.363 g/cm$^3$** |

and
a crystal form M, wherein:

an X-ray powder diffraction pattern of the crystal form M has one, two, or all characteristic peaks at 2θ values selected from the following: 4.22°±0.20°, 18.59°±0.20°, and 25.31°±0.20°;
preferably, the X-ray powder diffraction pattern of the crystal form M has one, two, more, or all characteristic peaks at 2θ values selected from the following: 4.22°±0.20°, 9.05°±0.20°, 10.94°±0.20°, 15.48°±0.20°, 18.59°±0.20°, 21.81°±0.20°, 25.31°±0.20°, and 26.48°±0.20°;
preferably, the crystal form M has an X-ray powder diffraction pattern substantially as shown in FIG. 13A;
preferably, a differential scanning calorimetry (DSC) profile of the crystal form M has endothermic peaks at a peak temperature of about 61.3 °C, as well as an onset temperature of about 181.4 °C and/or a peak temperature of about 184.2 °C;
preferably, the crystal form M has a differential scanning calorimetry (DSC) profile substantially as shown in FIG. 13B;
preferably, thermogravimetric analysis (TGA) of the crystal form M shows a weight loss of about 2.0% to about 2.1% (e.g., about 2.05%) upon heating from room temperature to 200 °C;
preferably, the crystal form M has a thermogravimetric analysis (TGA) profile substantially as shown in FIG. 13B;
preferably, the crystal form M is a hydrate;
and
a crystal form N, wherein:

an X-ray powder diffraction pattern of the crystal form N has one, two, or all characteristic peaks at 2θ values selected from the following: 5.82°±0.20°, 13.96°±0.20°, and 20.24°±0.20°;
preferably, the X-ray powder diffraction pattern of the crystal form N has one, two, more, or all characteristic peaks at 2θ values selected from the following: 5.82°±0.20°, 7.18°±0.20°, 12.35°±0.20°, 13.96°±0.20°, 15.77°±0.20°, 17.49°±0.20°, 18.40°±0.20°, and 20.24°±0.20°;
preferably, the crystal form N has an X-ray powder diffraction pattern substantially as shown in FIG. 14A;
preferably, a differential scanning calorimetry (DSC) profile of the crystal form N has an endothermic peak at an onset temperature of about 118.0 °C and/or a peak temperature of about 125.9 °C;
preferably, the crystal form N has a differential scanning calorimetry (DSC) profile substantially as shown

in FIG. 14B;
preferably, thermogravimetric analysis (TGA) of the crystal form N shows a weight loss of about 0.1% to about 0.2% (e.g., about 0.19%) upon heating from room temperature to 200 °C;
preferably, the crystal form N has a thermogravimetric analysis (TGA) profile substantially as shown in FIG. 14B;
preferably, the crystal form N is an anhydrous crystal form;
preferably, the crystal form N has a variable-temperature XRPD pattern substantially as shown in FIG. 14D.

**7.** The solid form according to claim 1, wherein the solid form is selected from one, two, three, or more of the following crystal forms:
a crystal form O, wherein:

an X-ray powder diffraction pattern of the crystal form O has one, two, or all characteristic peaks at 2θ values selected from the following: 11.06°±0.20°, 24.85°±0.20°, and 26.44°±0.20°;
preferably, the X-ray powder diffraction pattern of the crystal form O has one, two, more, or all characteristic peaks at 2θ values selected from the following: 4.32°±0.20°, 11.06°±0.20°, 15.11°±0.20°, 15.76°±0.20°, 17.22°±0.20°, 17.93°±0.20°, 24.85°±0.20°, and 26.44°±0.20°;
preferably, the crystal form O has an X-ray powder diffraction pattern substantially as shown in FIG. 15A;
preferably, a differential scanning calorimetry (DSC) profile of the crystal form O has endothermic peaks at peak temperatures of about 174.7 °C and about 187.1 °C;
preferably, the crystal form O has a differential scanning calorimetry (DSC) profile substantially as shown in FIG. 15B;
preferably, thermogravimetric analysis (TGA) of the crystal form O shows a weight loss of about 4.0% to about 4.1% (e.g., about 4.03%) upon heating from room temperature to 150 °C,
and a weight loss of about 1.5% to about 1.7% (e.g., about 1.60%) upon heating from 150 °C to 200 °C;
preferably, the crystal form O has a thermogravimetric analysis (TGA) profile substantially as shown in FIG. 15B;
preferably, the crystal form O is a methanol solvate;
according to an embodiment of the present disclosure, the crystal form O has a variable-temperature XRPD pattern substantially as shown in FIG. 15D;
and
a crystal form P, wherein:

an X-ray powder diffraction pattern of the crystal form P has one, two, or all characteristic peaks at 2θ values selected from the following: 6.36°±0.20°, 16.15°±0.20°, and 21.05°±0.20°;
preferably, the X-ray powder diffraction pattern of the crystal form P has one, two, more, or all characteristic peaks at 2θ values selected from the following: 6.36°±0.20°, 11.09°±0.20°, 16.15°±0.20°, 17.27°±0.20°, 21.05°±0.20°, 26.38°±0.20°, 26.62°±0.20°, and 34.88°±0.20°;
preferably, the crystal form P has an X-ray powder diffraction pattern substantially as shown in FIG. 16A;
preferably, the crystal form P is a hydrate;
and
a crystal form Q, wherein:

an X-ray powder diffraction pattern of the crystal form Q has one, two, or all characteristic peaks at 2θ values selected from the following: 18.41°±0.20°, 25.19°±0.20°, and 26.36°±0.20°;
preferably, the X-ray powder diffraction pattern of the crystal form Q has one, two, more, or all characteristic peaks at 2θ values selected from the following: 4.15°±0.20°, 10.85°±0.20°, 13.28°±0.20°, 18.41°±0.20°, 20.03°±0.20°, 21.69°±0.20°, 25.19°±0.20°, and 26.36°±0.20°;
preferably, the crystal form Q has an X-ray powder diffraction pattern substantially as shown in FIG. 17;
preferably, the crystal form Q is an anhydrous crystal form.

**8.** The solid form according to claim 1, wherein the solid form is selected from one, two, three, or more of the following crystal forms:
a crystal form R, wherein:

an X-ray powder diffraction pattern of the crystal form R has one, two, or all characteristic peaks at 2θ values selected from the following: 7.22°±0.20°, 13.03°±0.20°, and 18.81°±0.20°;
preferably, the X-ray powder diffraction pattern of the crystal form R has one, two, more, or all characteristic peaks

at 2θ values selected from the following: 7.22°±0.20°, 13.03°±0.20°, 18.81°±0.20°, and 19.15°±0.20°; preferably, the crystal form R has an X-ray powder diffraction pattern substantially as shown in 22A; preferably, the crystal form R is an anhydrous crystal form;
and
a crystal form S, wherein:

an X-ray powder diffraction pattern of the crystal form S has one, two, or all characteristic peaks at 2θ values selected from the following: 6.30°±0.20°, 16.11°±0.20°, and 21.01°±0.20°;
preferably, the X-ray powder diffraction pattern of the crystal form S has one, two, more, or all characteristic peaks at 2θ values selected from the following: 6.30°±0.20°, 11.06°±0.20°, 16.11°±0.20°, and 21.01°±0.20°;
preferably, the crystal form S has an X-ray powder diffraction pattern substantially as shown in FIG. 23;
preferably, the crystal form S is a hydrate;
and
a crystal form T, wherein the crystal form T has an X-ray powder diffraction pattern substantially as shown in 24A;
and
a crystal form U, wherein the crystal form U has an X-ray powder diffraction pattern substantially as shown in 25A.

**9.** A preparation method for the solid form according to any one of claims 1-8, comprising one selected from the following:

a preparation method for the crystal form A comprising dissolving the compound of formula (I) (such as an amorphous form thereof) in a mixed solvent of ethyl acetate and dichloromethane, and volatilizing the resulting solution at room temperature to obtain the crystal form A, wherein preferably, in the preparation method for the crystal form A, ethyl acetate and dichloromethane may be in a volume ratio of 1:1 to 20:1, e.g., 9:1;
and a preparation method for the crystal form A comprising heating the crystal form G to 150 °C, cooling to room temperature, and exposing to air to obtain the crystal form A;
and a preparation method for the crystal form A comprising drying the crystal form I at room temperature to obtain the crystal form A;
a preparation method for the crystal form L comprising mixing the compound of formula (I) (such as an amorphous form thereof) with a mixed solvent of methanol and water to obtain a solution, and volatilizing the resulting solution at room temperature to obtain the crystal form L, wherein preferably, in the preparation method for the crystal form L, methanol and water may be in a volume ratio of 1:1 to 20:1, e.g., 4:1;
a preparation method for the crystal form B comprising stirring the crystal form L in acetone to obtain the crystal form B, wherein preferably, the crystal form L and acetone may be in a mass-to-volume ratio of 20 mg:(0.1-1 mL), e.g., 20 mg:0.5 mL; preferably, the stirring is performed at 50-70 °C, e.g., 60°C;
a preparation method for the crystal form C comprising stirring the crystal form L in acetone at room temperature first, heating to 60 °C and stirring, cooling to 0-5 °C and stirring, and then mixing the separated solid with water and stirring to obtain the crystal form C, wherein preferably, the crystal form L and acetone may be in a mass-to-volume ratio of 20 mg:(0.1-1 mL), e.g., 20 mg:0.5 mL; the crystal form L and water may be in a mass-to-volume ratio of 20 mg:(0.1-1 mL), e.g., 20 mg:0.5 mL;
preferably, the stirring of the crystal form L in acetone is performed first at 50-70 °C, e.g., 60°C, and then at 0-5 °C; the separated solid is stirred in water at room temperature;
or the crystal form L is stirred in a mixed solvent of acetone and water, wherein acetone and water may be in a volume ratio of (1-2):1, e.g., 1.5:1;
or a wet sample of the crystal form P is dried in the air to obtain the crystal form C;
a preparation method for the crystal form D comprising stirring the crystal form L in acetone at room temperature first, heating to 60 °C and stirring, cooling to 0-5 °C and stirring, and then stirring the separated solid in a mixed solvent of acetone and water, wherein preferably, the crystal form L and acetone may be in a mass-to-volume ratio of 20 mg:(0.1-1 mL), e.g., 20 mg:0.5 mL; the crystal form L and the mixed solvent of acetone and water may be in a mass-to-volume ratio of 20 mg:(0.1-1 mL), e.g., 20 mg:0.5 mL; preferably, in the mixed solvent of acetone and water, acetone and water may be in a volume ratio of (5-7):1, e.g., 1.5:1;
preferably, the stirring of the crystal form L in acetone is performed first at 50-70 °C, e.g., 60°C, and then at 0-5 °C; the separated solid is stirred in the mixed solvent of acetone and water at room temperature;
or the crystal form L is stirred in a mixed solvent of acetone and water, wherein acetone and water may be in a volume ratio of (3-6):1, e.g., 4:1;
a preparation method for the crystal form E comprising stirring the crystal form L in methanol to obtain the crystal

form E, wherein preferably, the stirring is performed at room temperature;

preferably, the crystal form L and methanol may be in a mass-to-volume ratio of 20 mg:(0.1-1 mL), e.g., 20 mg:0.5 mL;

and a preparation method for the crystal form F comprising stirring the compound of formula (I) (such as an amorphous form thereof) in a mixed solvent of acetone and ethanol to obtain the crystal form F, wherein preferably, in the preparation method for the crystal form L, acetone and ethanol may be in a volume ratio of 1:1;

a preparation method for the crystal form G comprising stirring the crystal form L in acetone first, and then stirring the separated solid in acetonitrile to obtain the crystal form G, wherein preferably, the crystal form L and acetone may be in a mass-to-volume ratio of 20 mg:(0.1-1 mL), e.g., 20 mg:0.5 mL; the crystal form L and acetonitrile may be in a mass-to-volume ratio of 20 mg:(0.1-1 mL), e.g., 20 mg:0.5 mL;

a preparation method for the crystal form H comprising stirring the crystal form L in acetone at room temperature first, heating to 60 °C and stirring, cooling to 0-5 °C and stirring, and then performing gas-solid diffusion on the separated solid in a dichloromethane atmosphere to obtain the crystal form H, wherein preferably, the crystal form L and acetone may be in a mass-to-volume ratio of 20 mg:(0.1-1 mL), e.g., 20 mg:0.5 mL;

a preparation method for the crystal form I comprising dissolving the compound of formula (I) (such as an amorphous form thereof) in N-methylpyrrolidone, then adding an anti-solvent such as acetonitrile, and allowing to stand at room temperature to precipitate a solid, thereby obtaining the crystal form I, wherein preferably, the compound of formula (I) and *N*-methylpyrrolidone may be in a mass-to-volume ratio of 20 mg:(1.0-1.5 mL); the compound of formula (I) and N-methylpyrrolidone may be in a mass-to-volume ratio of 20 mg:(3-5 mL), e.g., 20 mg:4 mL;

a preparation method for the crystal form J comprising stirring the crystal form L in acetone at room temperature first, heating to 60 °C and stirring, cooling to 0-5 °C and stirring, mixing the separated solid with 1,4-dioxane and stirring at 50 °C, and then stirring at room temperature to obtain the crystal form J, wherein preferably, the crystal form L and acetone may be in a mass-to-volume ratio of 20 mg:(0.1-1 mL), e.g., 20 mg:0.5 mL; the crystal form L and 1,4-dioxane may be in a mass-to-volume ratio of 20 mg:(0.1-1 mL), e.g., 20 mg:0.5 mL;

a preparation method for the crystal form M comprising heating the crystal form D to 150 °C, then cooling to room temperature, and exposing to air for about 10 min to obtain the crystal form M;

and a preparation method for the crystal form M comprising heating the crystal form F to 160 °C, cooling to room temperature, and exposing to air to obtain the crystal form M;

and a preparation method for the crystal form M comprising heating a crystal form O sample to 175 °C to obtain the crystal form M;

and a preparation method for the crystal form M comprising placing the crystal form Q at room temperature and room humidity to obtain the crystal form M;

a preparation method for the crystal form N comprising heating the crystal form E to 100 °C, then cooling to room temperature, and exposing to air to obtain the crystal form N;

a preparation method for the crystal form O comprising dissolving the compound of formula (I) (such as an amorphous form thereof) in a mixed solvent of methanol and water, and volatilizing at room temperature to obtain the crystal form O, wherein preferably, in the mixed solvent of methanol and water, methanol and water may be in a volume ratio of (3-5):1, e.g., 4:1;

a preparation method for the crystal form P comprising stirring the crystal form L in acetone at room temperature first, heating to 60 °C and stirring, cooling to 0-5 °C and stirring, and then slurring the separated solid in a mixed solvent of MEK, THF, and $H_2O$ to obtain the crystal form P, wherein preferably, in the mixed solvent of MEK, THF, and $H_2O$, MEK, THF, and $H_2O$ may be in a volume ratio of 1:0.1:2;

a preparation method for the crystal form Q comprising purging the crystal form D under $N_2$ atmosphere for 20 min, heating the sample to 150 °C, and cooling to 30 °C to obtain the crystal form Q;

a preparation method for the crystal form R comprising mixing the compound of formula (I) (such as an amorphous form) with a mixed solvent of dichloromethane and acetone, dissolving completely, concentrating to dryness under reduced pressure, adding acetone, heating for complete dissolution, adding water, naturally cooling to room temperature, stirring, and filtering to obtain the crystal form R;

a preparation method for the crystal form S comprising mixing the compound of formula (I) (such as an amorphous form) with 1,4-dioxane, dissolving completely, adding water, precipitating a solid, filtering, and drying to obtain the crystal form S;

a preparation method for the crystal form T comprising mixing the compound of formula (I) (such as an amorphous form) with trichloromethane to obtain a solution 1; mixing maleic acid with ethanol to obtain a solution 2; and mixing the solution 2 with the solution 1, adding methyl *tert*-butyl ether, stirring, centrifuging, and drying to obtain the crystal form T;

a preparation method for the crystal form U comprising mixing the compound of formula (I) (such as an amorphous form) with trichloromethane to obtain a solution 1; mixing methanesulfonic acid with trichloromethane to obtain a

solution 2; and mixing the solution 2 with the solution 1, stirring, and drying to obtain the crystal form U.

**10.** A mixture, comprising at least one selected from the solid forms of the compound of formula (I) or the pharmaceutically acceptable salt thereof according to any one of claims 1-8,
wherein preferably, the content of the crystal form A in percentages by weight is 80% or more, preferably 95% or more, and further preferably 99% or more based on the total weight of the compound of formula (I) in the mixture.

**11.** A pharmaceutical composition, comprising at least one selected from the solid forms of the compound of formula (I) or the pharmaceutically acceptable salt thereof according to any one of claims 1-8, or comprising the mixture,

wherein preferably, the pharmaceutical composition may further comprise a pharmaceutically acceptable auxiliary material;
more preferably, the pharmaceutical composition further comprises at least one additional therapeutic agent.

**12.** Use of the solid form of the compound of formula (I) or the pharmaceutically acceptable salt thereof according to any one of claims 1-8, the mixture according to claim 10, or the pharmaceutical composition according to claim 11, wherein the use is selected from at least one of the following:

use in inhibiting cell proliferation *in vitro* or *in vivo;* and
use in treating an RET kinase-mediated disease; and
use in inhibiting RET kinase activity; and
use in treating a cancer and/or inhibiting metastasis associated with a specific cancer; and
use in treating irritable bowel syndrome (IBS) or pain associated with IBS; and
use in providing supportive care, including preventing or minimizing a gastrointestinal condition, such as diarrhea, associated with a treatment (including chemotherapy), for a cancer patient; and
use in treating a disease or condition associated with RET; and
use in reversing or preventing acquired resistance to an anti-cancer drug; and
use in delaying and/or preventing the development of resistance to an anti-cancer drug in an individual; and
use in treating an individual suffering from a cancer and having an increased possibility of developing resistance to an anti-cancer drug.

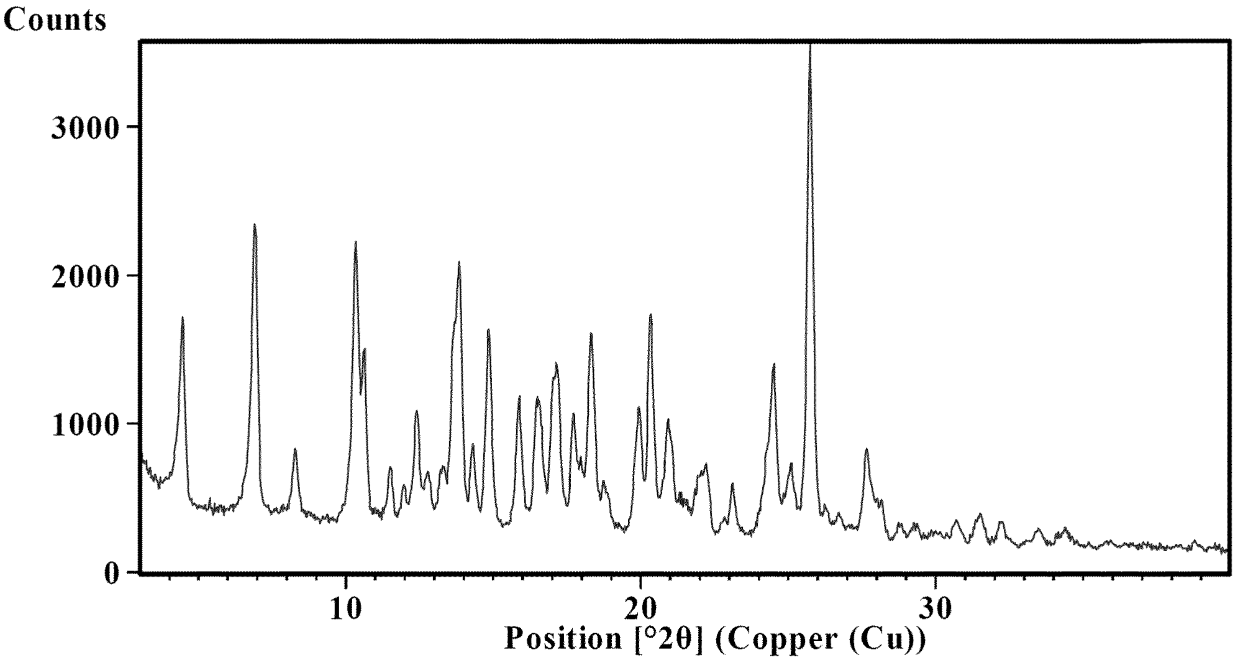
Counts
3000
2000
1000
0
10
20
30
Position [°2θ] (Copper (Cu))

FIG. 1A

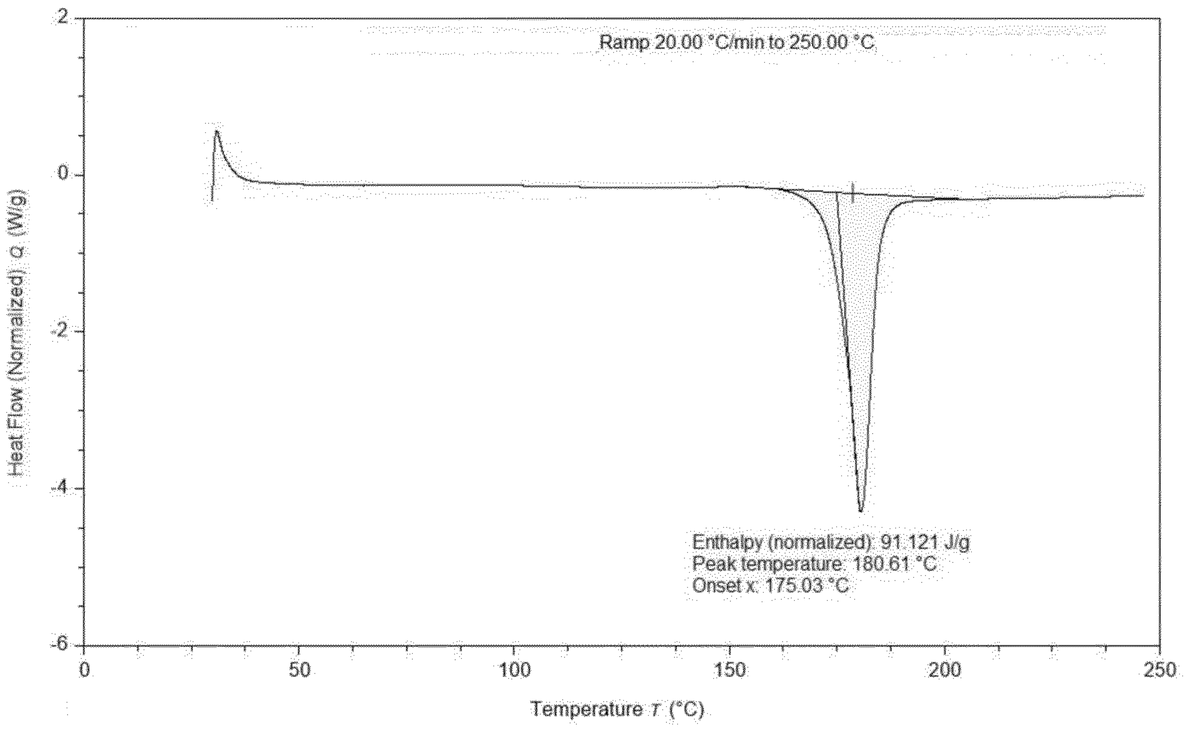
Ramp 20.00 °C/min to 250.00 °C
Heat Flow (Normalized) Q (W/g)
2
0
-2
-4
-6
Enthalpy (normalized): 91.121 J/g
Peak temperature: 180.61 °C
Onset x: 175.03 °C
0
50
100
150
200
250
Temperature T (°C)

FIG. 1B

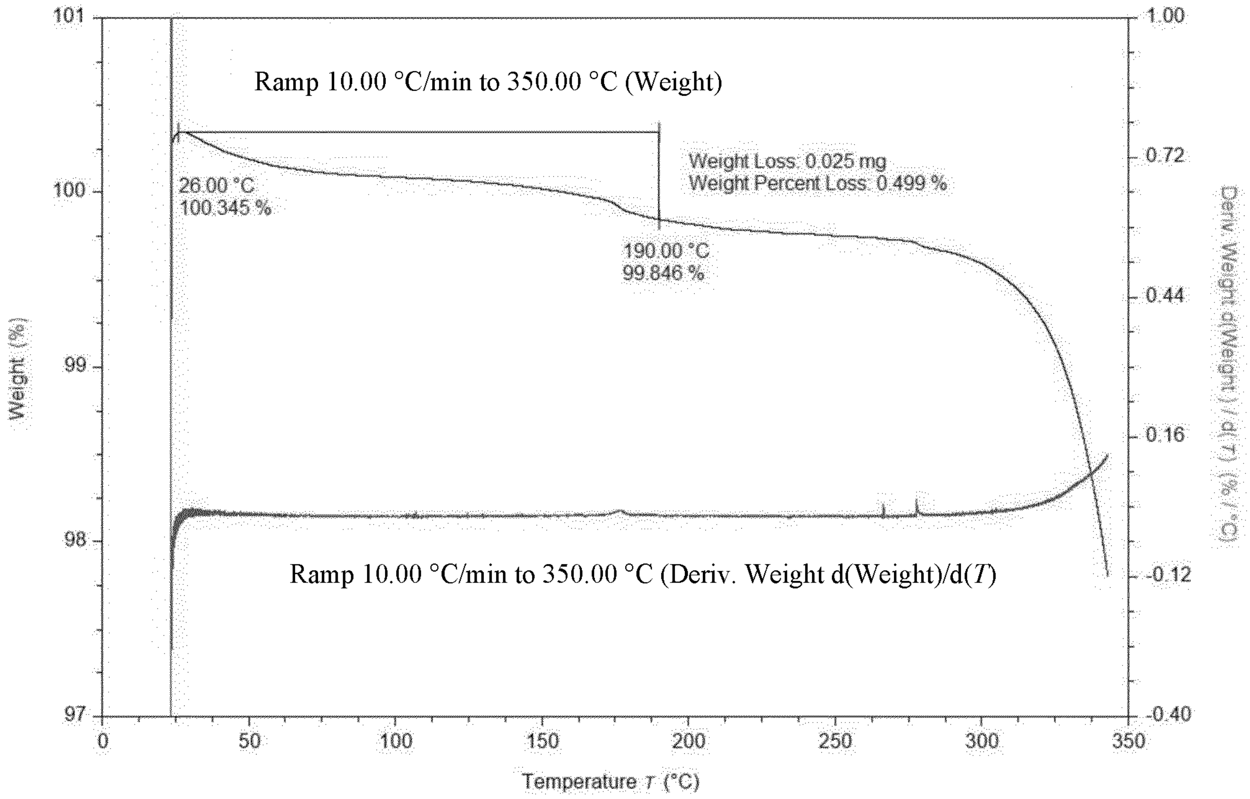
Ramp 10.00 °C/min to 350.00 °C (Weight)
26.00 °C
100.345 %
Weight Loss: 0.025 mg
Weight Percent Loss: 0.499 %
190.00 °C
99.846 %
Ramp 10.00 °C/min to 350.00 °C (Deriv. Weight d(Weight)/d(T)
Weight (%)
Deriv. Weight d(Weight) / d(T) (% / °C)
Temperature T (°C)
101
100
99
98
97
1.00
0.72
0.44
0.16
-0.12
-0.40
0
50
100
150
200
250
300
350

FIG. 1C

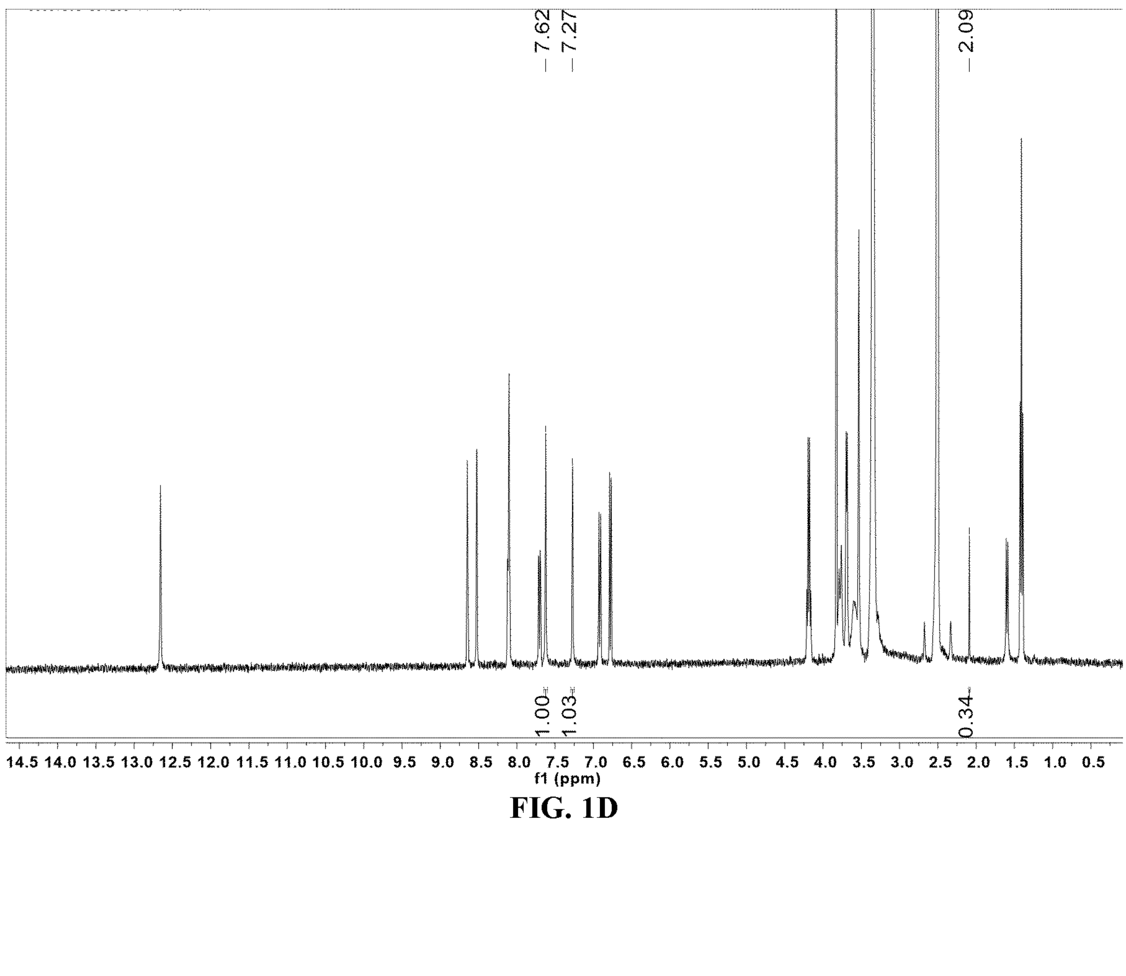
7.62
7.27
2.09
1.00
1.03
0.34
14.5 14.0 13.5 13.0 12.5 12.0 11.5 11.0 10.5 10.0 9.5 9.0 8.5 8.0 7.5 7.0 6.5 6.0 5.5 5.0 4.5 4.0 3.5 3.0 2.5 2.0 1.5 1.0 0.5
f1 (ppm)

FIG. 1D

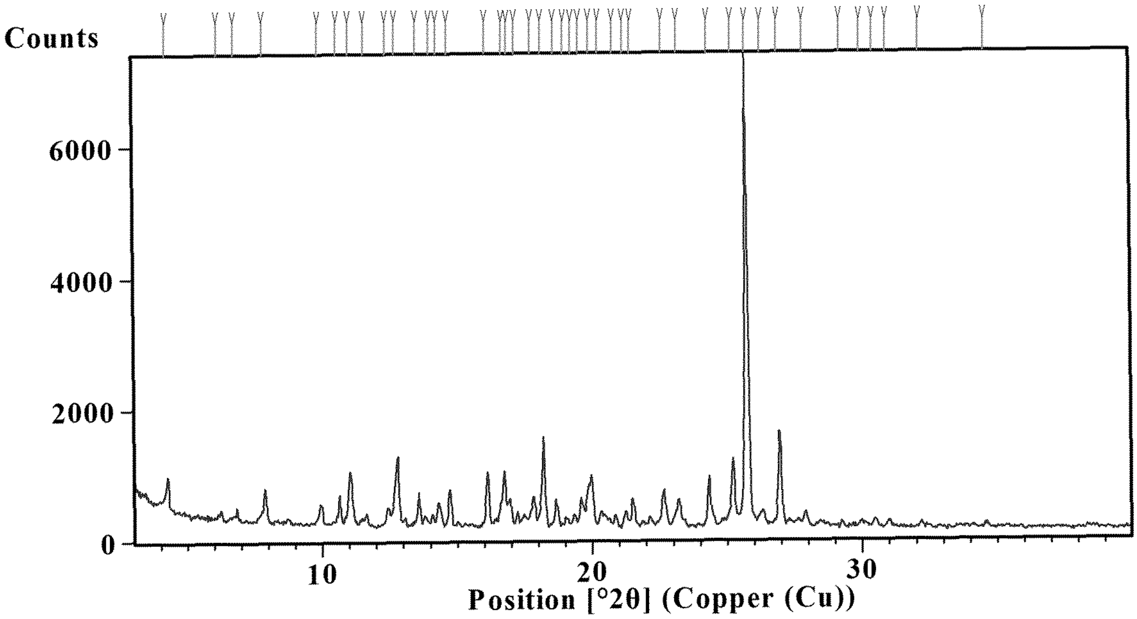
Counts
6000
4000
2000
0
10
20
30
Position [°2θ] (Copper (Cu))

FIG. 2A

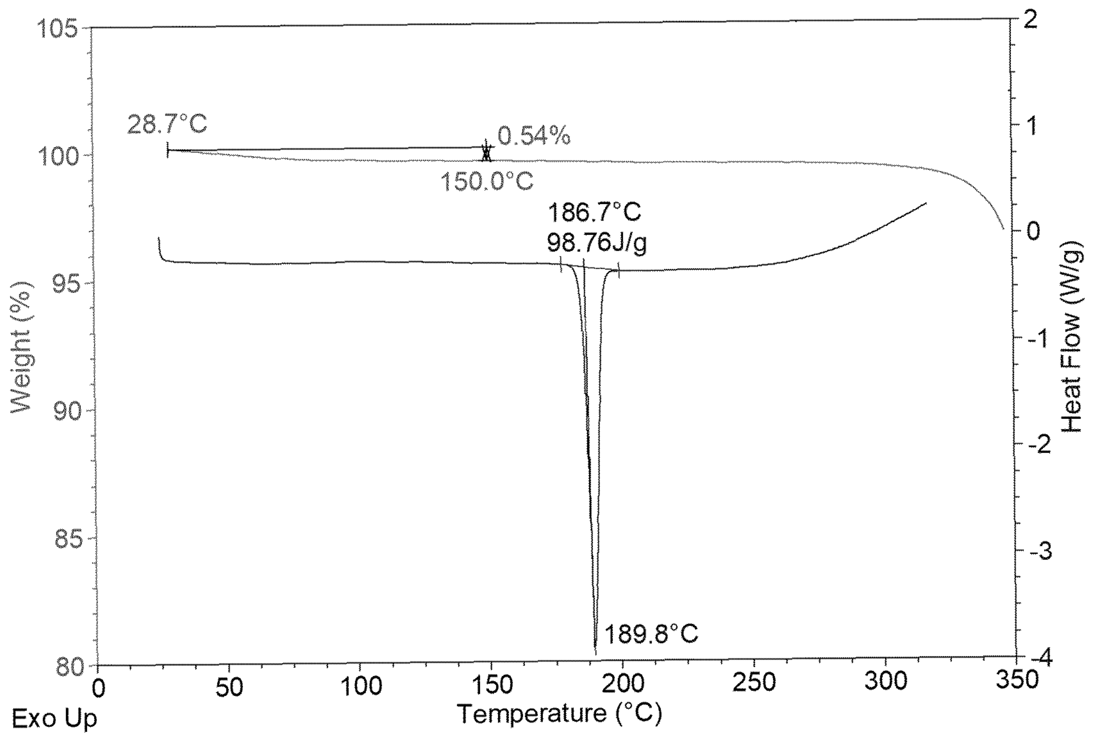
105
100
95
90
85
80
Weight (%)
28.7°C
0.54%
150.0°C
186.7°C
98.76J/g
189.8°C
2
1
0
-1
-2
-3
-4
Heat Flow (W/g)
0
50
100
150
200
250
300
350
Exo Up
Temperature (°C)

FIG. 2B

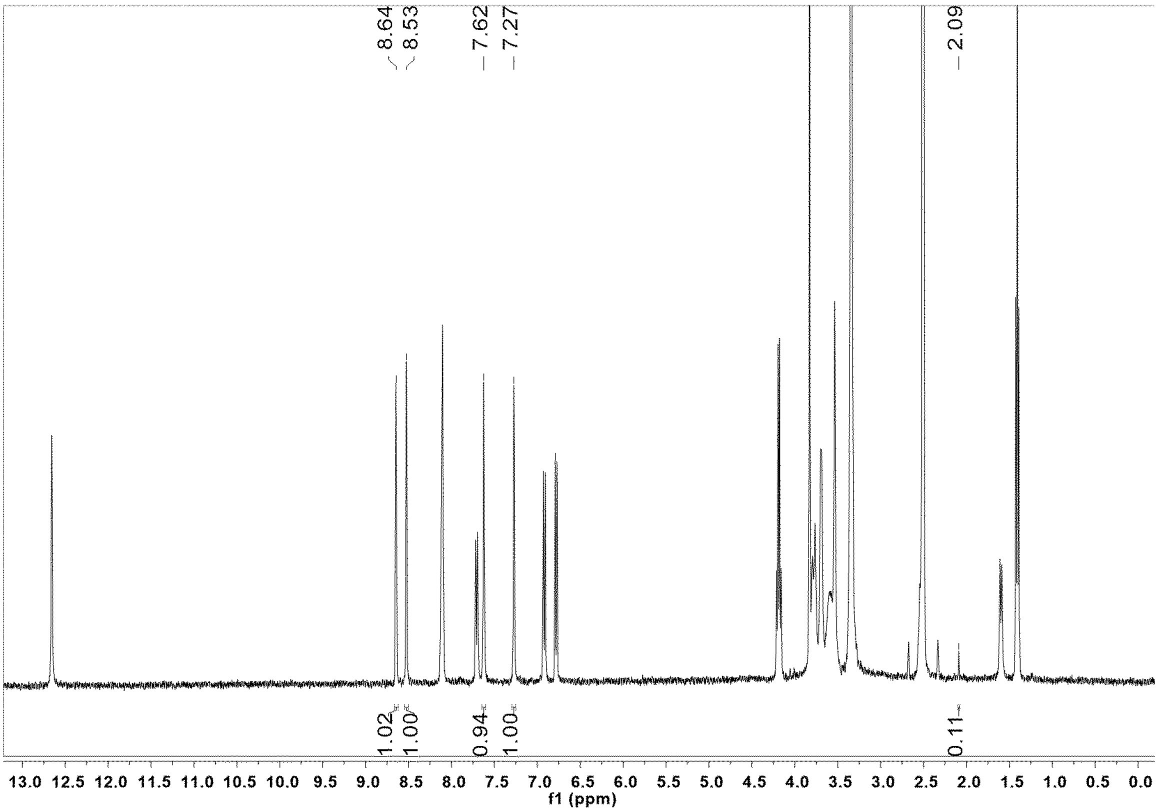
8.64
8.53
7.62
7.27
2.09
1.02
1.00
0.94
1.00
0.11
13.0 12.5 12.0 11.5 11.0 10.5 10.0 9.5 9.0 8.5 8.0 7.5 7.0 6.5 6.0 5.5 5.0 4.5 4.0 3.5 3.0 2.5 2.0 1.5 1.0 0.5 0.0
f1 (ppm)

FIG. 2C

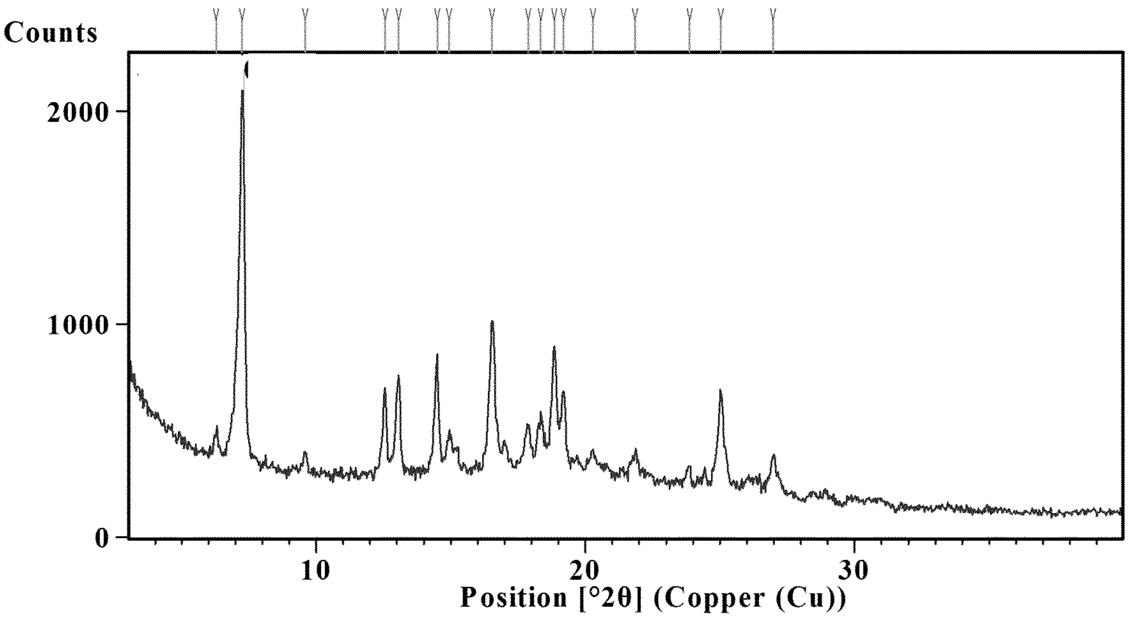
Counts
2000
1000
0
10
20
30
Position [°2θ] (Copper (Cu))

FIG. 3A

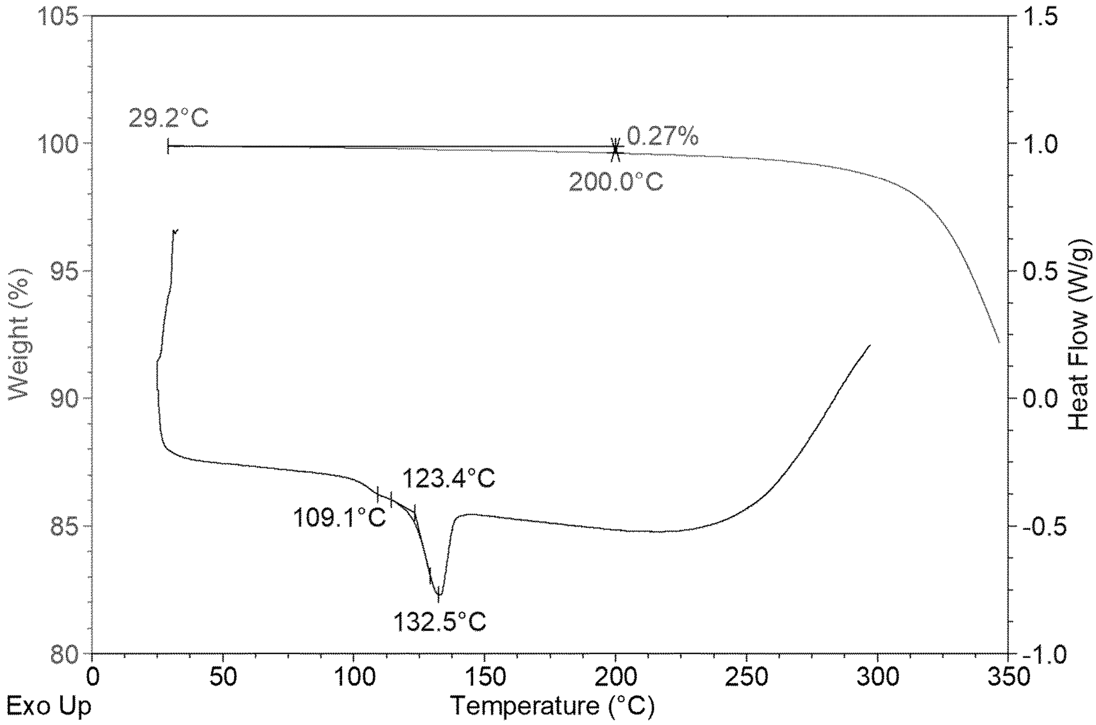
105
100
95
90
85
80
Weight (%)
1.5
1.0
0.5
0.0
-0.5
-1.0
Heat Flow (W/g)
29.2°C
0.27%
200.0°C
123.4°C
109.1°C
132.5°C
0
50
100
150
200
250
300
350
Exo Up
Temperature (°C)

FIG. 3B

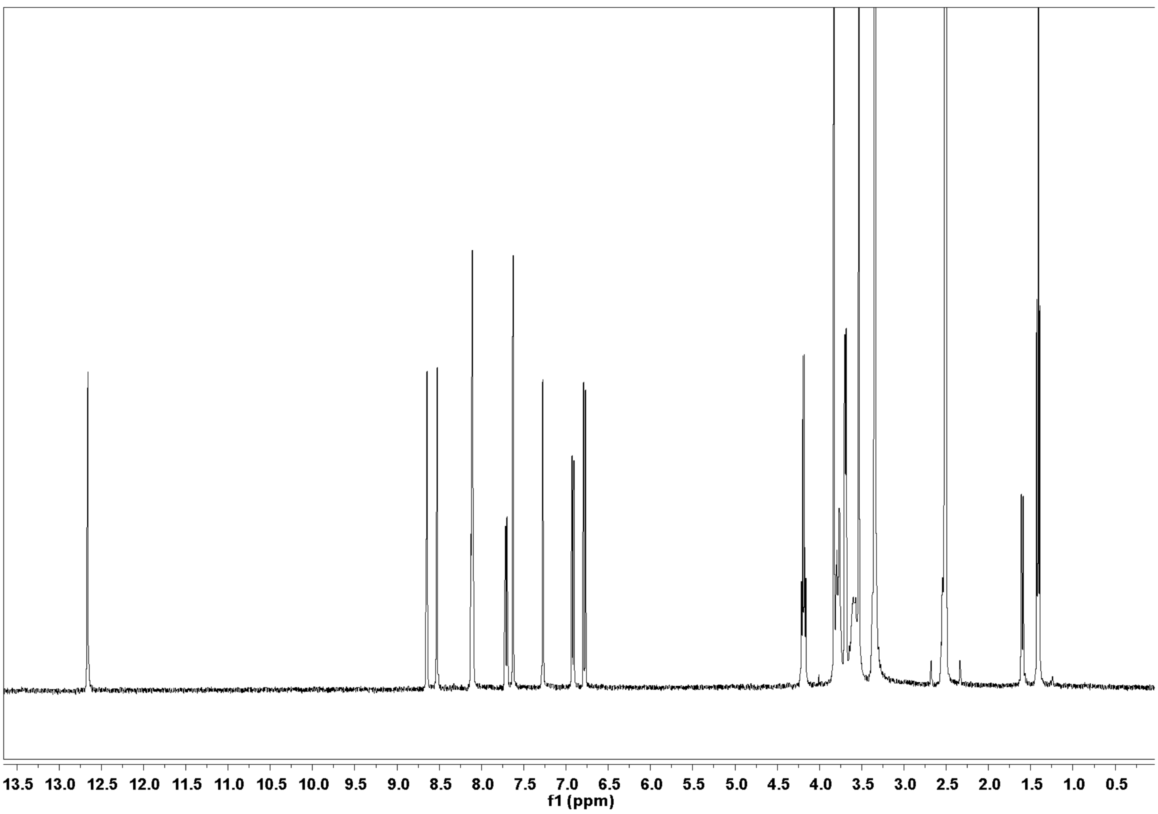
13.5 13.0 12.5 12.0 11.5 11.0 10.5 10.0 9.5 9.0 8.5 8.0 7.5 7.0 6.5 6.0 5.5 5.0 4.5 4.0 3.5 3.0 2.5 2.0 1.5 1.0 0.5
f1 (ppm)

FIG. 3C

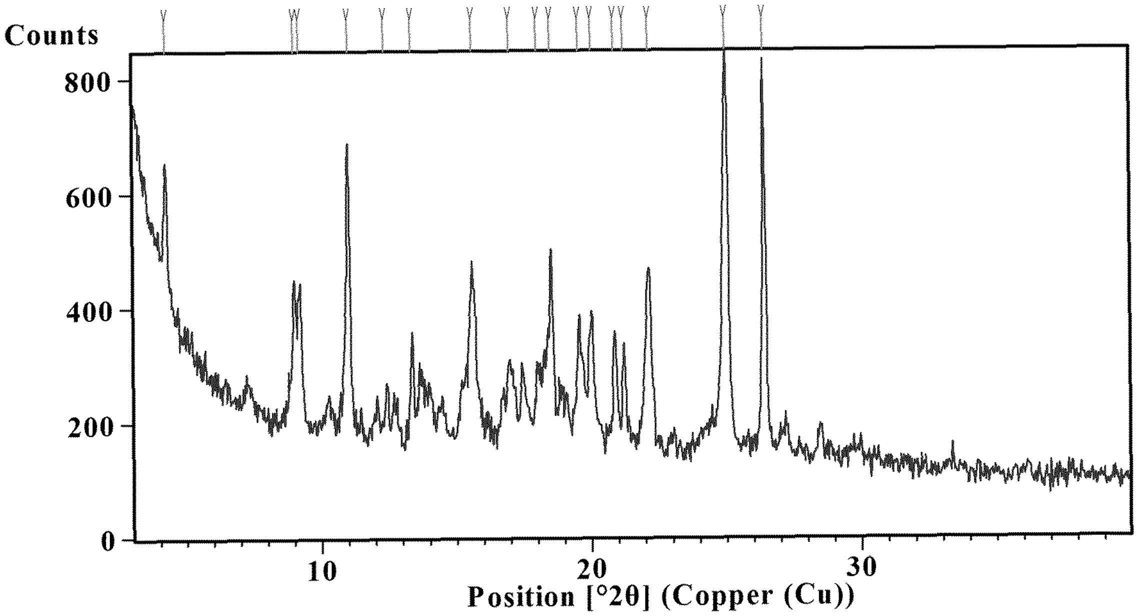
Counts
800
600
400
200
0
10
20
30
Position [°2θ] (Copper (Cu))

FIG. 4A

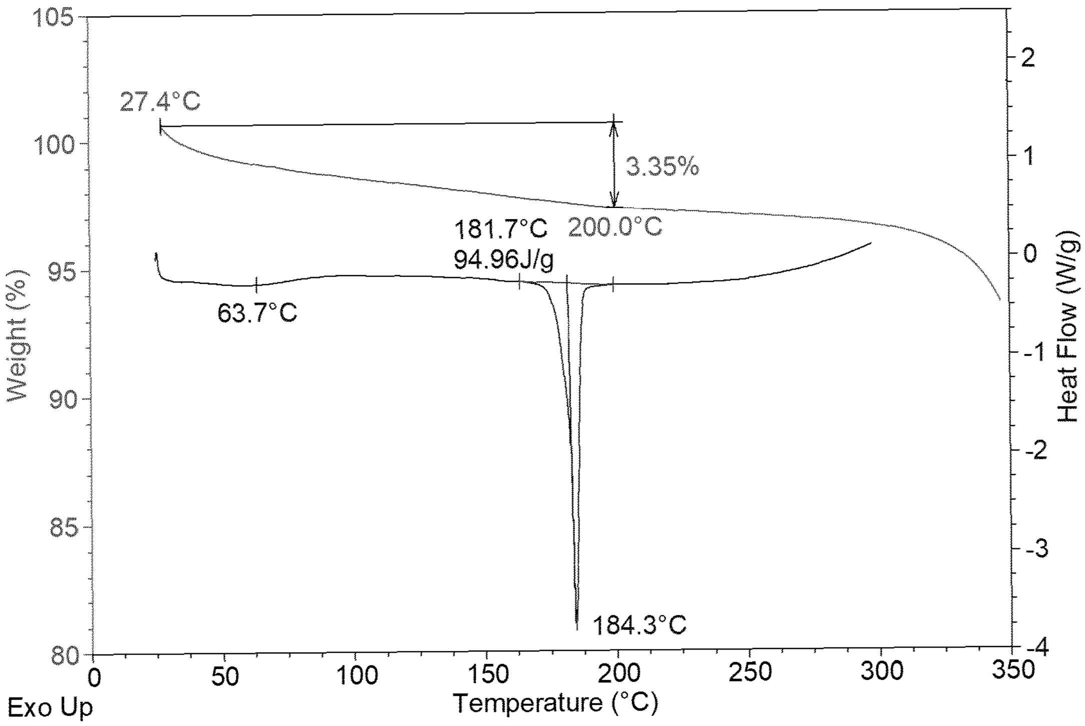
105
100
95
90
85
80
Weight (%)
2
1
0
-1
-2
-3
-4
Heat Flow (W/g)
27.4°C
3.35%
181.7°C
200.0°C
94.96J/g
63.7°C
184.3°C
0
50
100
150
200
250
300
350
Exo Up
Temperature (°C)

FIG. 4B

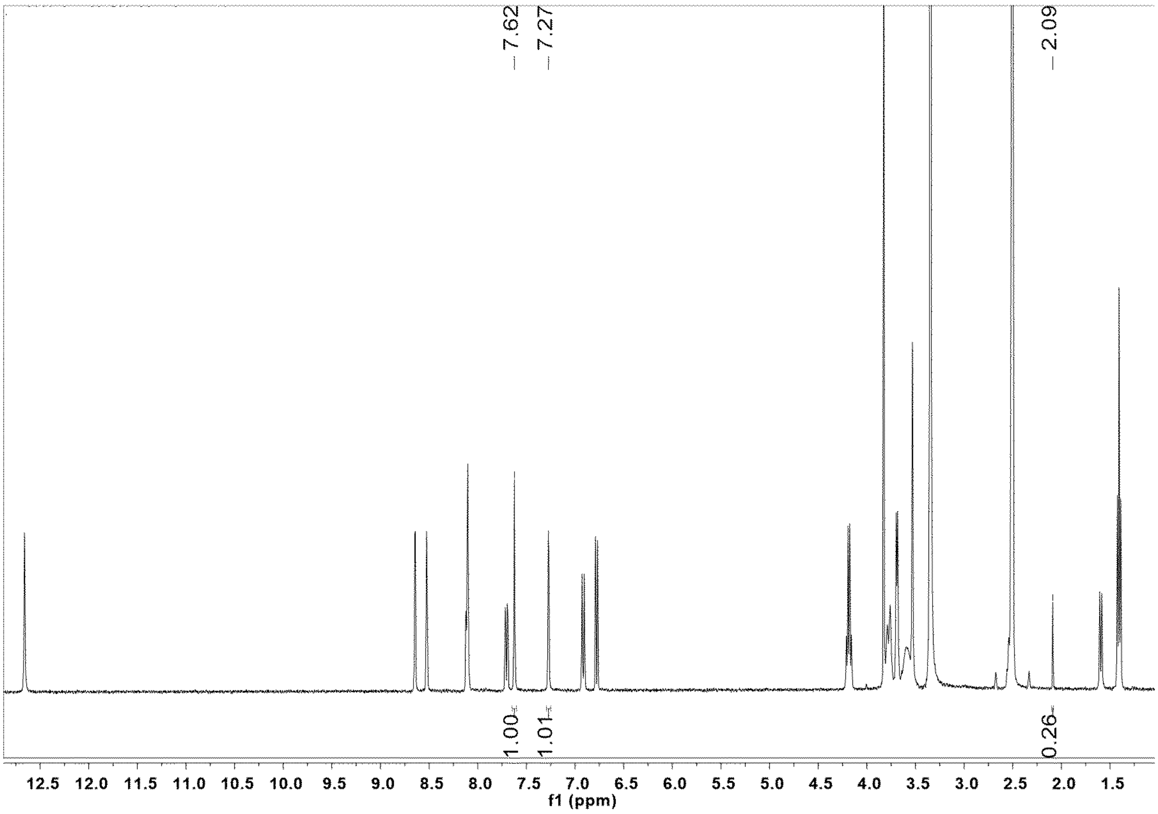
7.62
7.27
2.09
1.00
1.01
0.26
12.5 12.0 11.5 11.0 10.5 10.0 9.5 9.0 8.5 8.0 7.5 7.0 6.5 6.0 5.5 5.0 4.5 4.0 3.5 3.0 2.5 2.0 1.5
f1 (ppm)

**FIG. 4C**

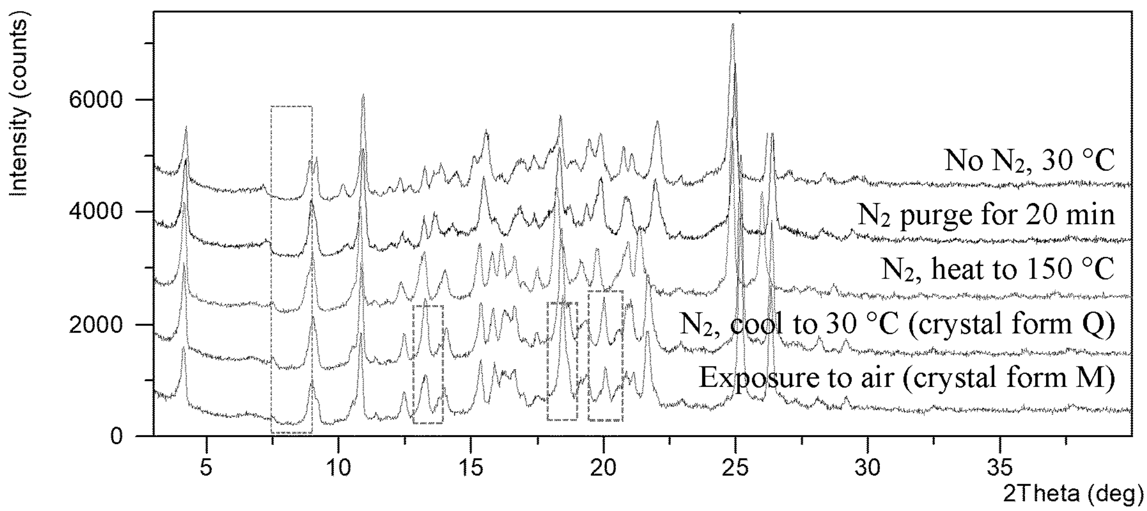
Intensity (counts)
6000
4000
2000
0
5
10
15
20
25
30
35
2Theta (deg)
No N2, 30 °C
N2 purge for 20 min
N2, heat to 150 °C
N2, cool to 30 °C (crystal form Q)
Exposure to air (crystal form M)

**FIG. 4D**

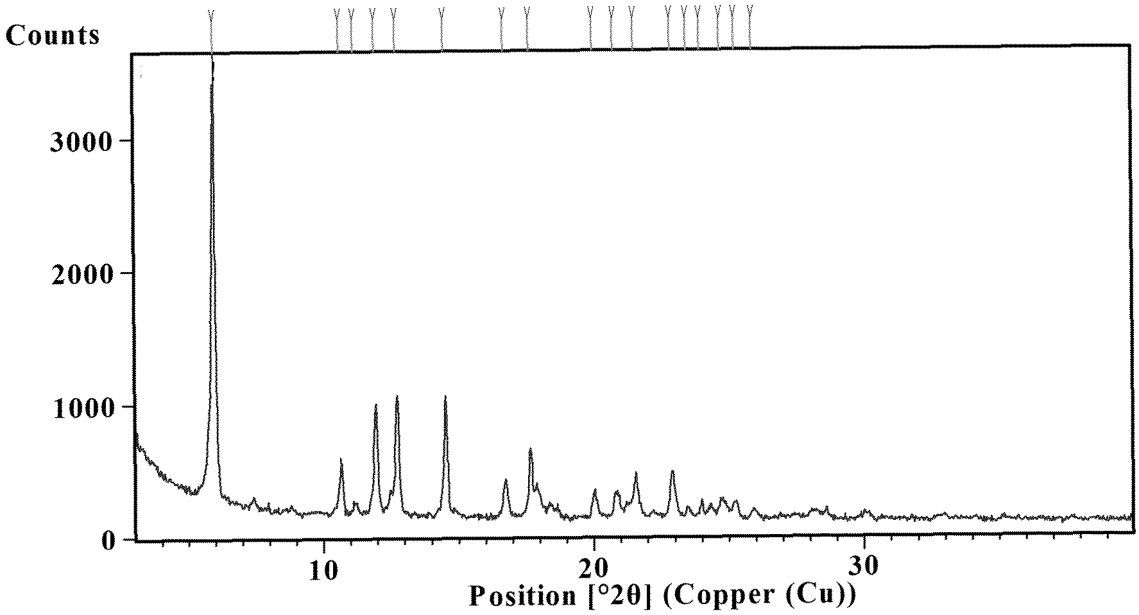
Counts
3000
2000
1000
0
10
20
30
Position [°2θ] (Copper (Cu))

FIG. 5A

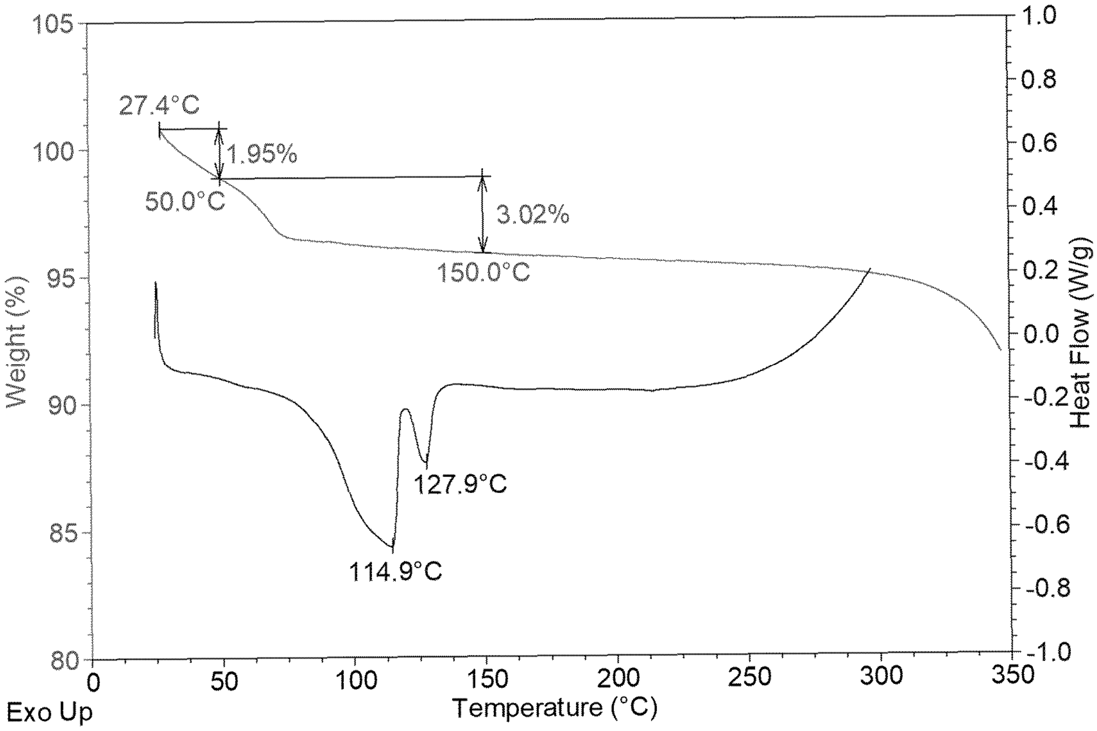
105
100
95
90
85
80
Weight (%)
27.4°C
1.95%
50.0°C
3.02%
150.0°C
127.9°C
114.9°C
1.0
0.8
0.6
0.4
0.2
0.0
-0.2
-0.4
-0.6
-0.8
-1.0
Heat Flow (W/g)
0
50
100
150
200
250
300
350
Exo Up
Temperature (°C)

FIG. 5B

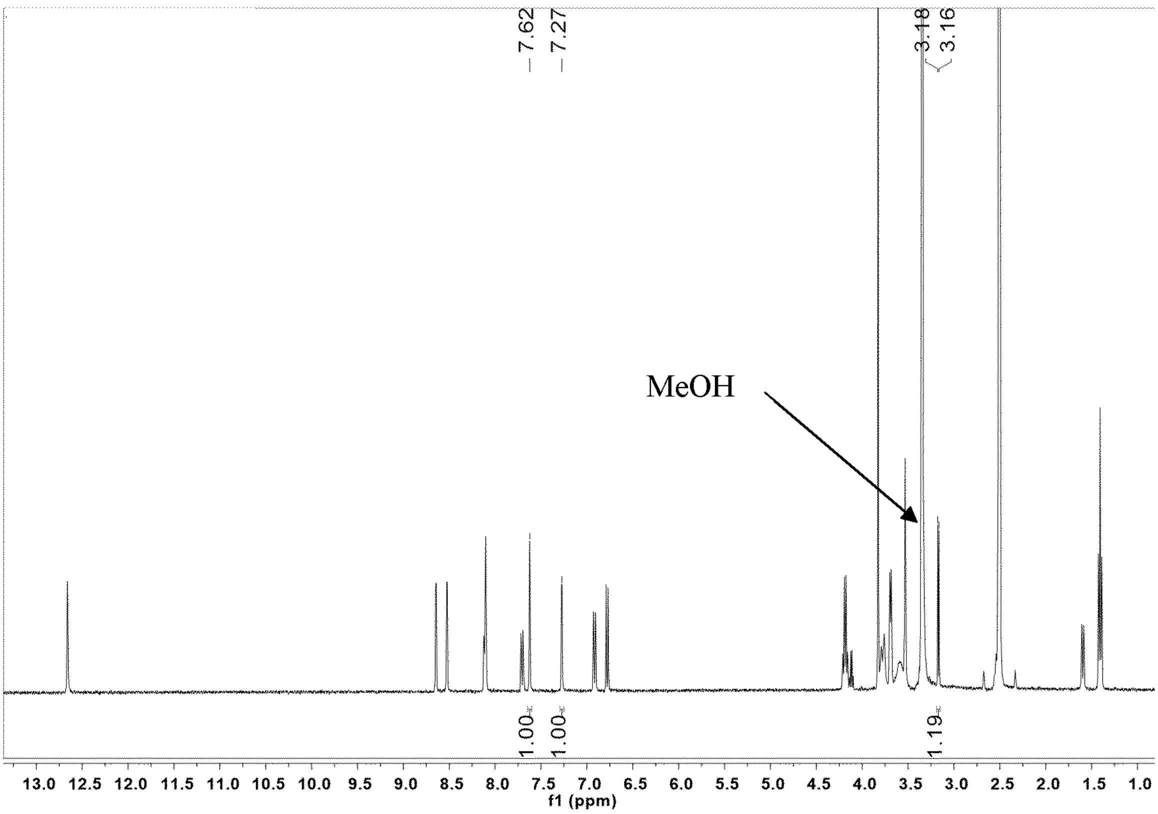
7.62
7.27
3.18
3.16
MeOH
1.00
1.00
1.19
13.0 12.5 12.0 11.5 11.0 10.5 10.0 9.5 9.0 8.5 8.0 7.5 7.0 6.5 6.0 5.5 5.0 4.5 4.0 3.5 3.0 2.5 2.0 1.5 1.0
f1 (ppm)

FIG. 5C

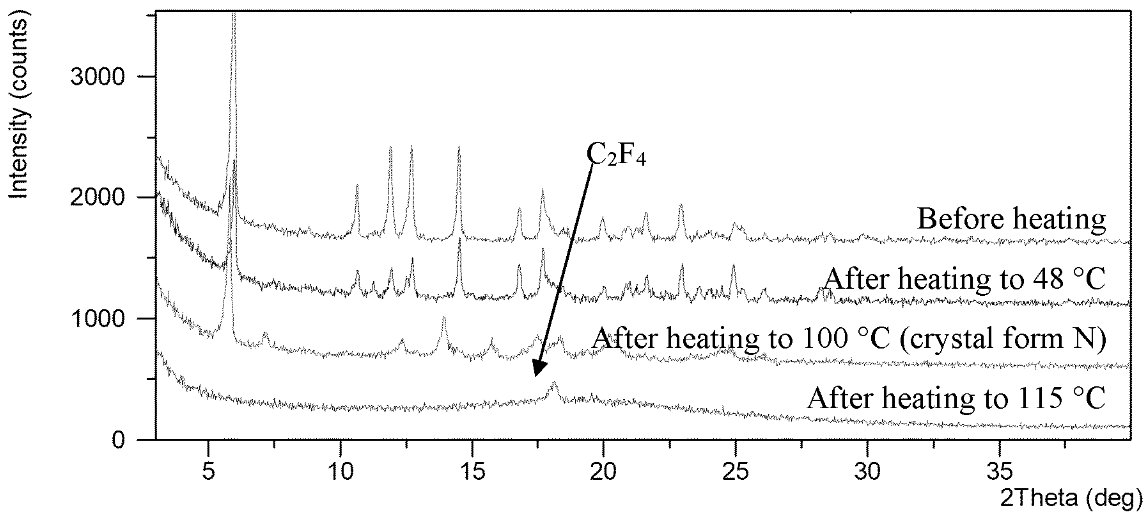
Intensity (counts)
3000
2000
1000
0
$C_2F_4$
Before heating
After heating to 48 °C
After heating to 100 °C (crystal form N)
After heating to 115 °C
5
10
15
20
25
30
35
2Theta (deg)

FIG. 5D

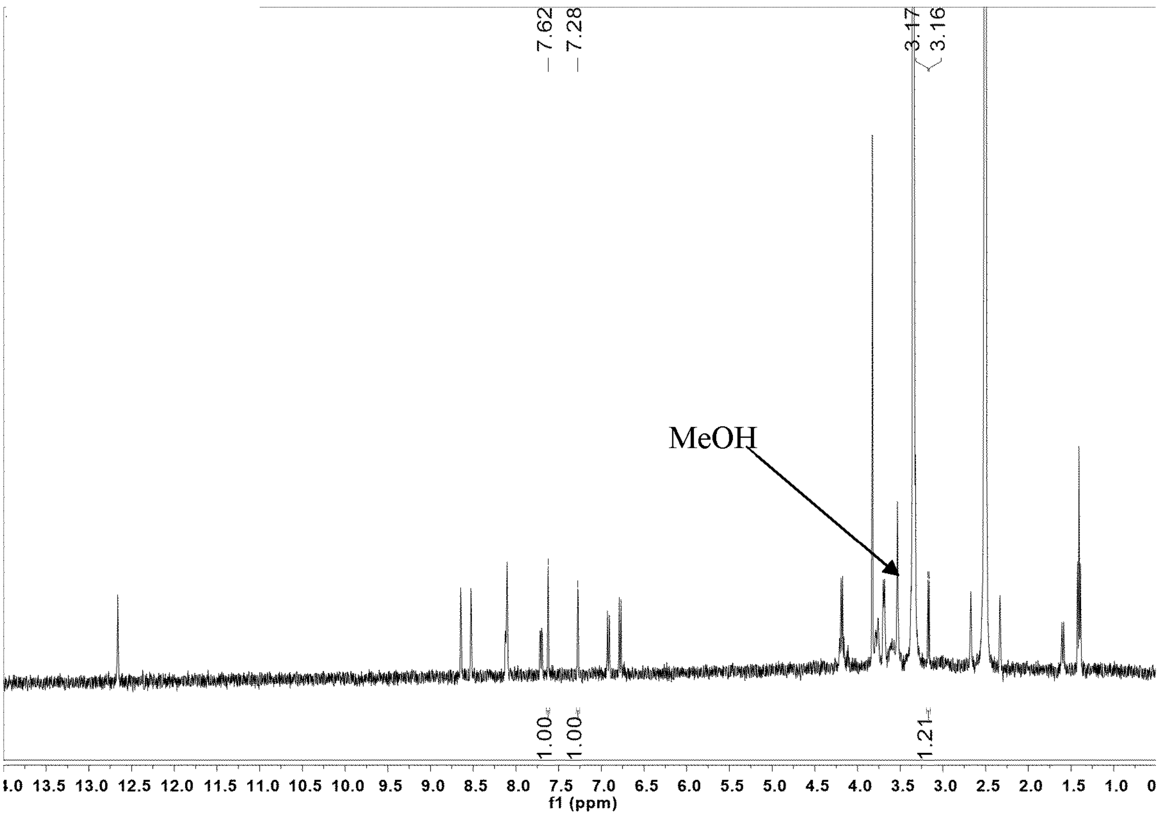
7.62
7.28
3.17
3.16
MeOH
1.00
1.00
1.21
f1 (ppm)

FIG. 5E

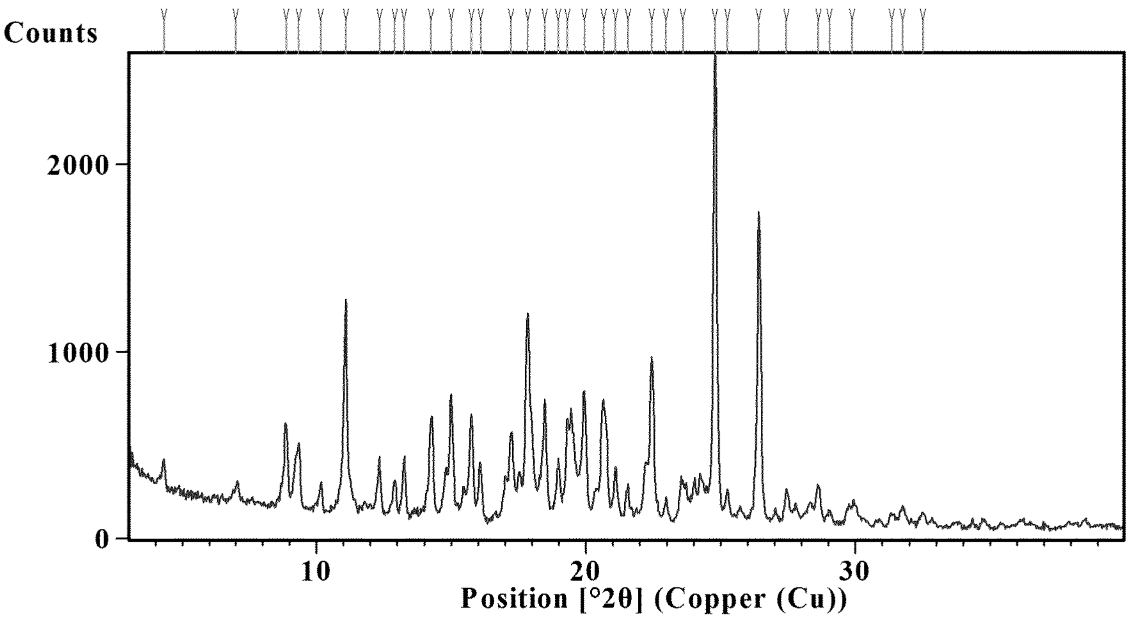
Counts
2000
1000
0
10
20
30
Position [°2θ] (Copper (Cu))

FIG. 6A

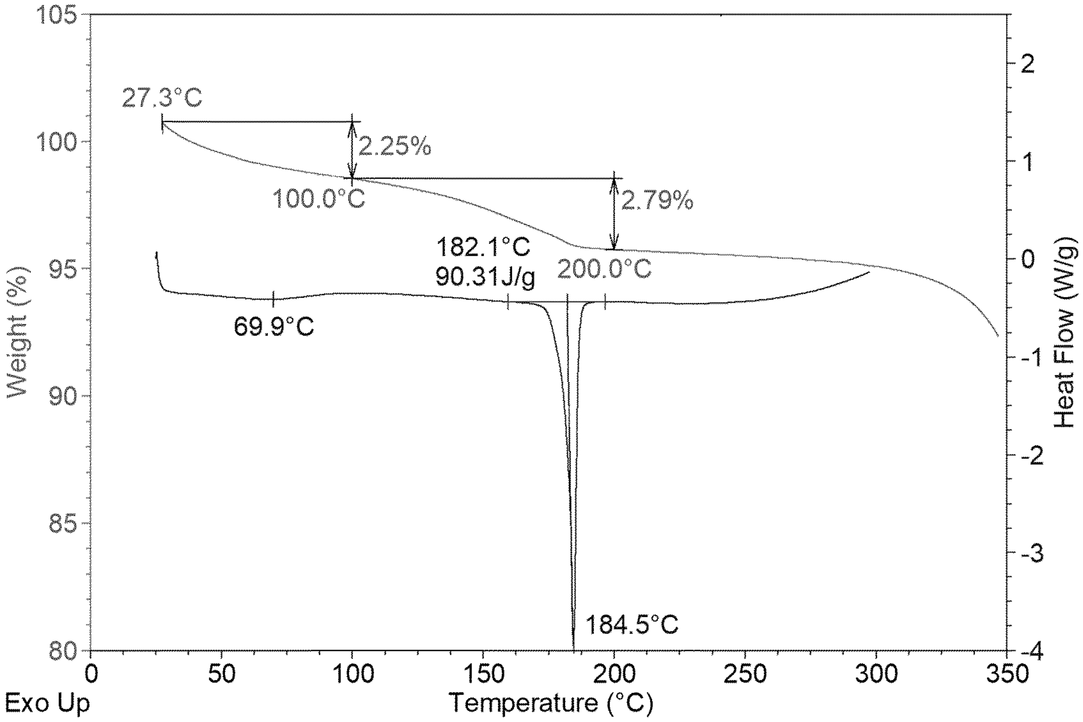
105
100
95
90
85
80
Weight (%)
2
1
0
-1
-2
-3
-4
Heat Flow (W/g)
27.3°C
2.25%
100.0°C
2.79%
182.1°C
90.31J/g
200.0°C
69.9°C
184.5°C
0
50
100
150
200
250
300
350
Exo Up
Temperature (°C)

FIG. 6B

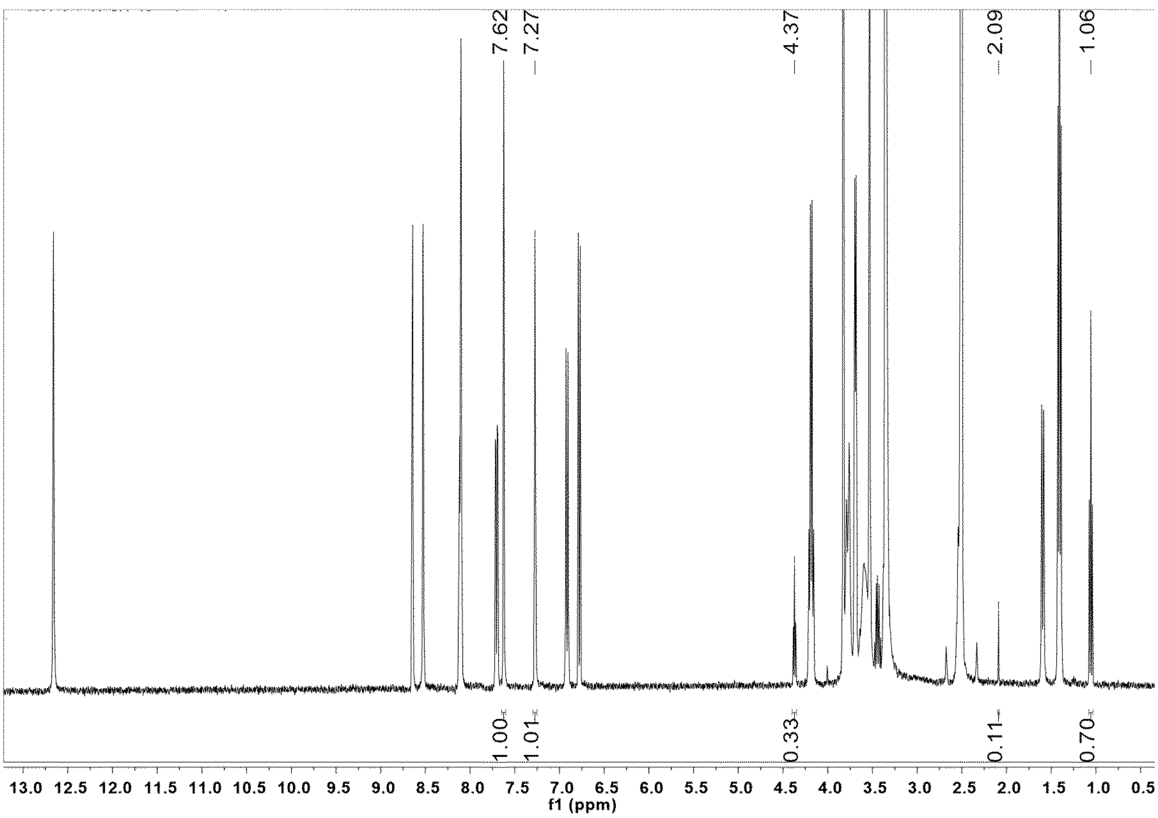
7.62
7.27
4.37
2.09
1.06
1.00
1.01
0.33
0.11
0.70
13.0 12.5 12.0 11.5 11.0 10.5 10.0 9.5 9.0 8.5 8.0 7.5 7.0 6.5 6.0 5.5 5.0 4.5 4.0 3.5 3.0 2.5 2.0 1.5 1.0 0.5
f1 (ppm)

FIG. 6C

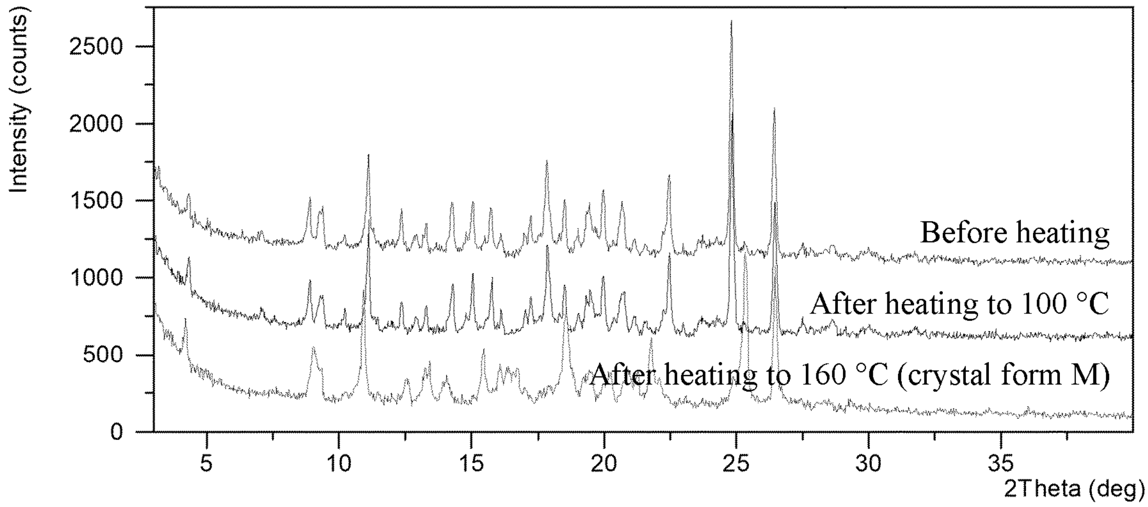
Intensity (counts)
2500
2000
1500
1000
500
0
5
10
15
20
25
30
35
2Theta (deg)
Before heating
After heating to 100 °C
After heating to 160 °C (crystal form M)

FIG. 6D

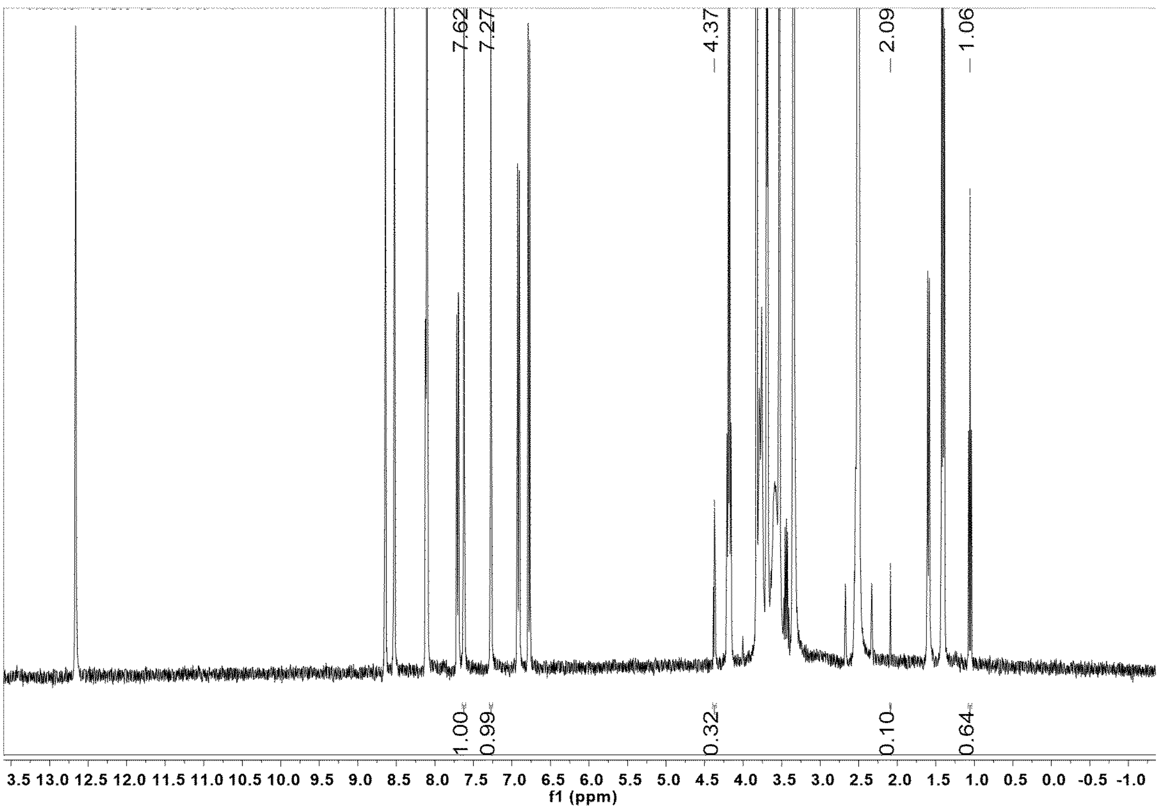
7.62
7.27
4.37
2.09
1.06
1.00
0.99
0.32
0.10
0.64
3.5 13.0 12.5 12.0 11.5 11.0 10.5 10.0 9.5 9.0 8.5 8.0 7.5 7.0 6.5 6.0 5.5 5.0 4.5 4.0 3.5 3.0 2.5 2.0 1.5 1.0 0.5 0.0 -0.5 -1.0
f1 (ppm)

FIG. 6E

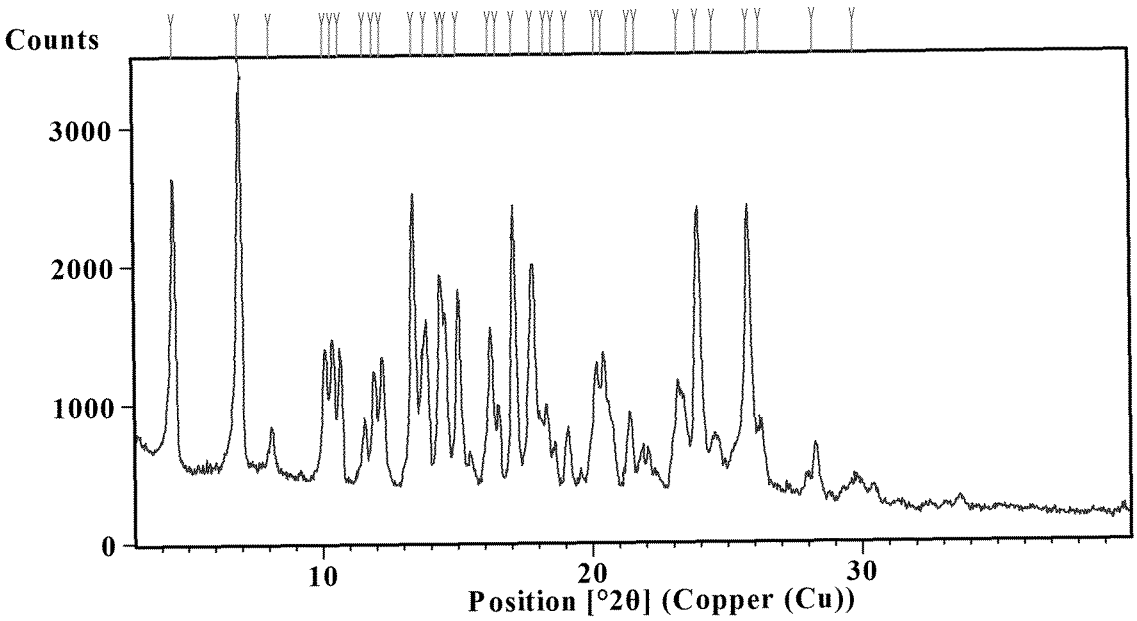
Counts
3000
2000
1000
0
10
20
30
Position [°2θ] (Copper (Cu))

FIG. 7A

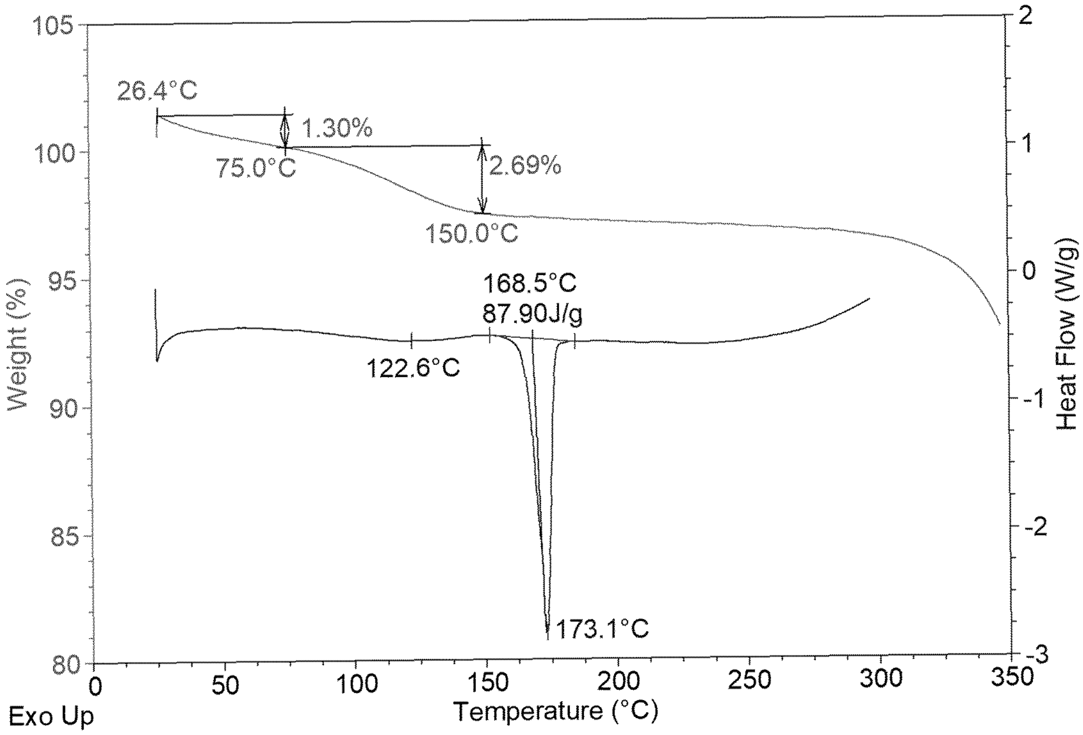
105
100
95
90
85
80
2
1
0
-1
-2
-3
26.4°C
1.30%
75.0°C
2.69%
150.0°C
168.5°C
87.90J/g
122.6°C
173.1°C
Weight (%)
Heat Flow (W/g)
0
50
100
150
200
250
300
350
Exo Up
Temperature (°C)

FIG. 7B

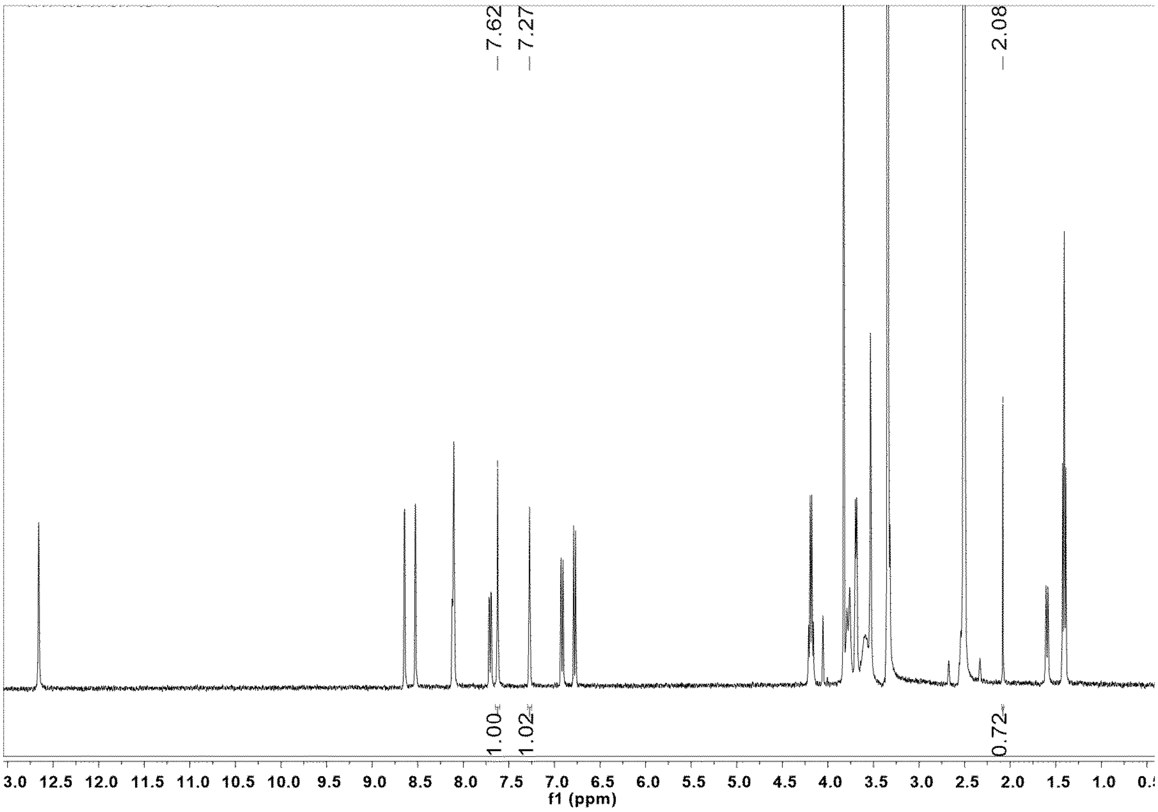
7.62
7.27
2.08
1.00
1.02
0.72
3.0 12.5 12.0 11.5 11.0 10.5 10.0 9.5 9.0 8.5 8.0 7.5 7.0 6.5 6.0 5.5 5.0 4.5 4.0 3.5 3.0 2.5 2.0 1.5 1.0
f1 (ppm)

FIG. 7C

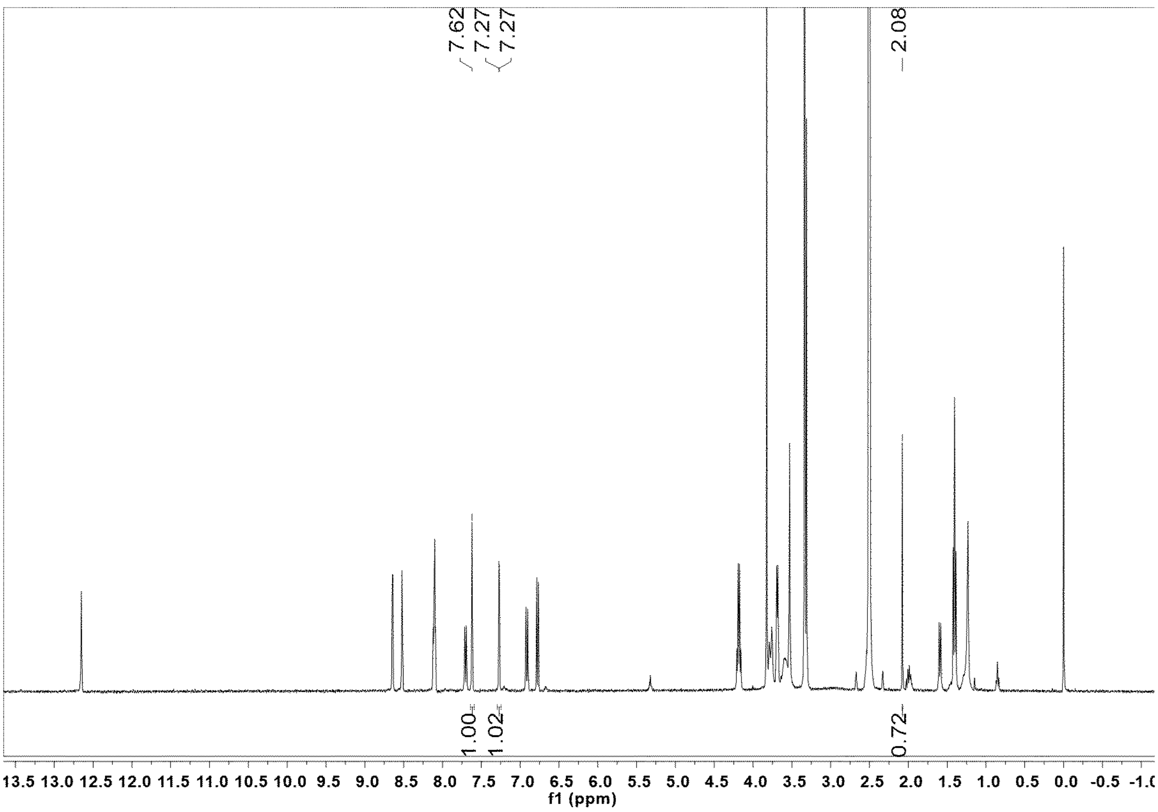
7.62
7.27
7.27
2.08
1.00
1.02
0.72
13.5 13.0 12.5 12.0 11.5 11.0 10.5 10.0 9.5 9.0 8.5 8.0 7.5 7.0 6.5 6.0 5.5 5.0 4.5 4.0 3.5 3.0 2.5 2.0 1.5 1.0 0.5 0.0 -0.5
f1 (ppm)

FIG. 7D

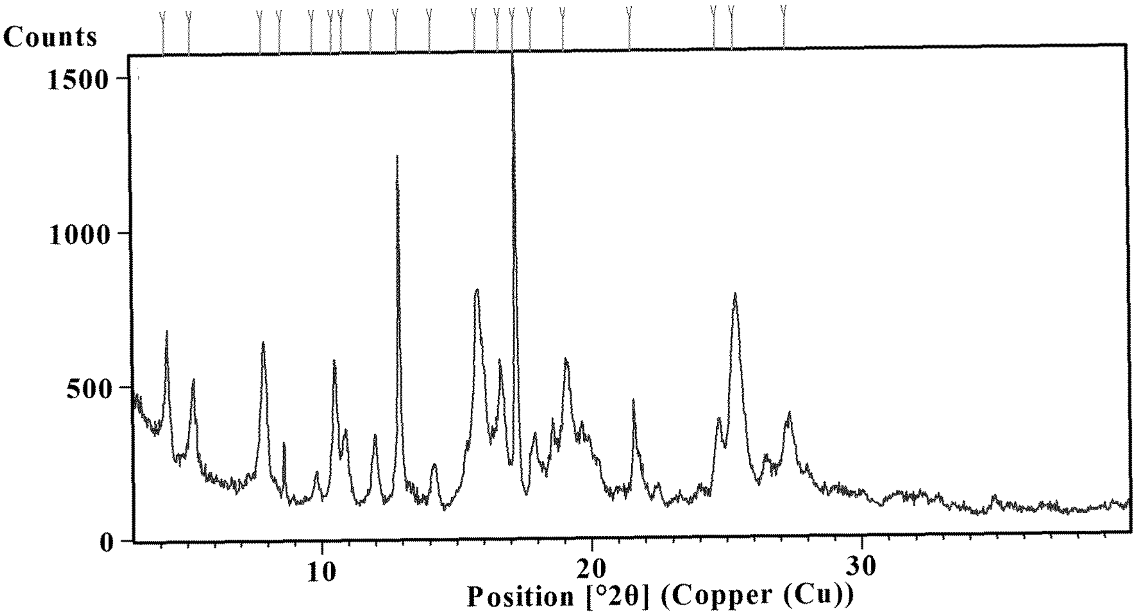
Counts
1500
1000
500
0
10
20
30
Position [°2θ] (Copper (Cu))

FIG. 8A

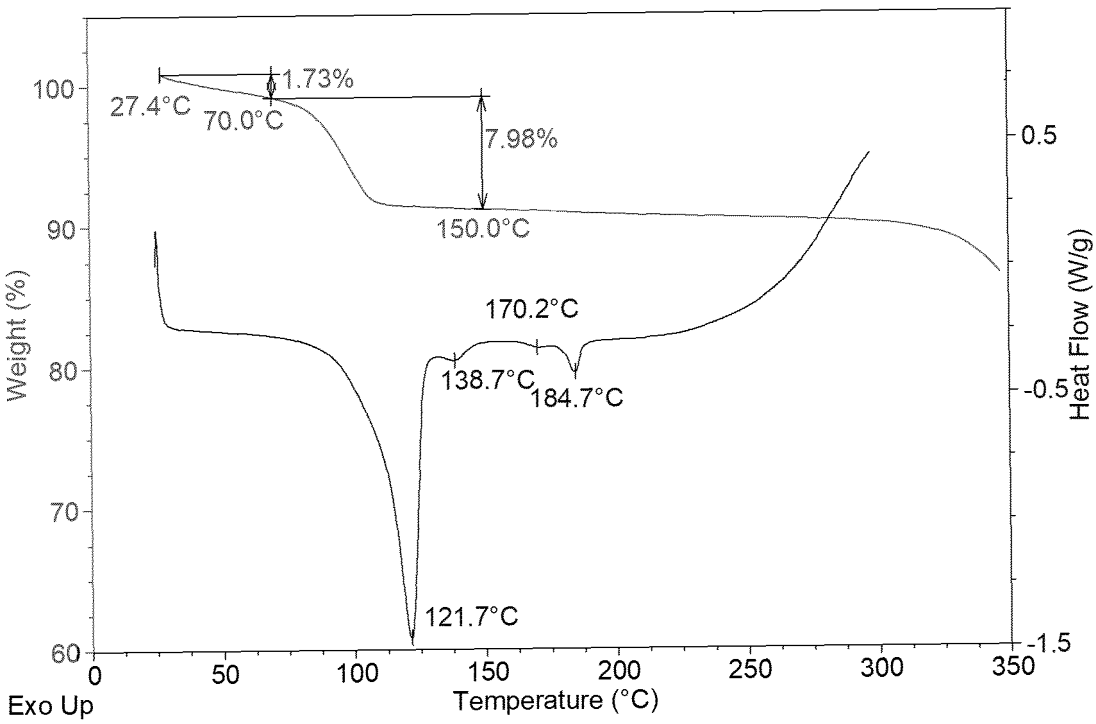
100
90
80
70
60
Weight (%)
27.4°C
70.0°C
1.73%
7.98%
150.0°C
170.2°C
138.7°C
184.7°C
121.7°C
0.5
-0.5
-1.5
Heat Flow (W/g)
0
50
100
150
200
250
300
350
Exo Up
Temperature (°C)

FIG. 8B

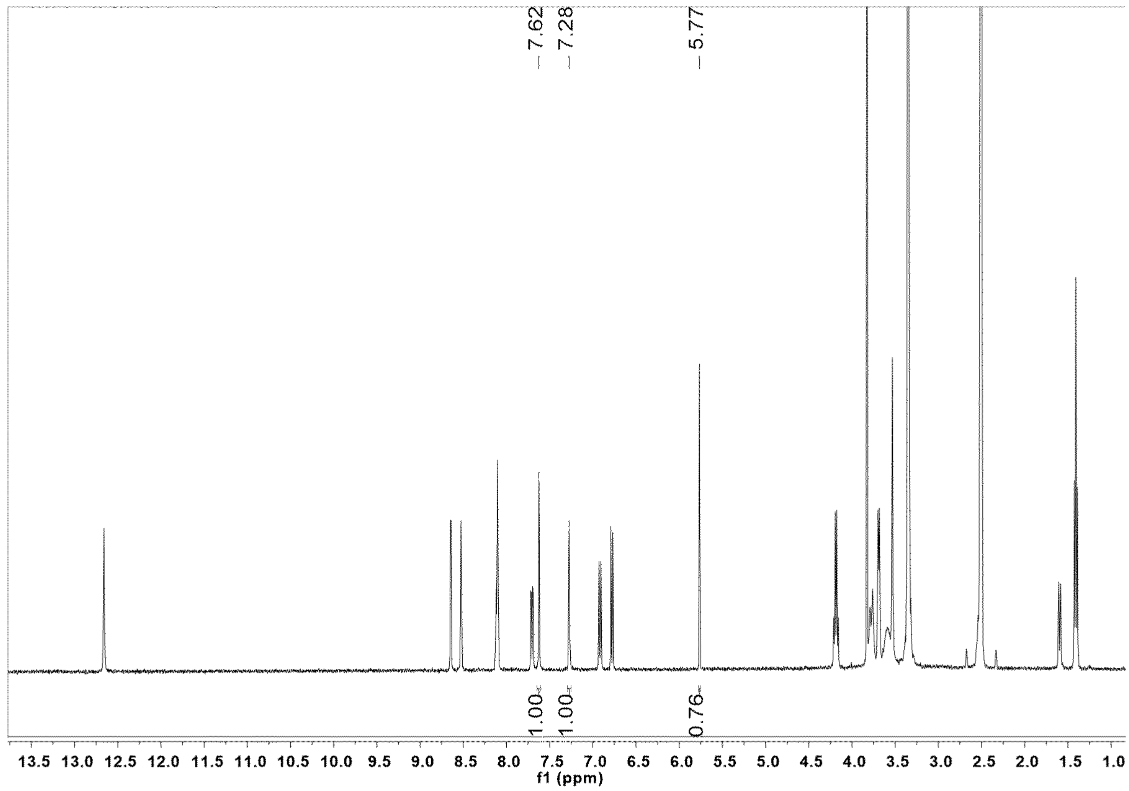
7.62
7.28
5.77
1.00
1.00
0.76
13.5 13.0 12.5 12.0 11.5 11.0 10.5 10.0 9.5 9.0 8.5 8.0 7.5 7.0 6.5 6.0 5.5 5.0 4.5 4.0 3.5 3.0 2.5 2.0 1.5 1.0
f1 (ppm)

FIG. 8C

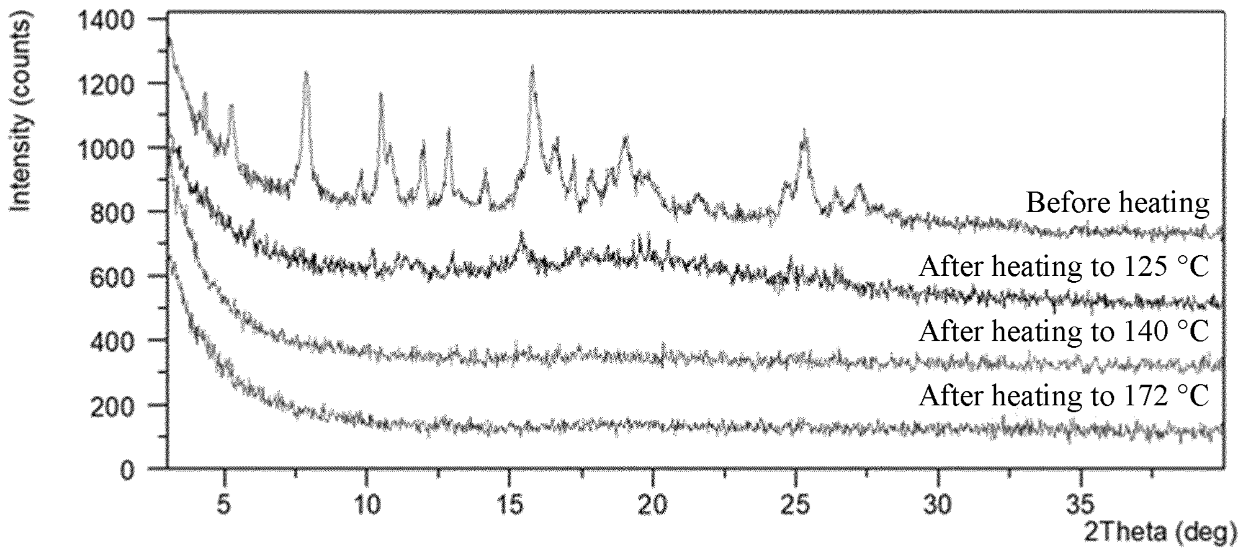
Intensity (counts)
1400
1200
1000
800
600
400
200
0
5
10
15
20
25
30
35
2Theta (deg)
Before heating
After heating to 125 °C
After heating to 140 °C
After heating to 172 °C

FIG. 8D

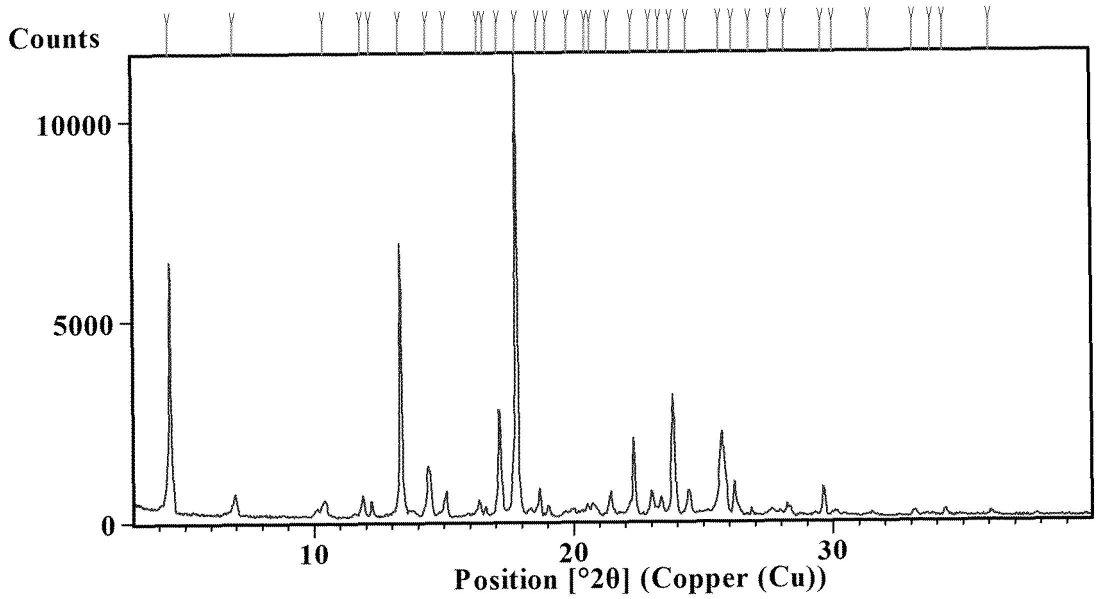
Counts
10000
5000
0
10
20
30
Position [°2θ] (Copper (Cu))

FIG. 9

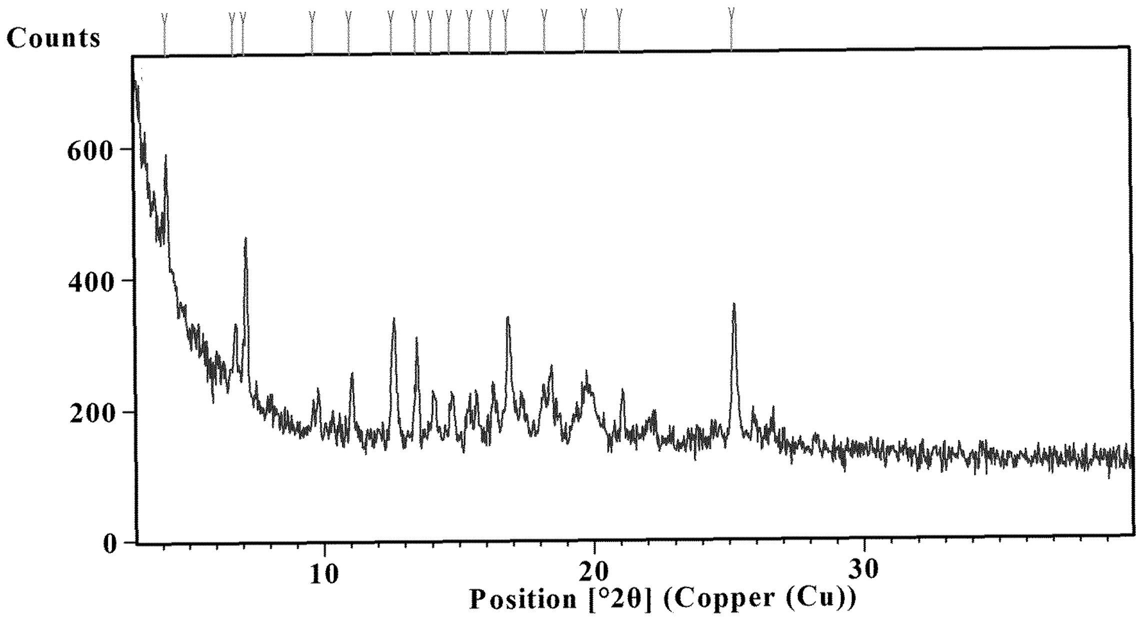
Counts
600
400
200
0
10
20
30
Position [°2θ] (Copper (Cu))

FIG. 10A

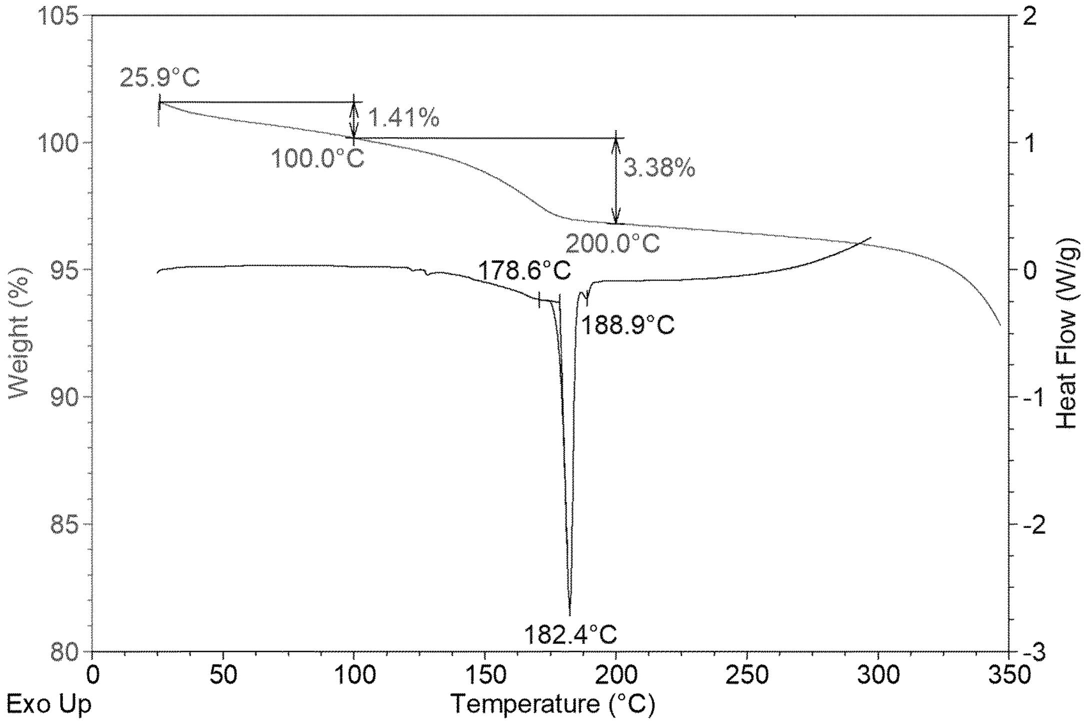


FIG. 10B

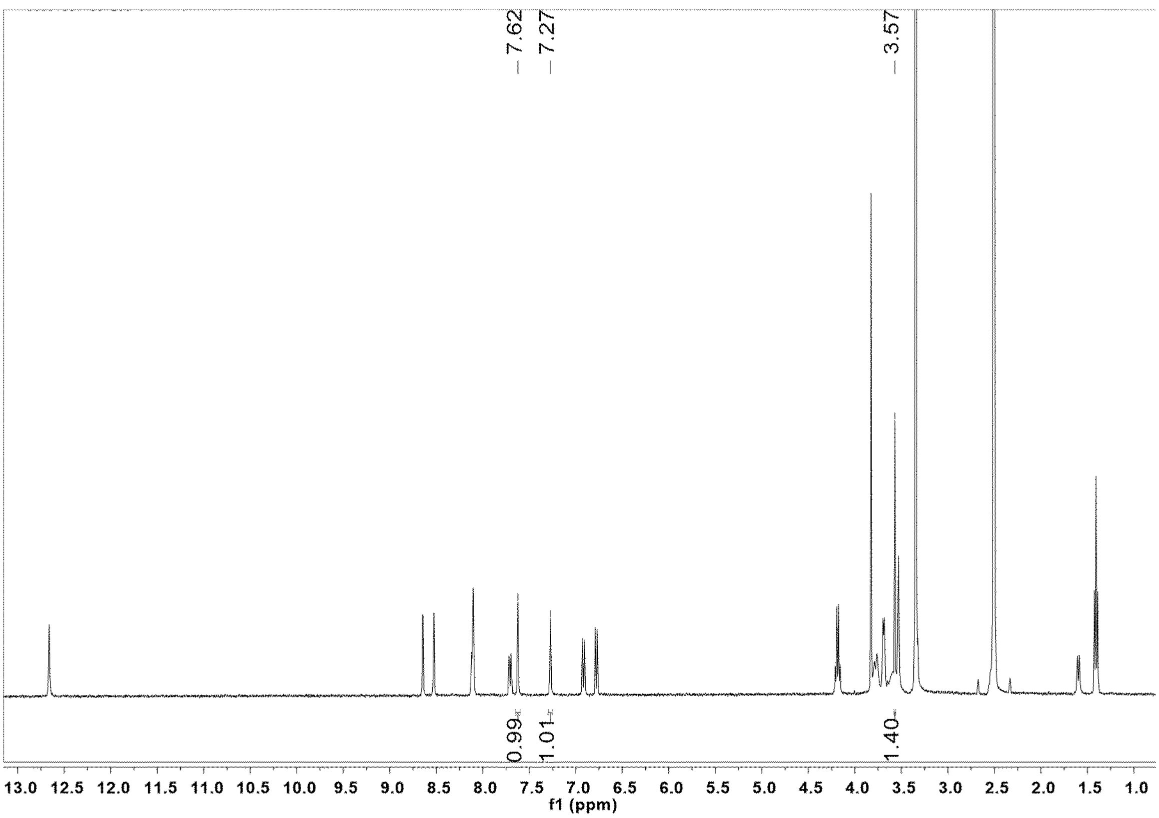


FIG. 10C

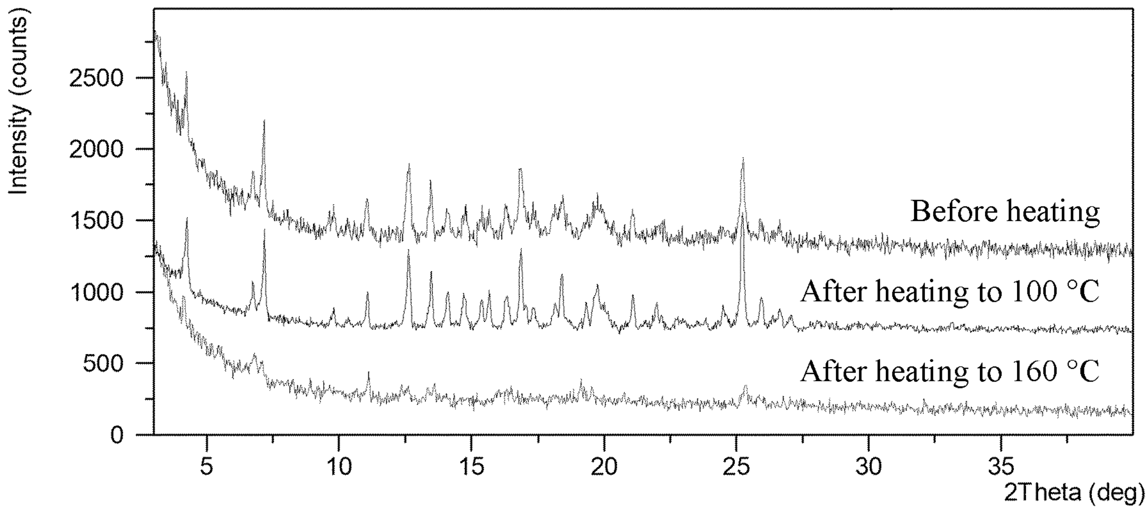


FIG. 10D

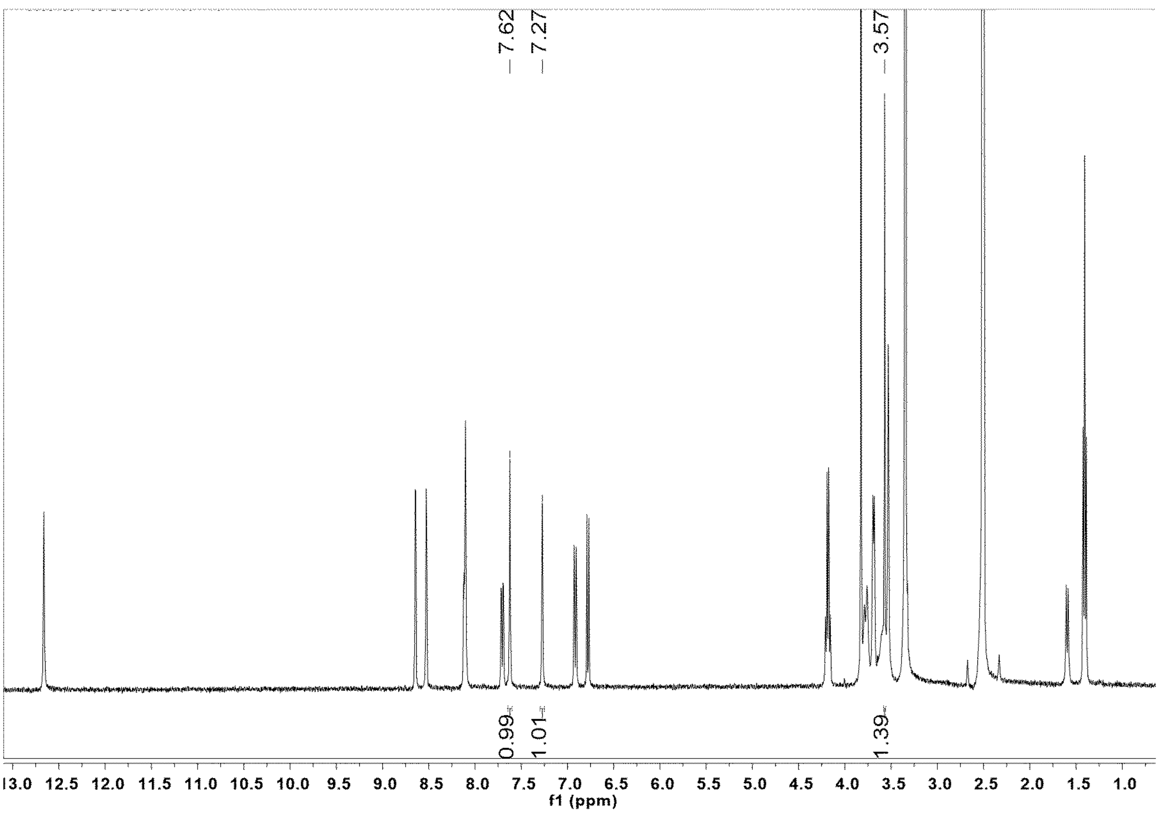


FIG. 10E

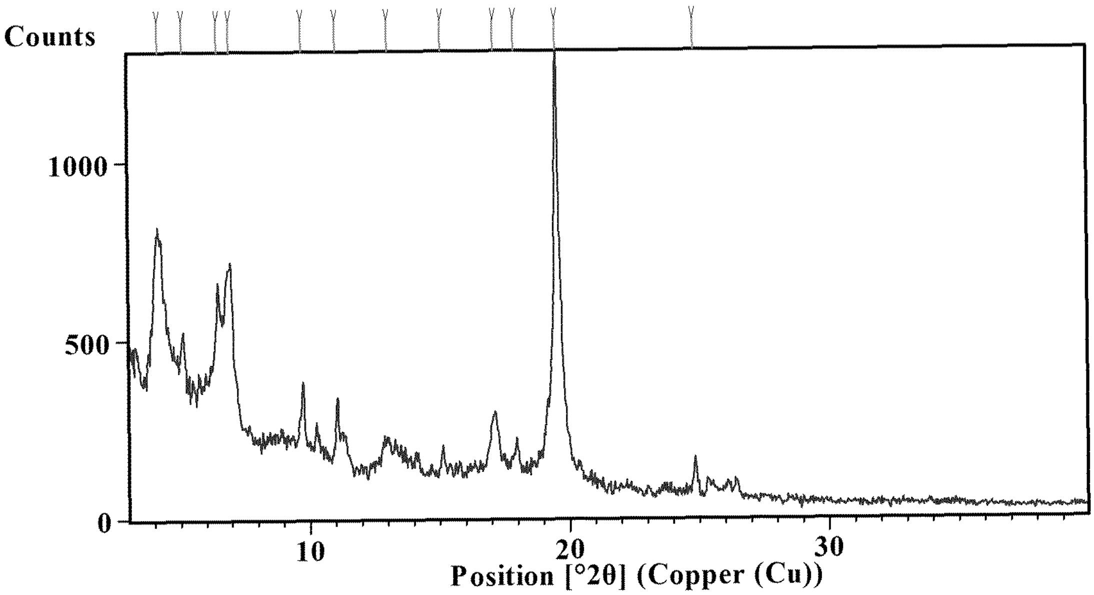


FIG. 11A

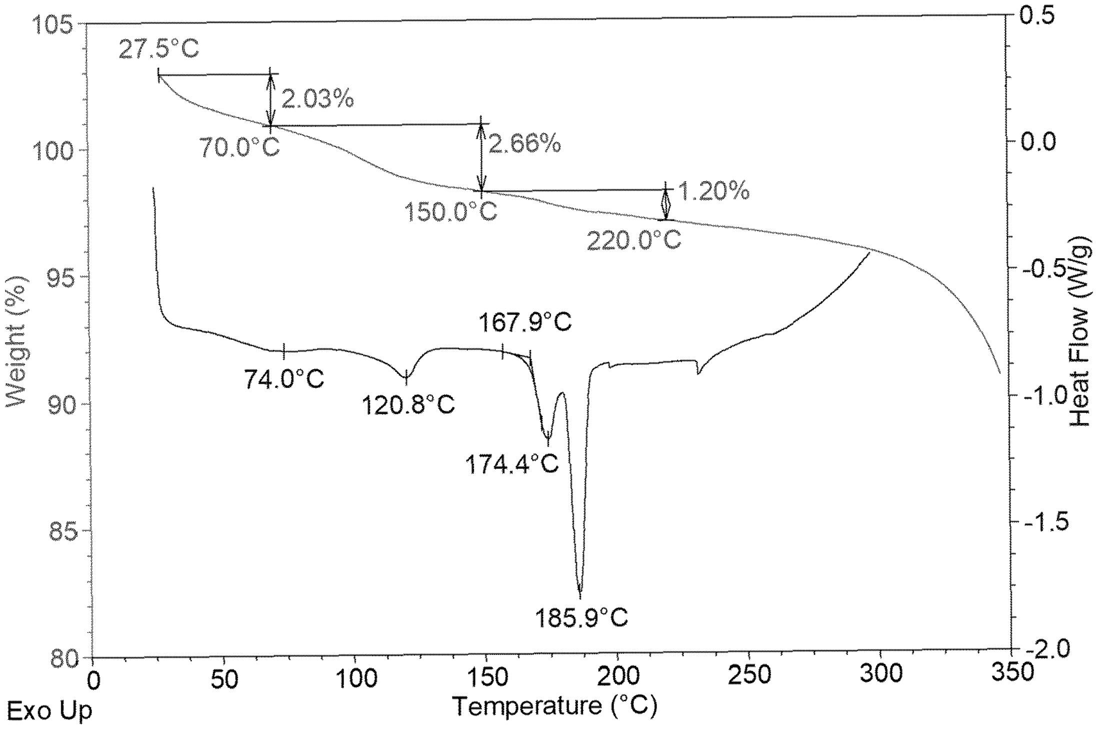


FIG. 11B

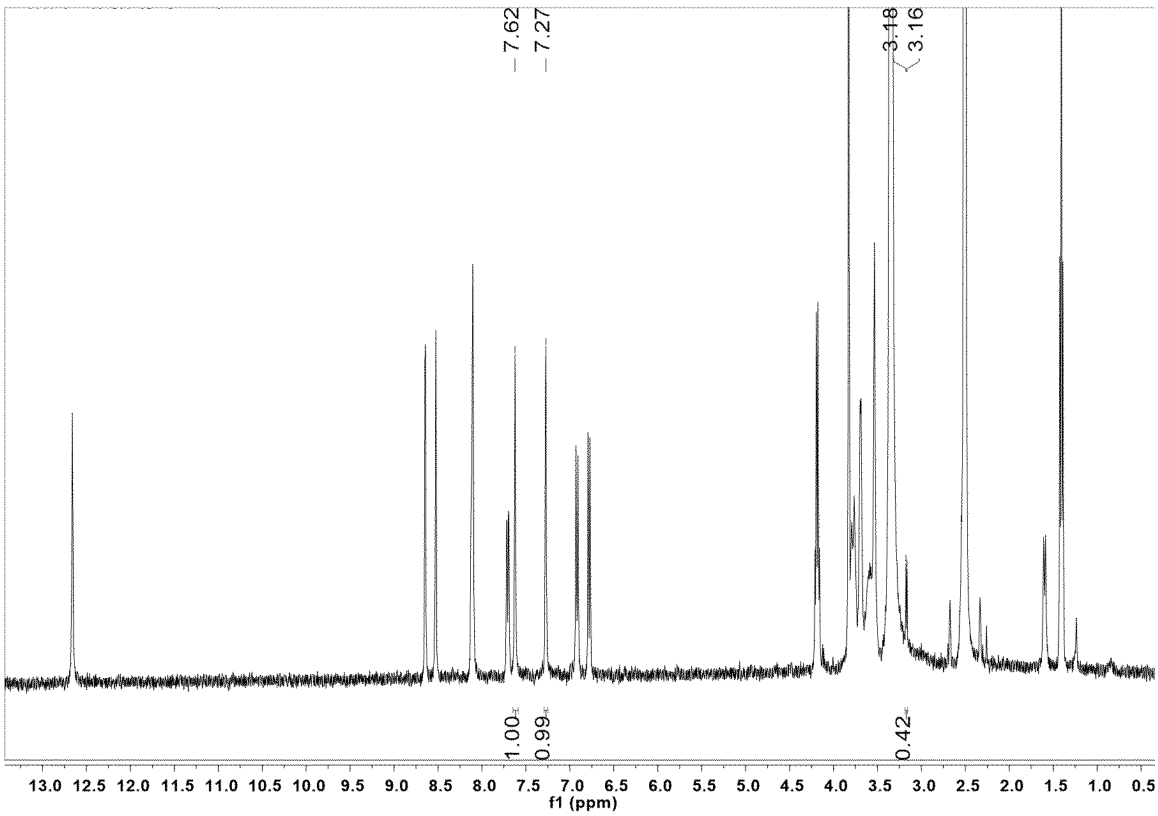


FIG. 11C

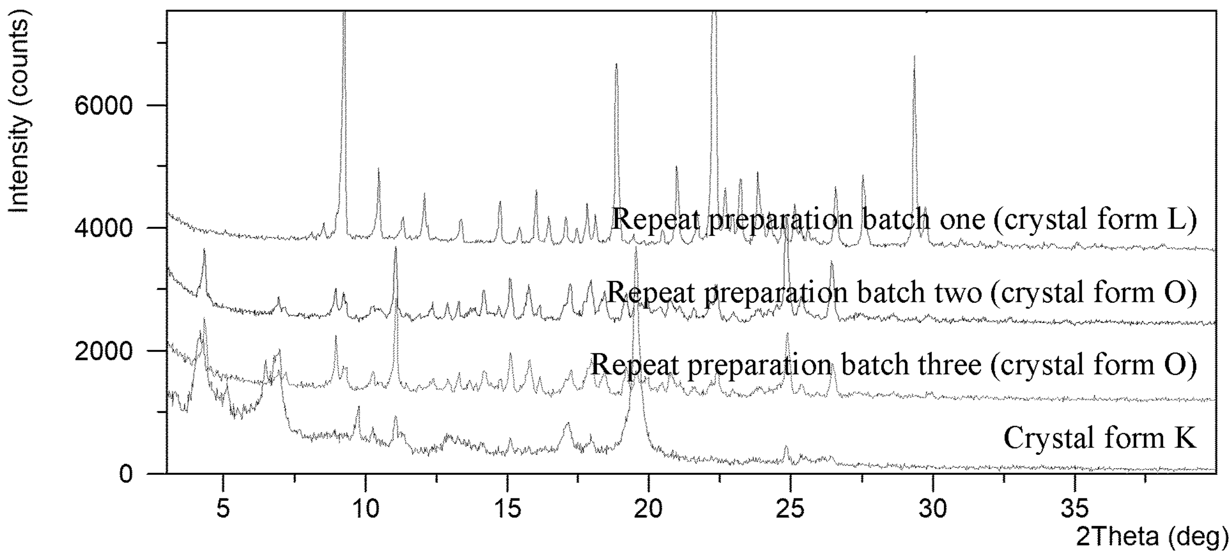


FIG. 11D

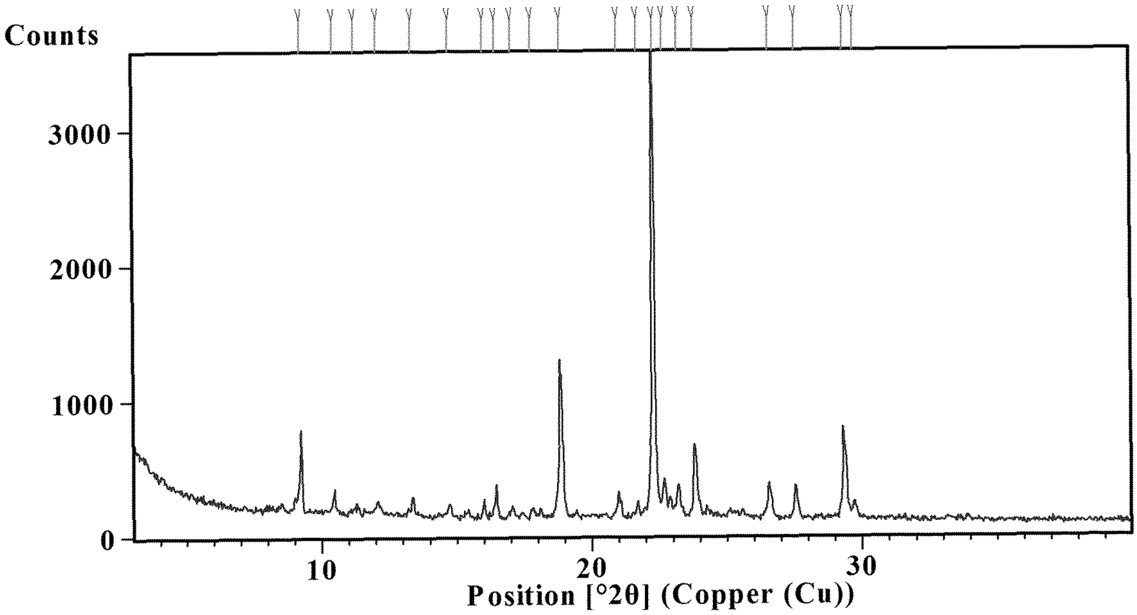


FIG. 12A

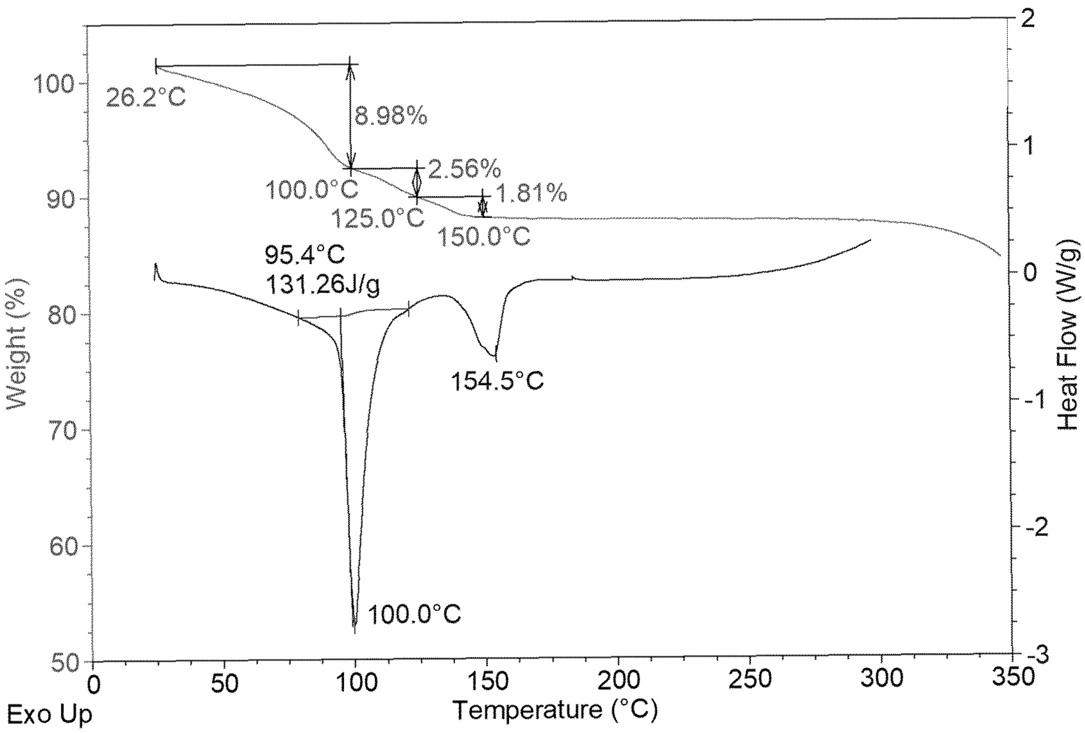


FIG. 12B

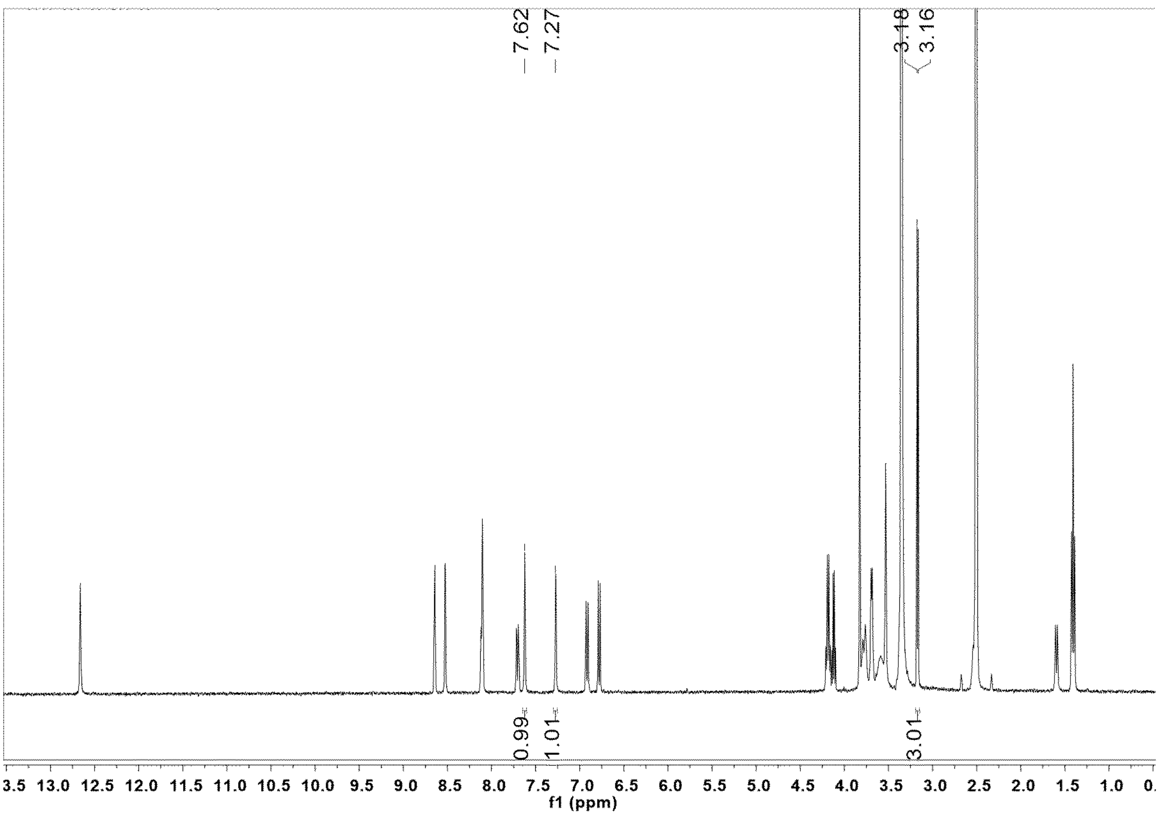


**FIG. 12C**

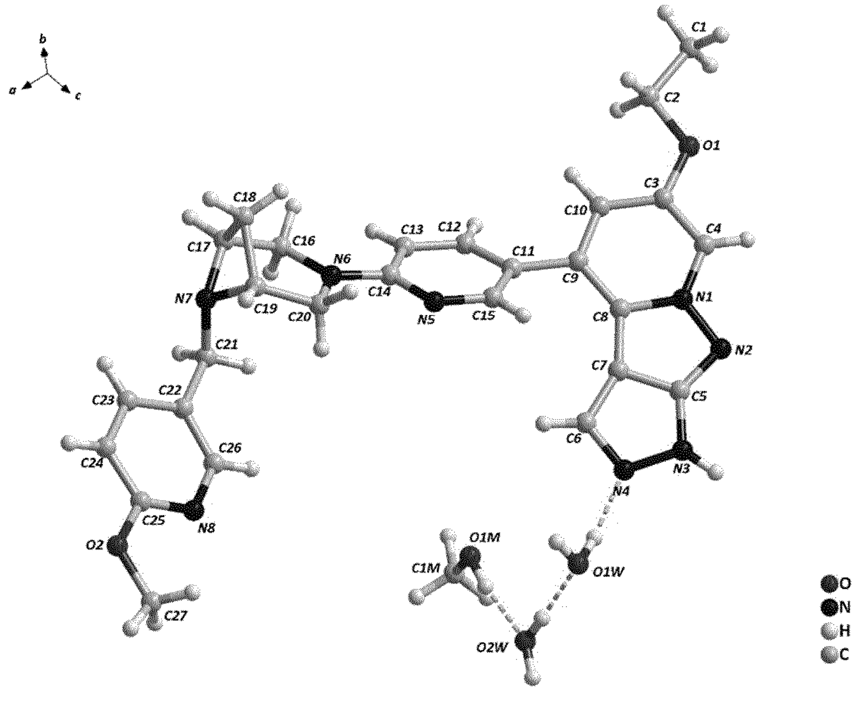

**FIG. 12D**

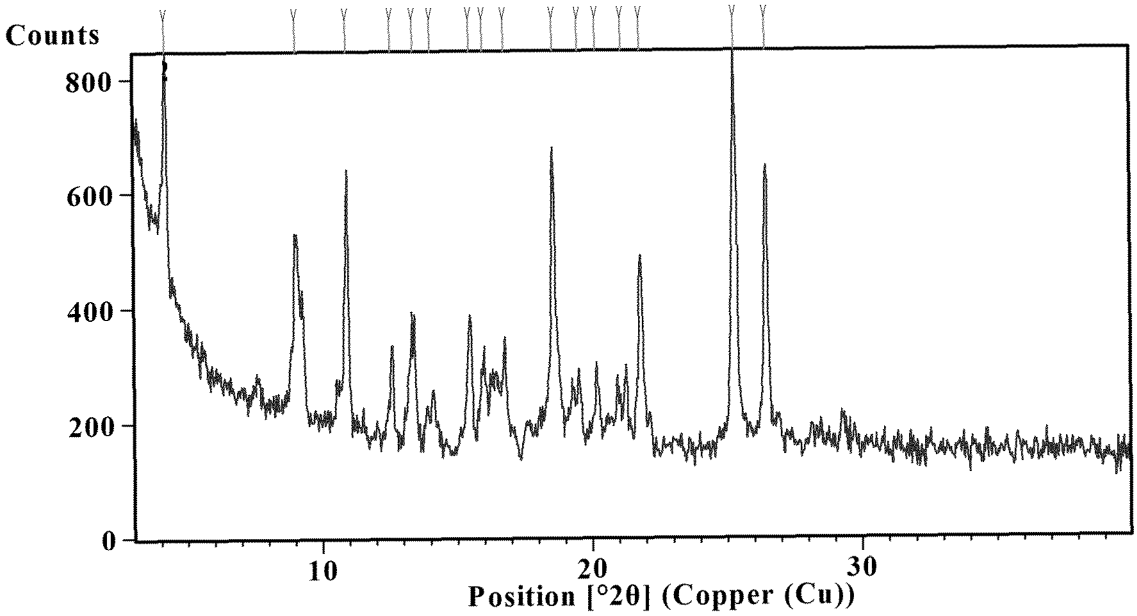


FIG. 13A

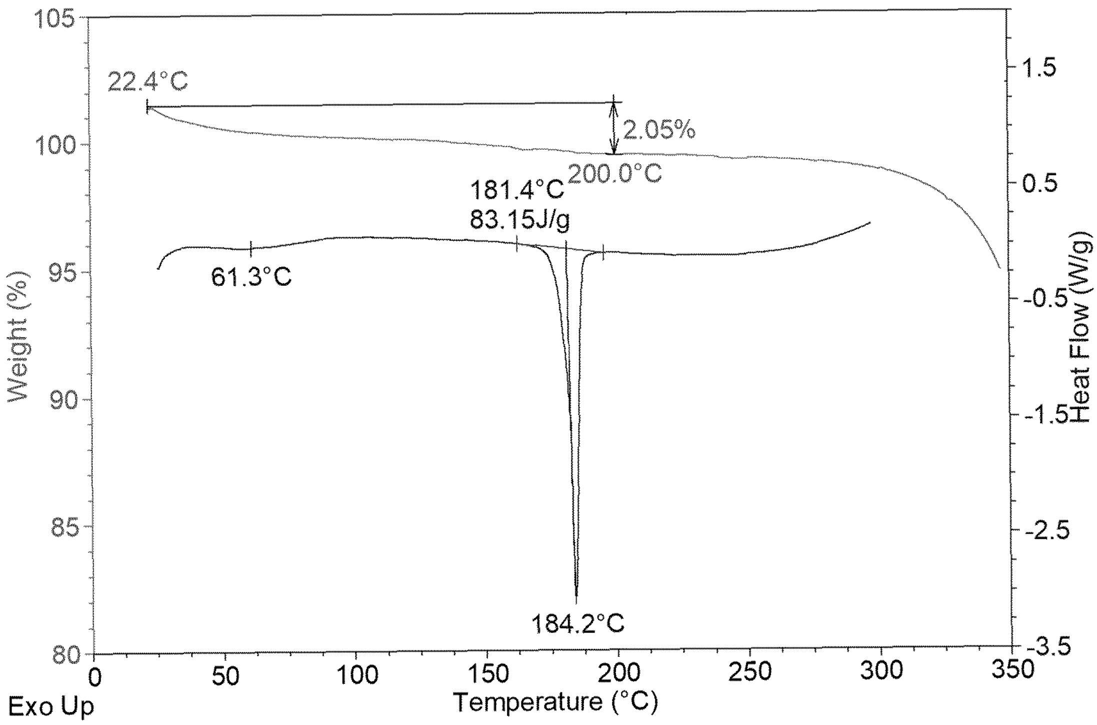


FIG. 13B

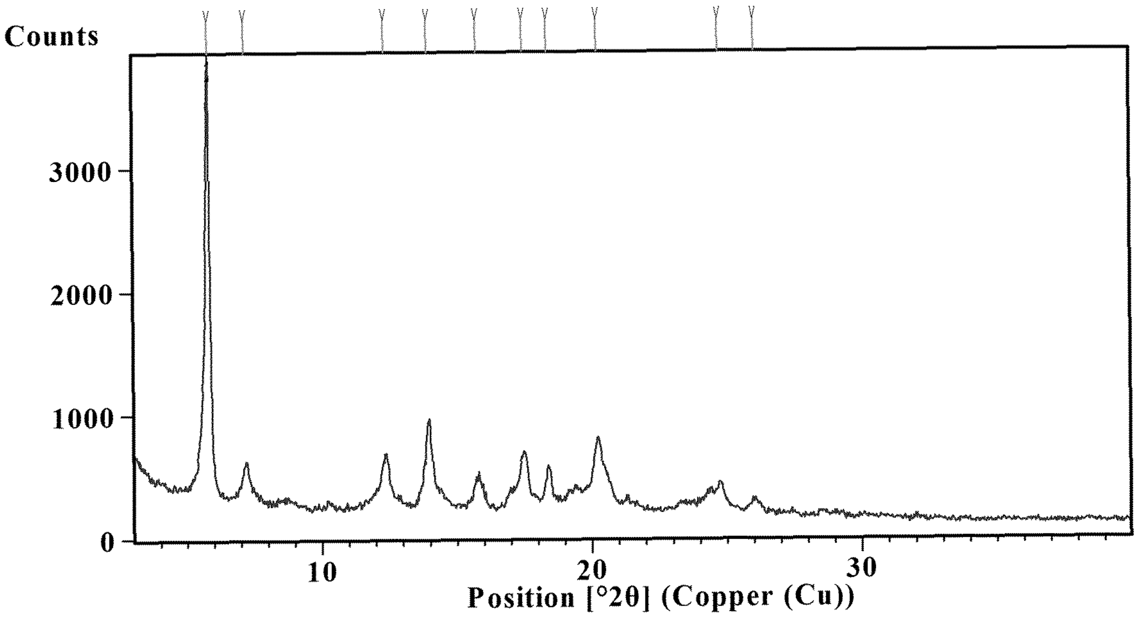


FIG. 14A

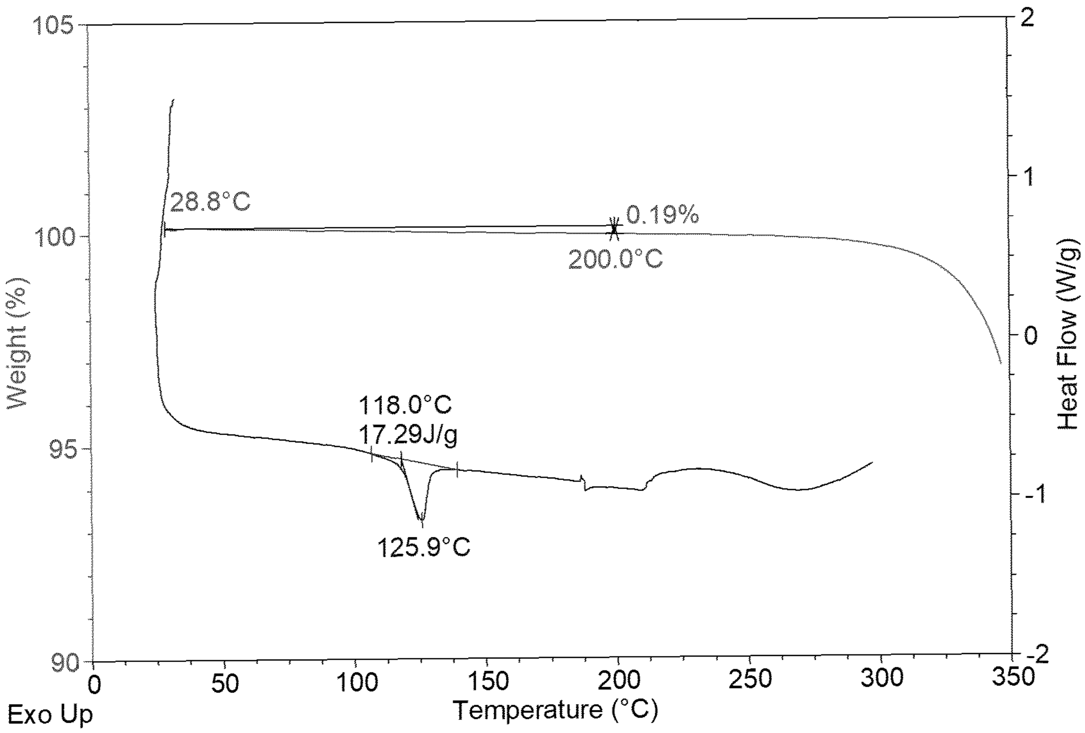


FIG. 14B

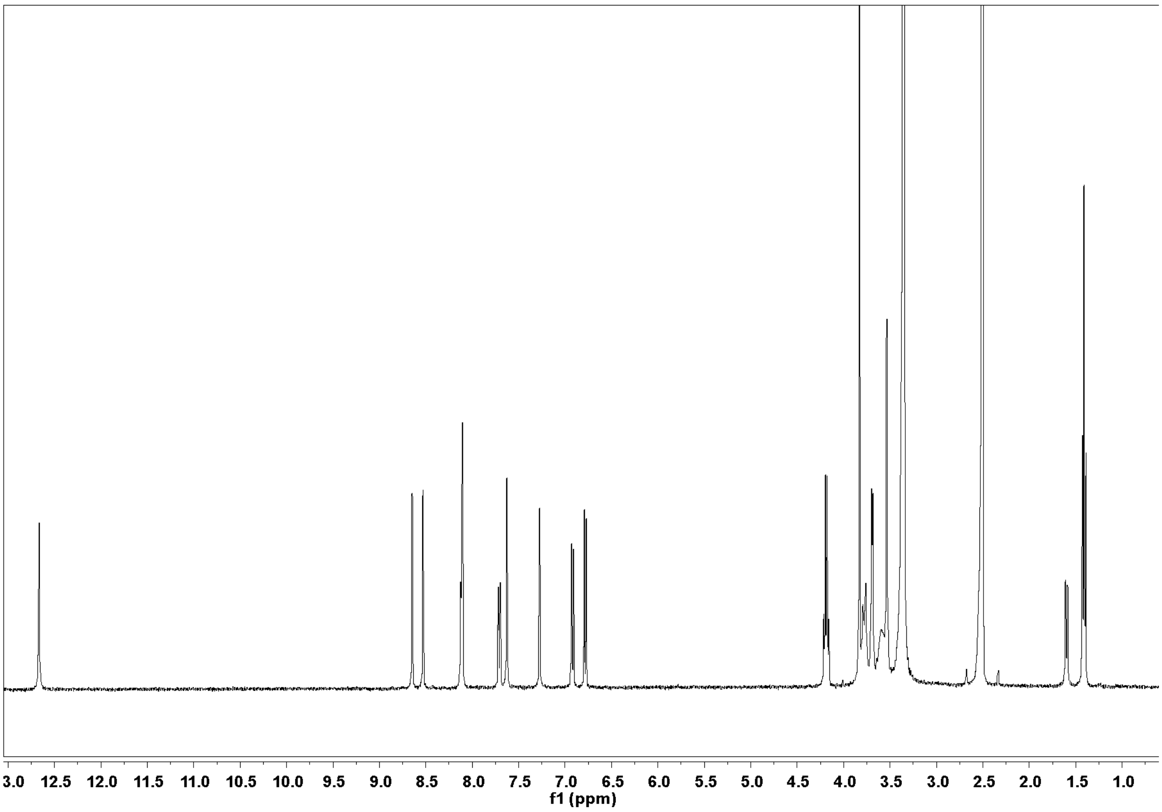


**FIG. 14C**

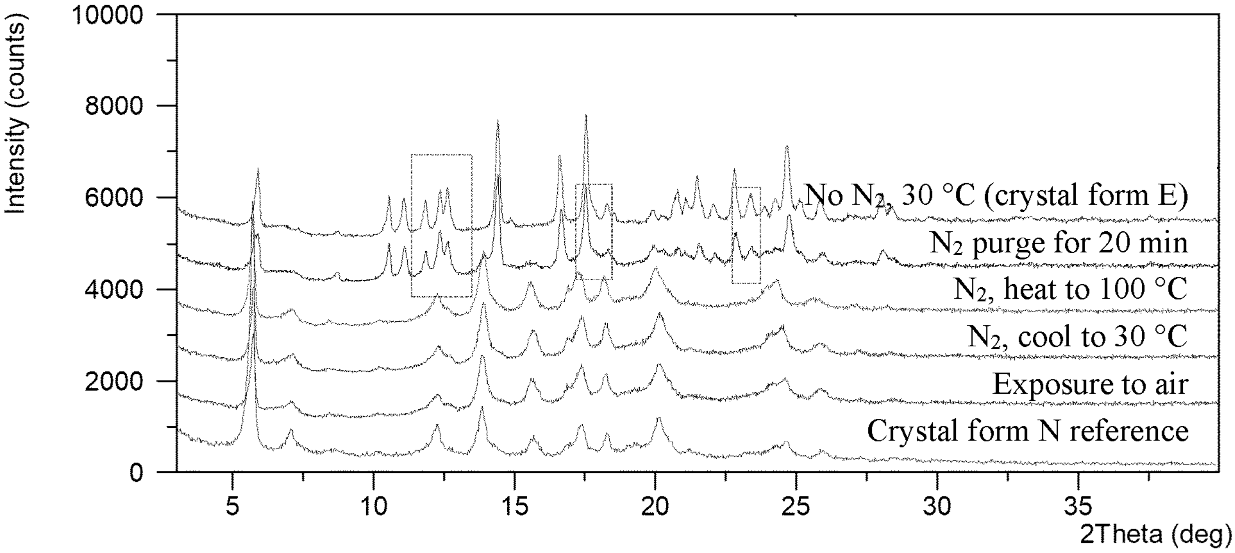


**FIG. 14D**

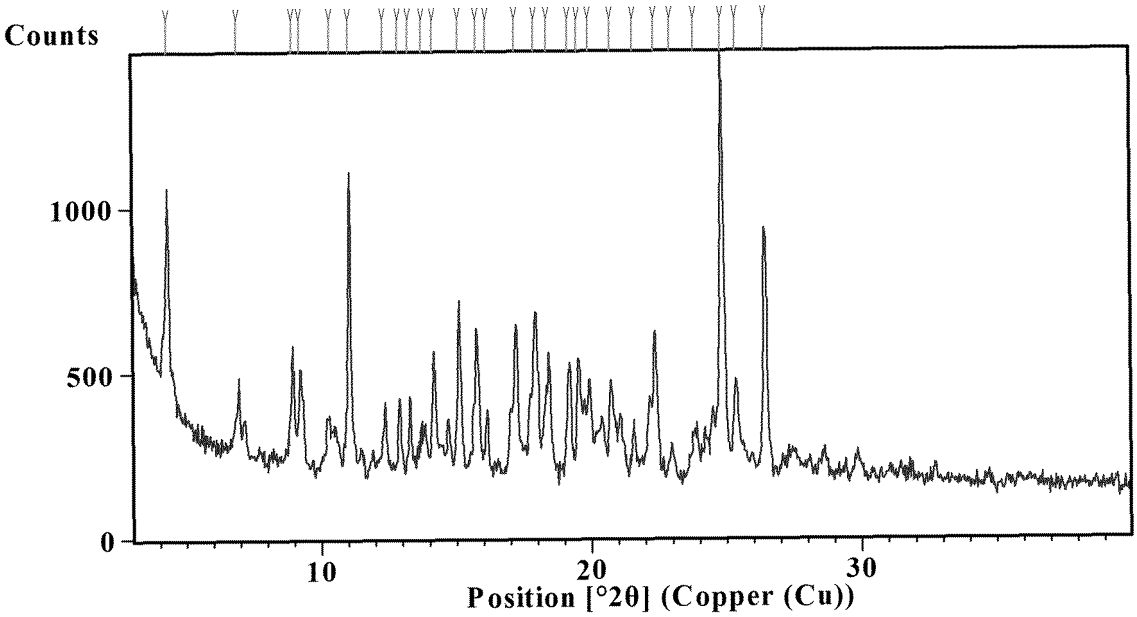


FIG. 15A

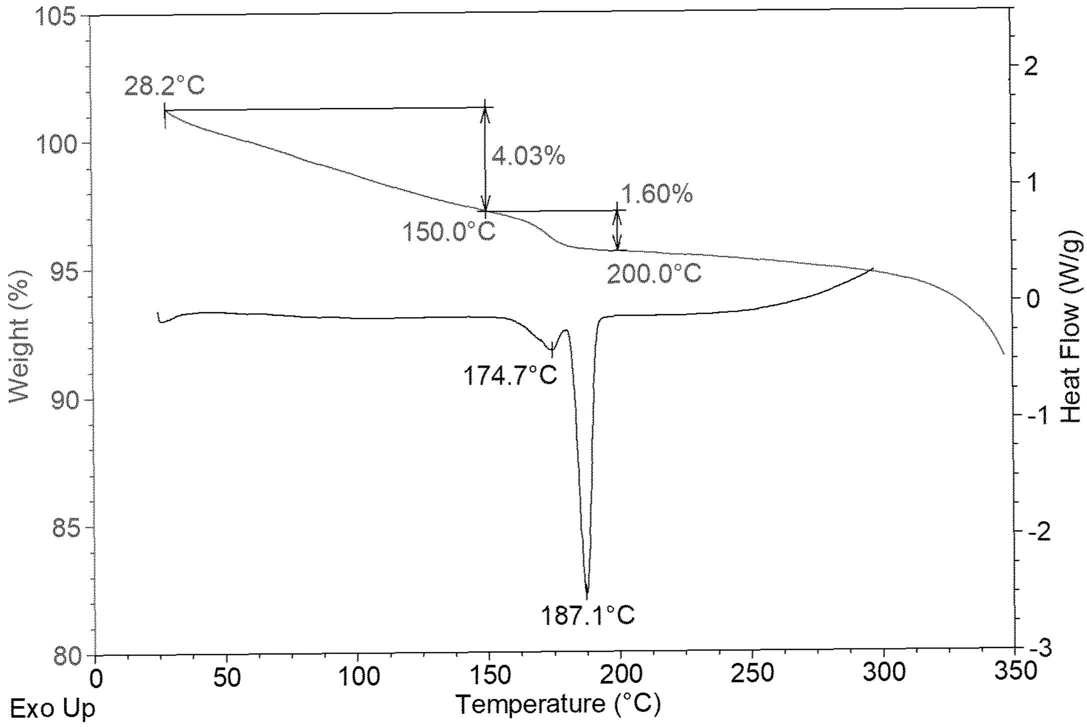


FIG. 15B

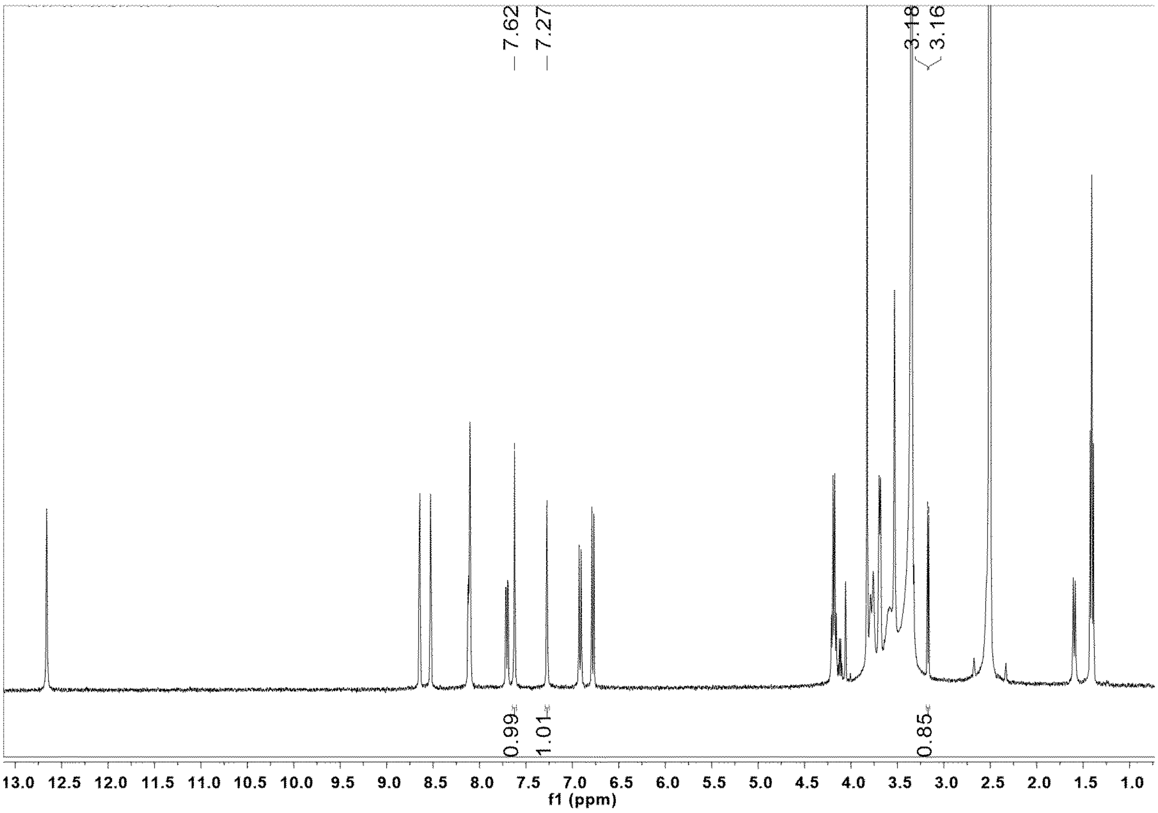


**FIG. 15C**

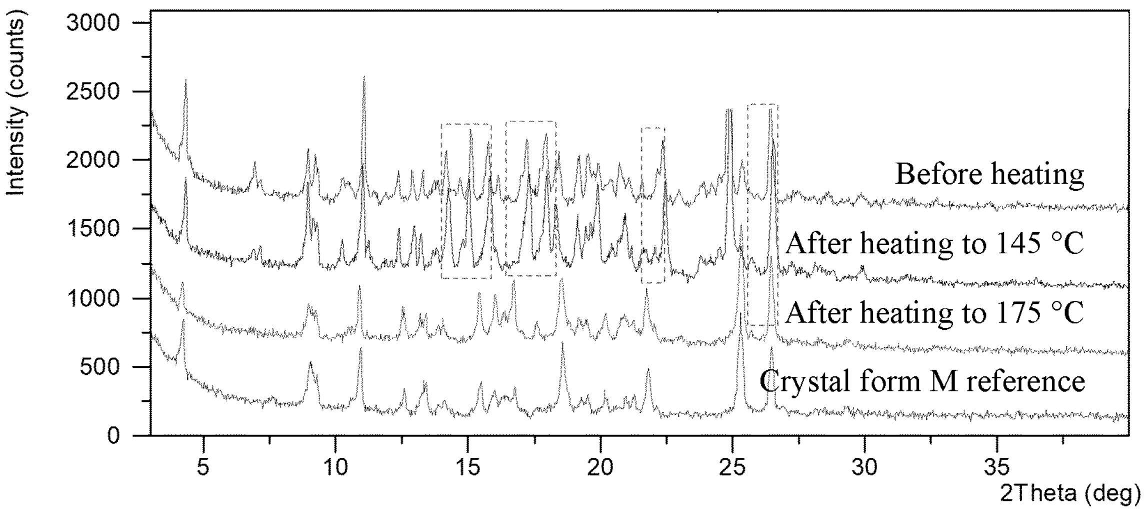


**FIG. 15D**

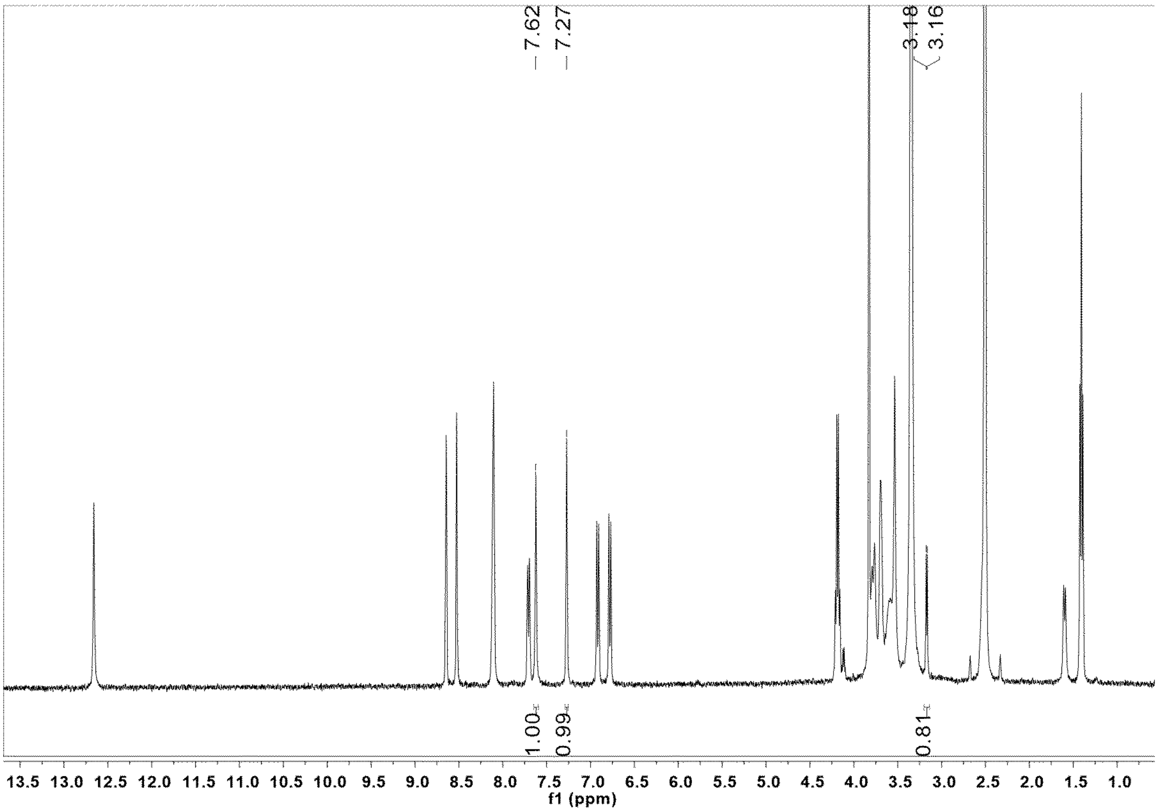


FIG. 15E

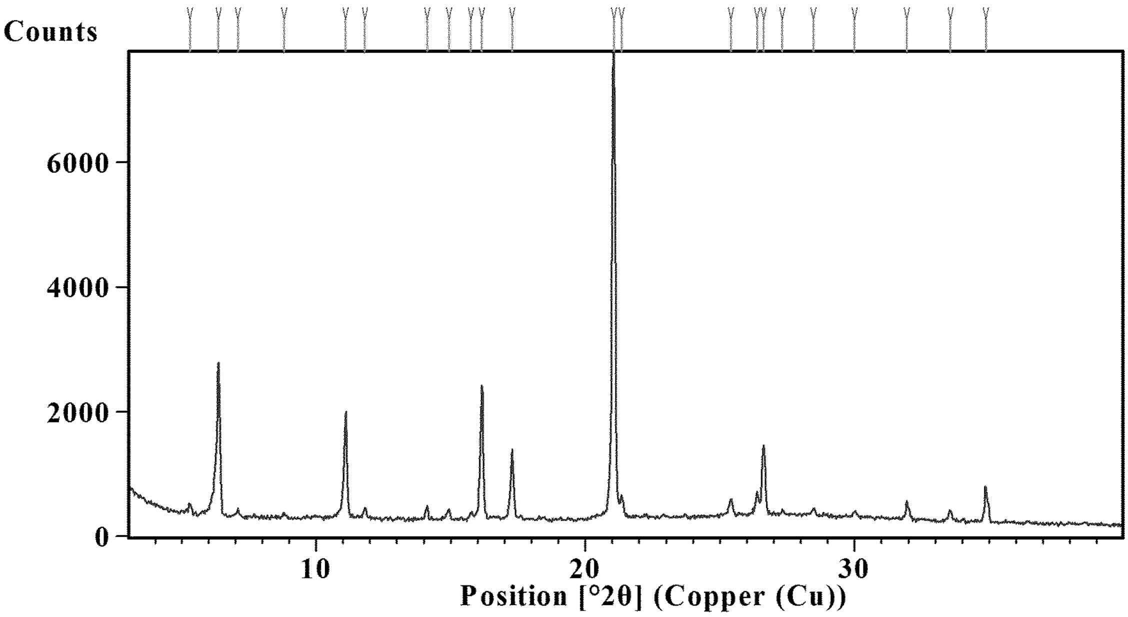


FIG. 16A

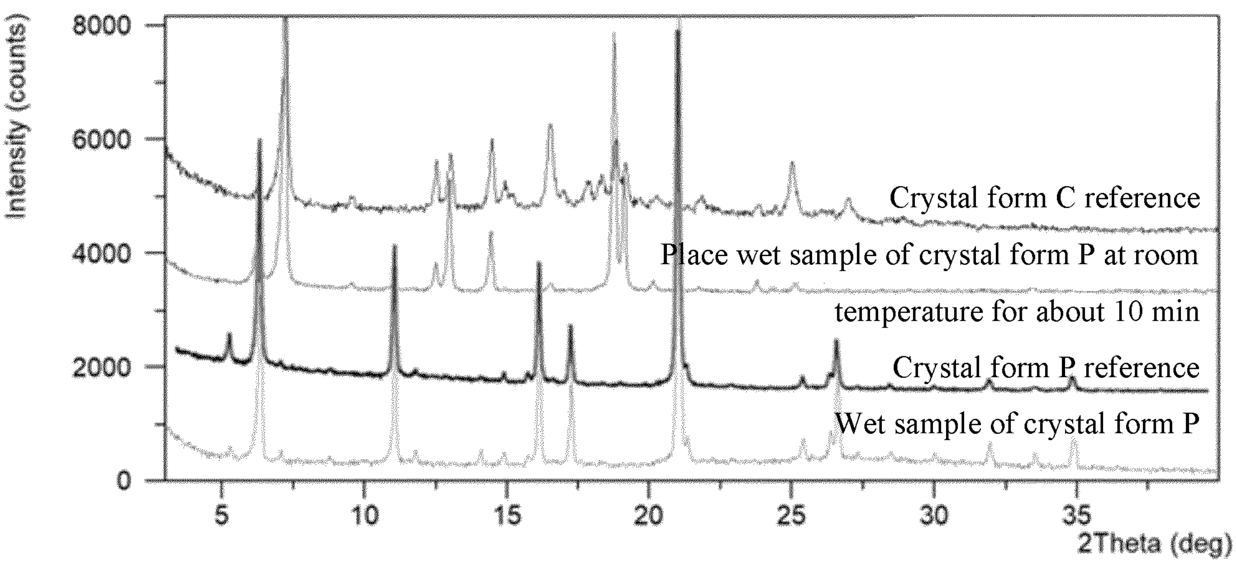


FIG. 16B

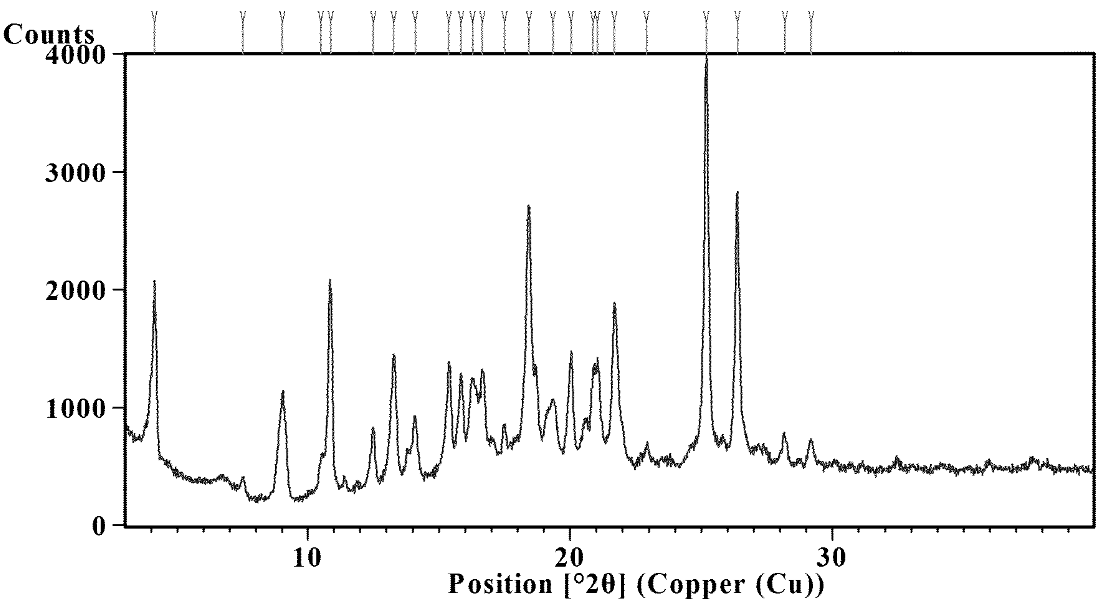


FIG. 17

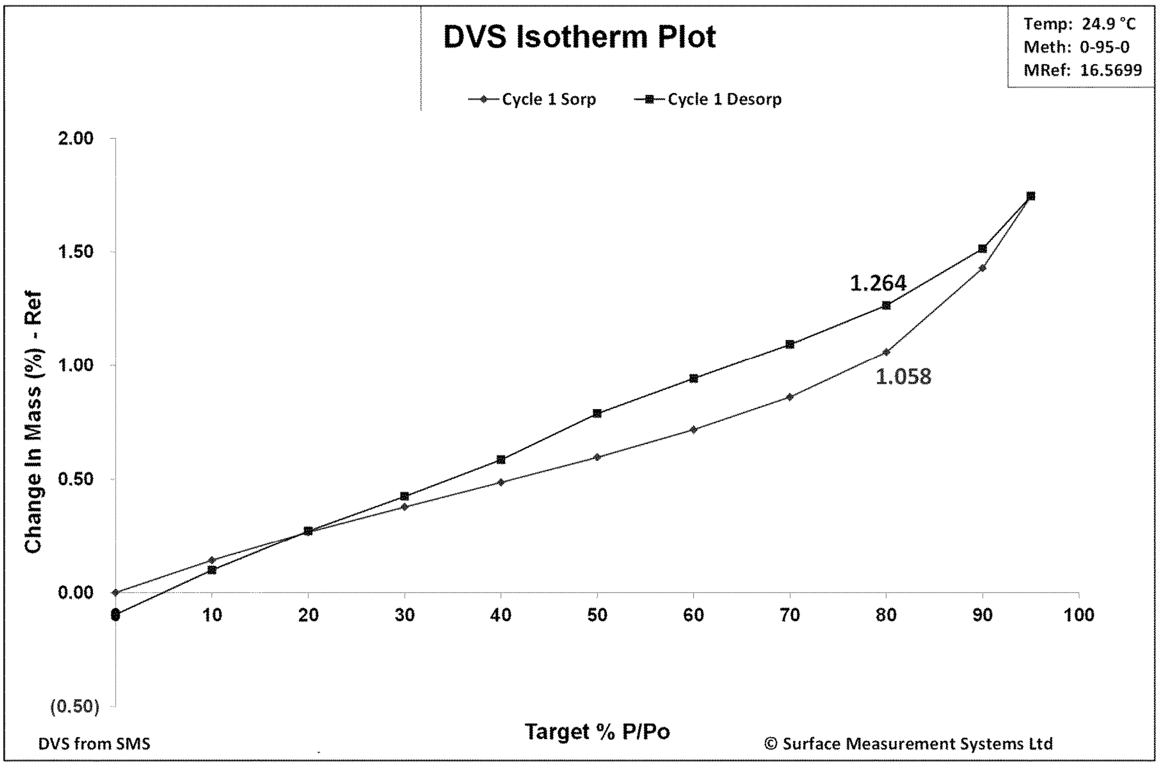
DVS Isotherm Plot
Temp: 24.9 °C
Meth: 0-95-0
MRef: 16.5699
Cycle 1 Sorp
Cycle 1 Desorp
2.00
1.50
1.00
0.50
0.00
(0.50)
Change In Mass (%) - Ref
10
20
30
40
50
60
70
80
90
100
1.264
1.058
Target % P/Po
DVS from SMS
© Surface Measurement Systems Ltd

FIG. 18

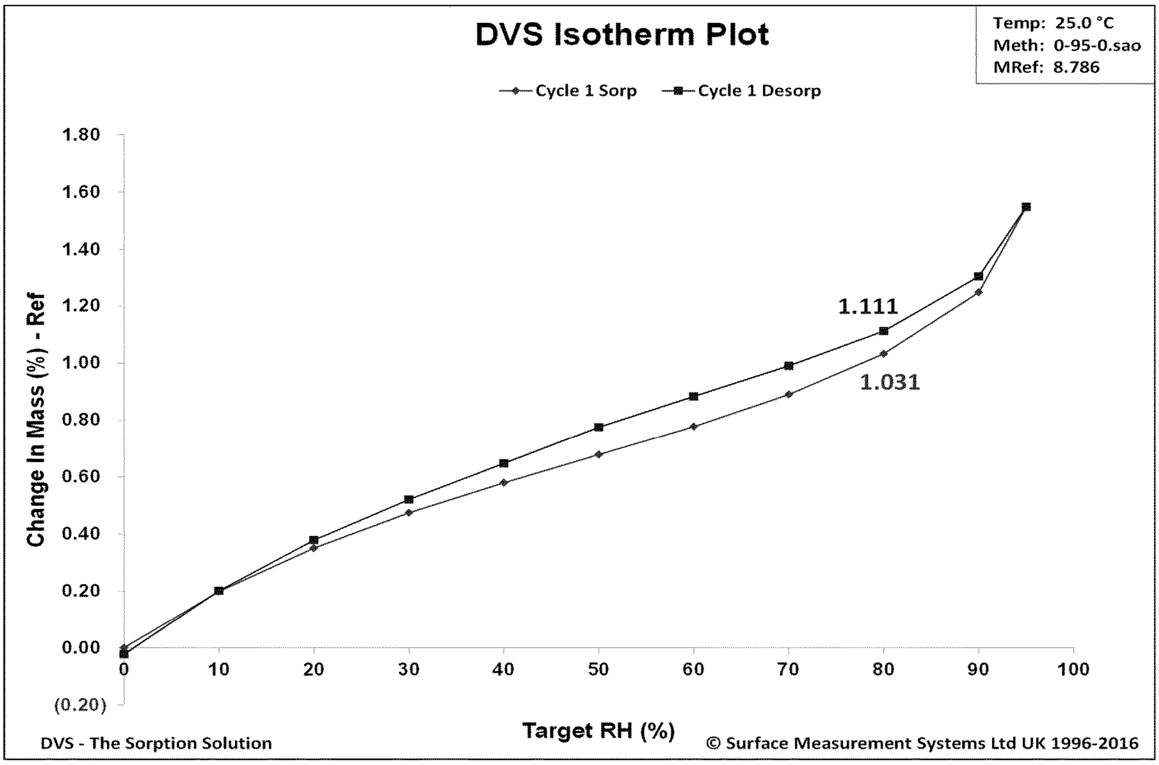
DVS Isotherm Plot
Temp: 25.0 °C
Meth: 0-95-0.sao
MRef: 8.786
Cycle 1 Sorp
Cycle 1 Desorp
1.80
1.60
1.40
1.20
1.00
0.80
0.60
0.40
0.20
0.00
(0.20)
Change In Mass (%) - Ref
0
10
20
30
40
50
60
70
80
90
100
1.111
1.031
Target RH (%)
DVS - The Sorption Solution
© Surface Measurement Systems Ltd UK 1996-2016

FIG. 19

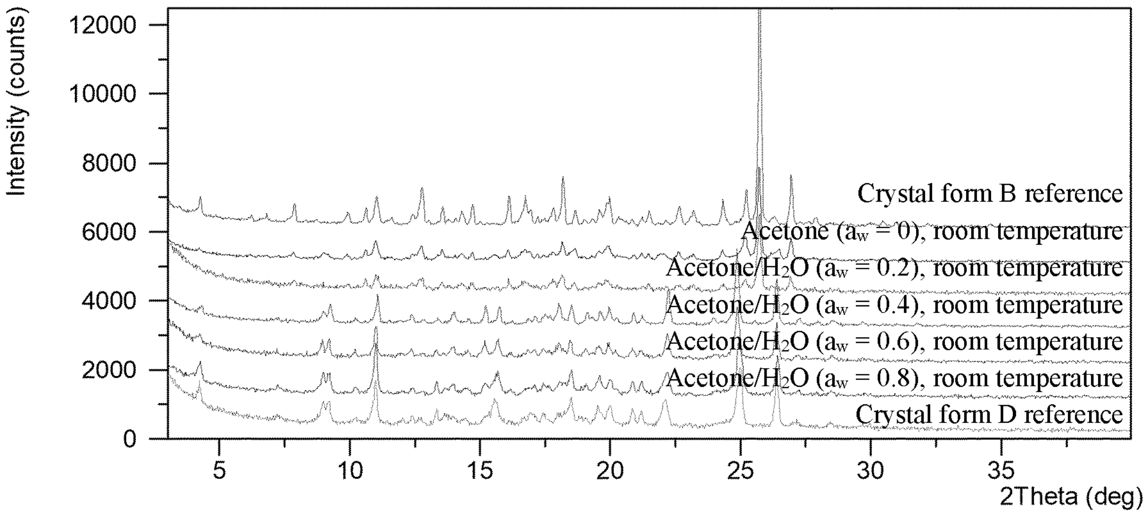


**FIG. 20A**

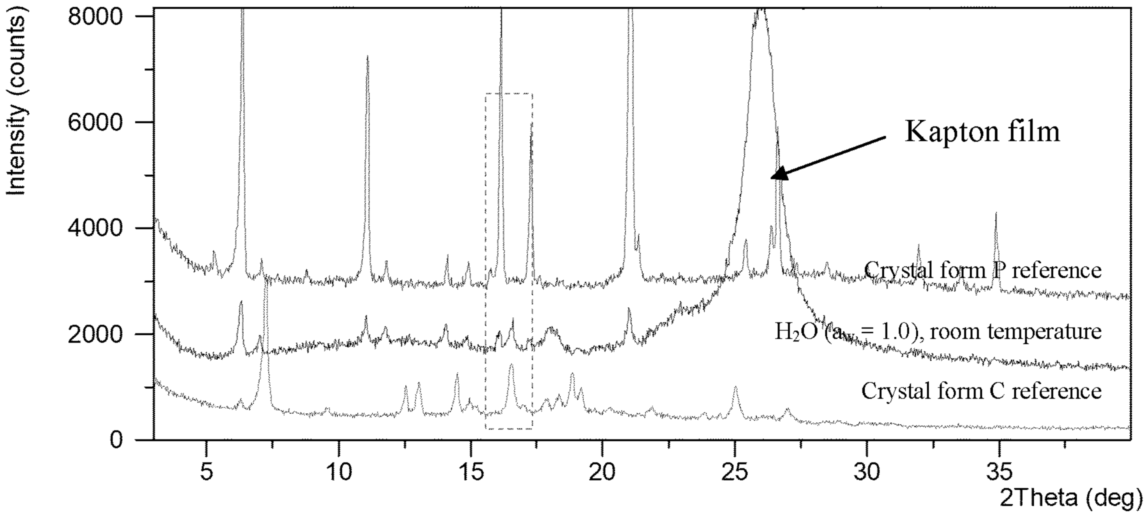


**FIG. 20B**

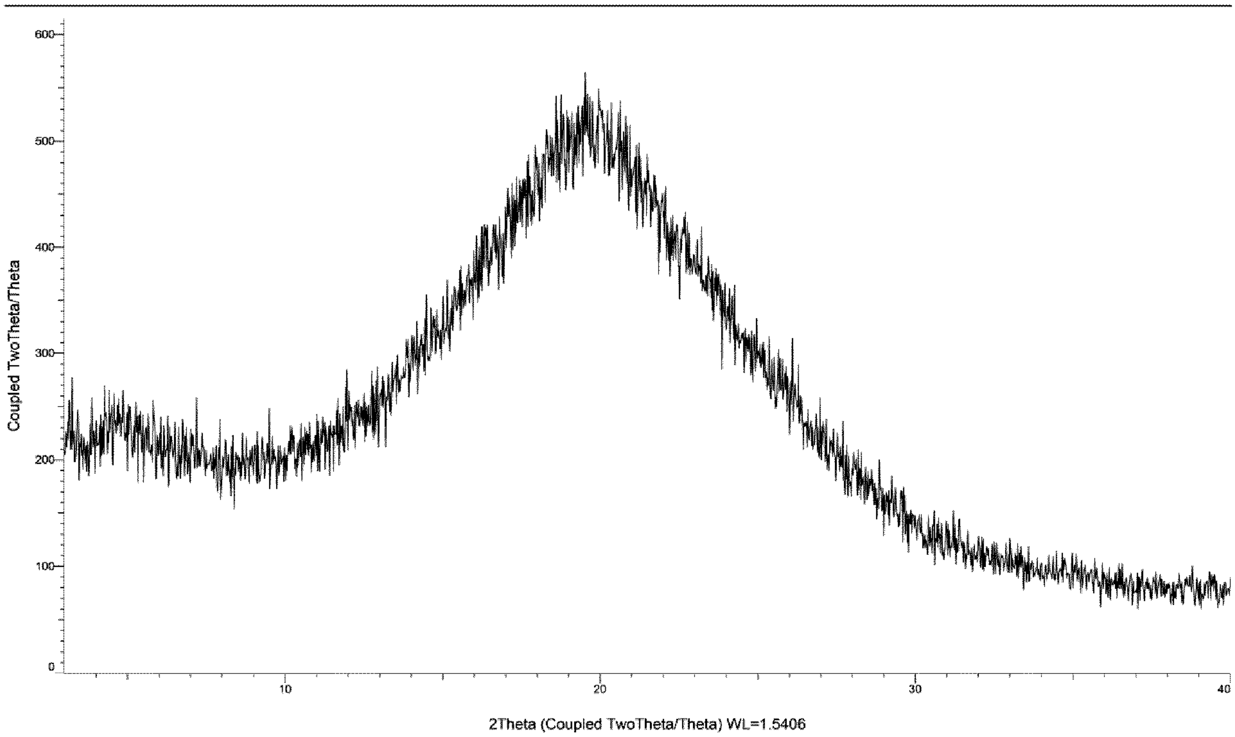


**FIG. 21A**

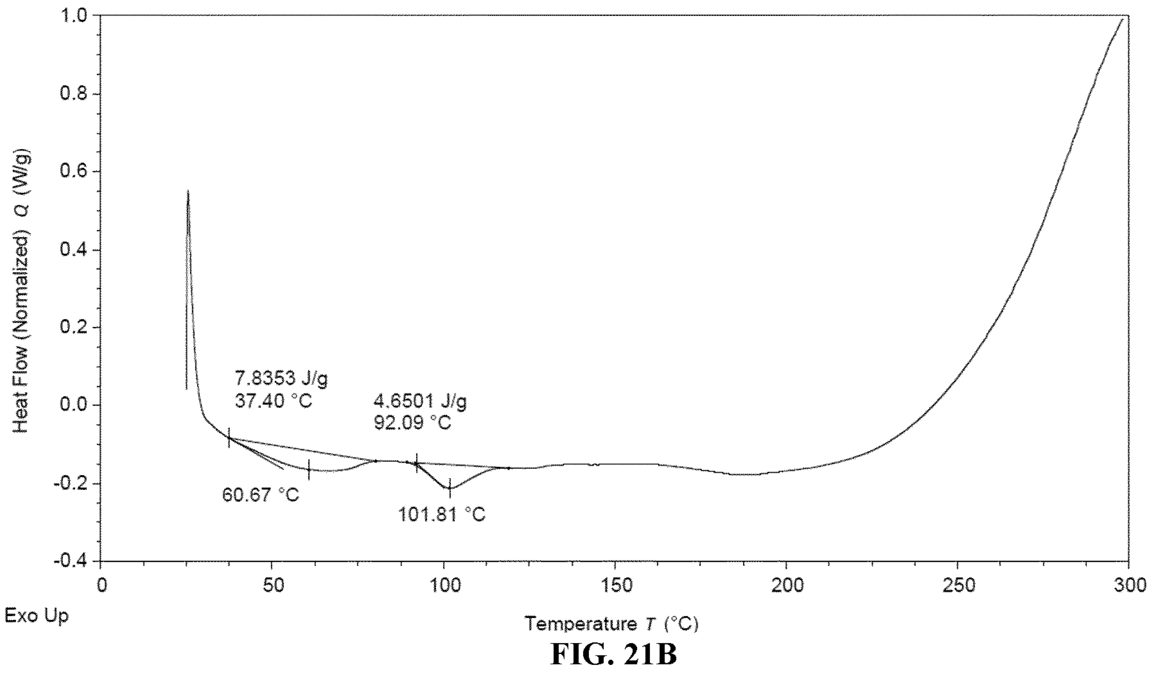


**FIG. 21B**

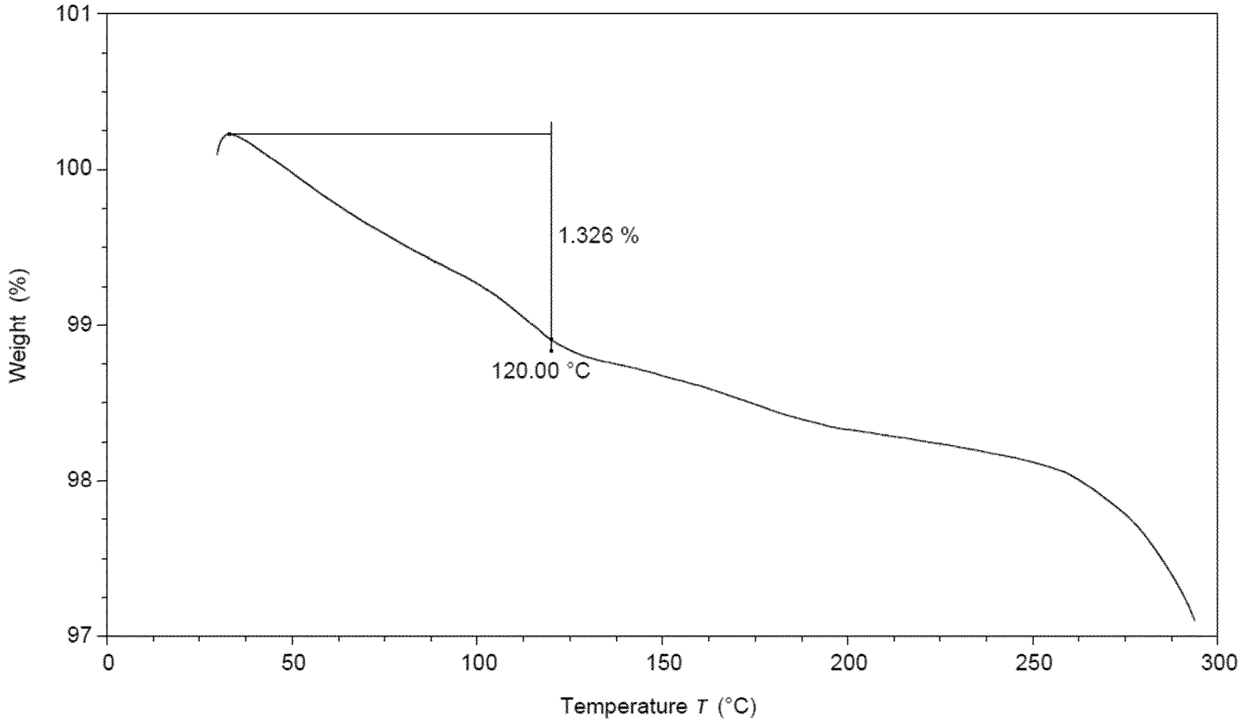


FIG. 21C

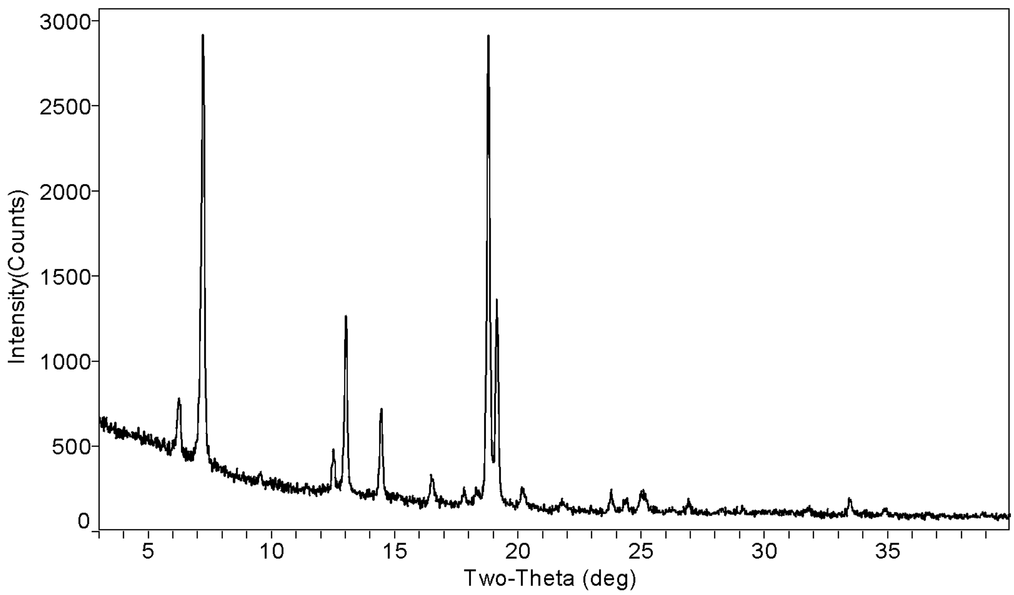


FIG. 22A

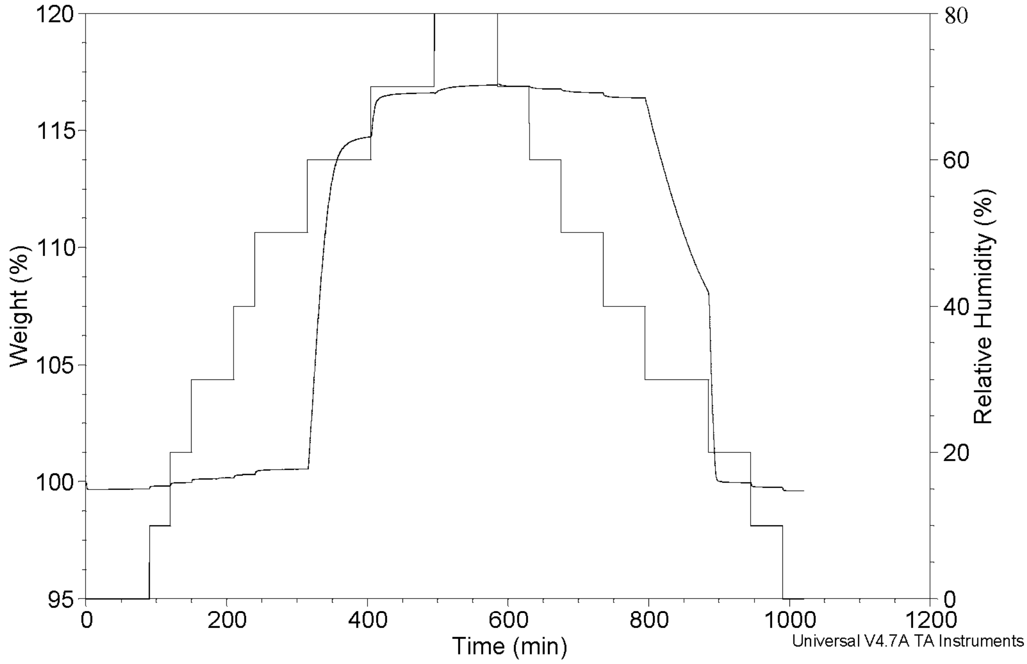


FIG. 22B

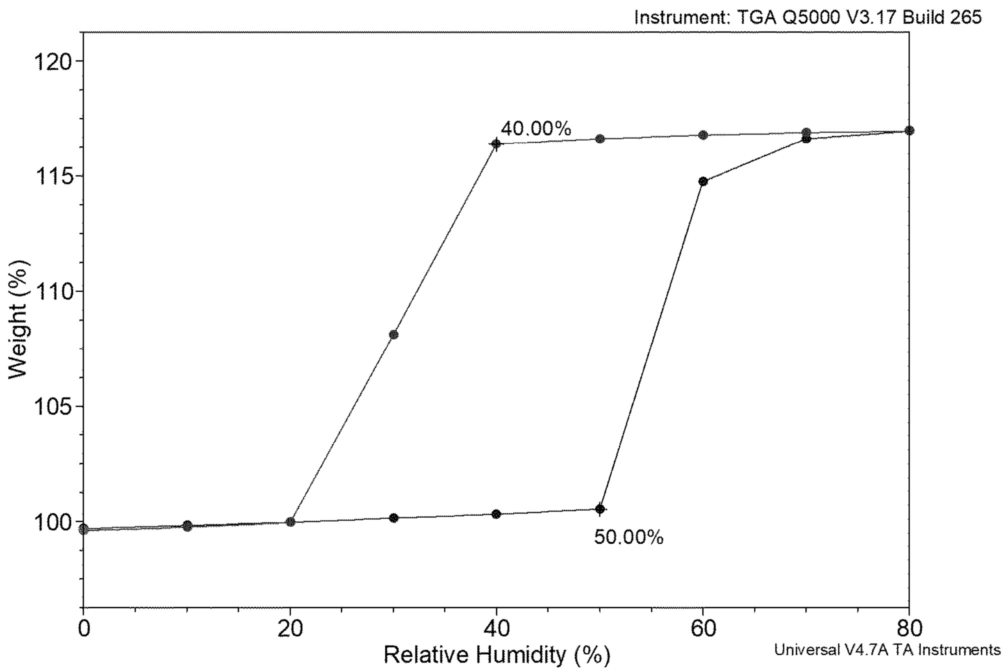


FIG. 22C

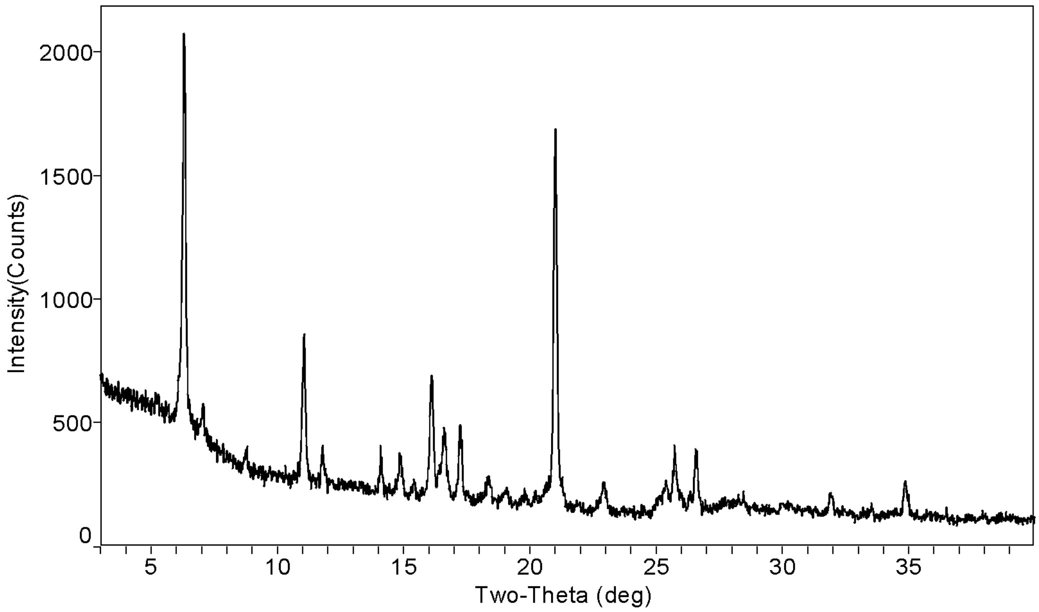


FIG. 23

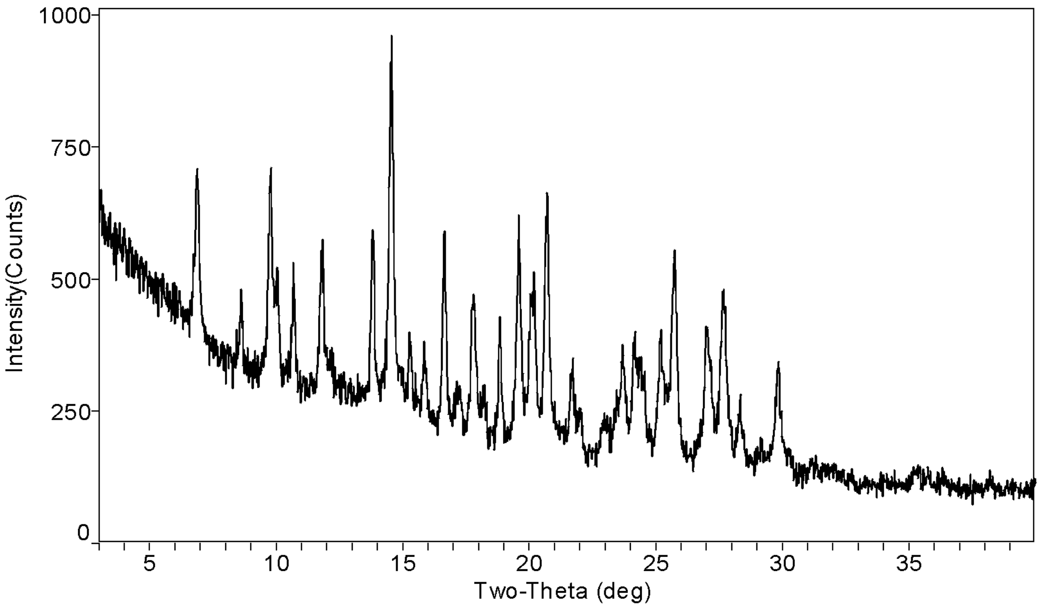


FIG. 24A

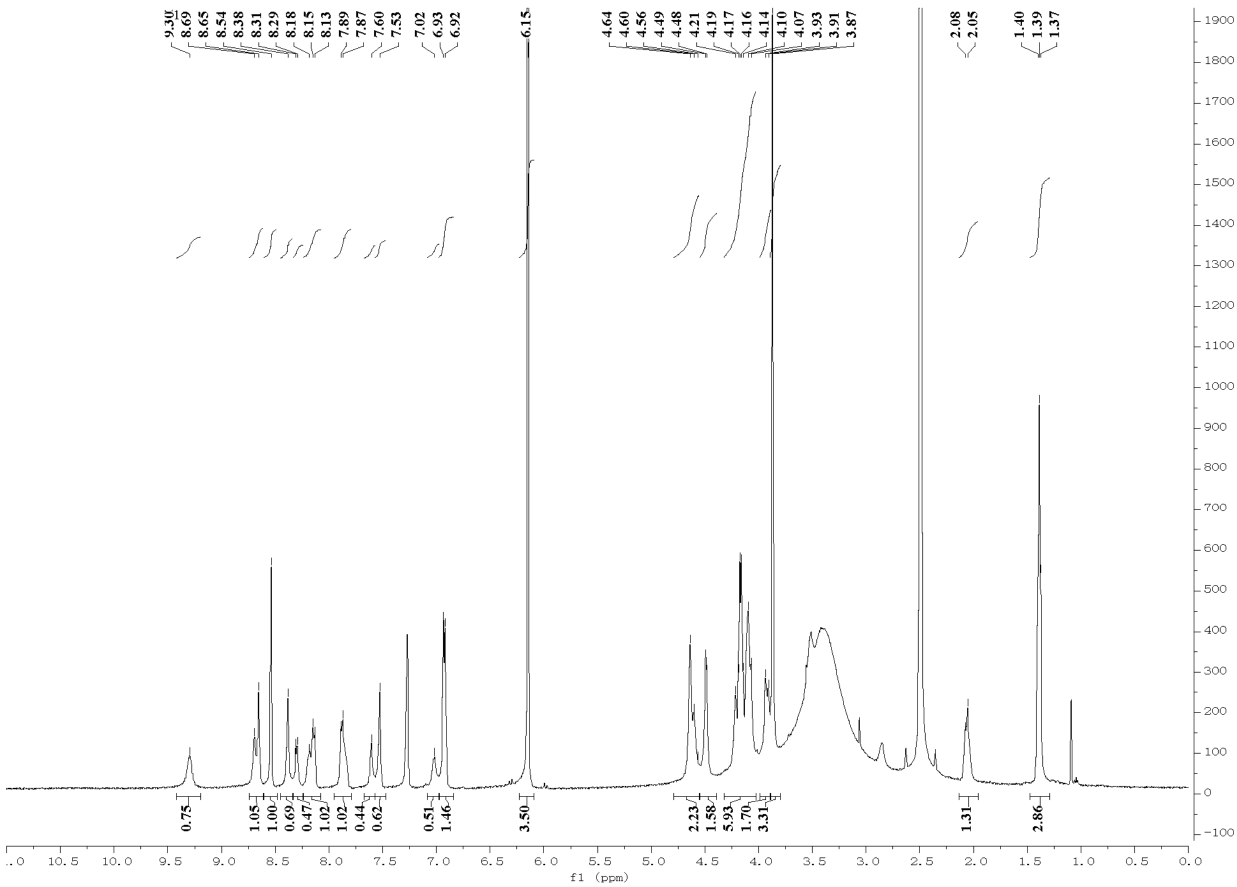


FIG. 24B

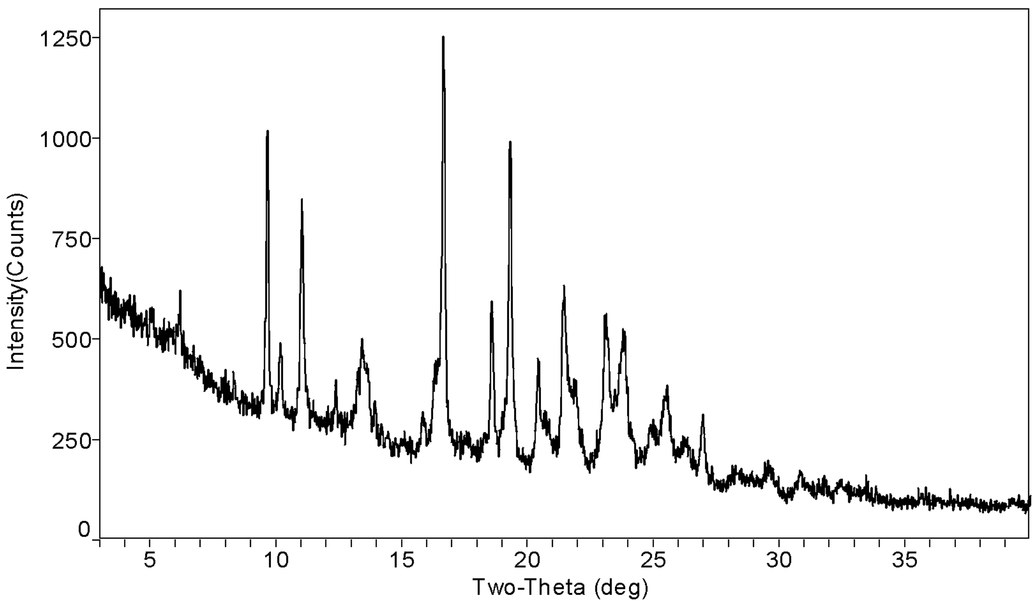


FIG. 25A

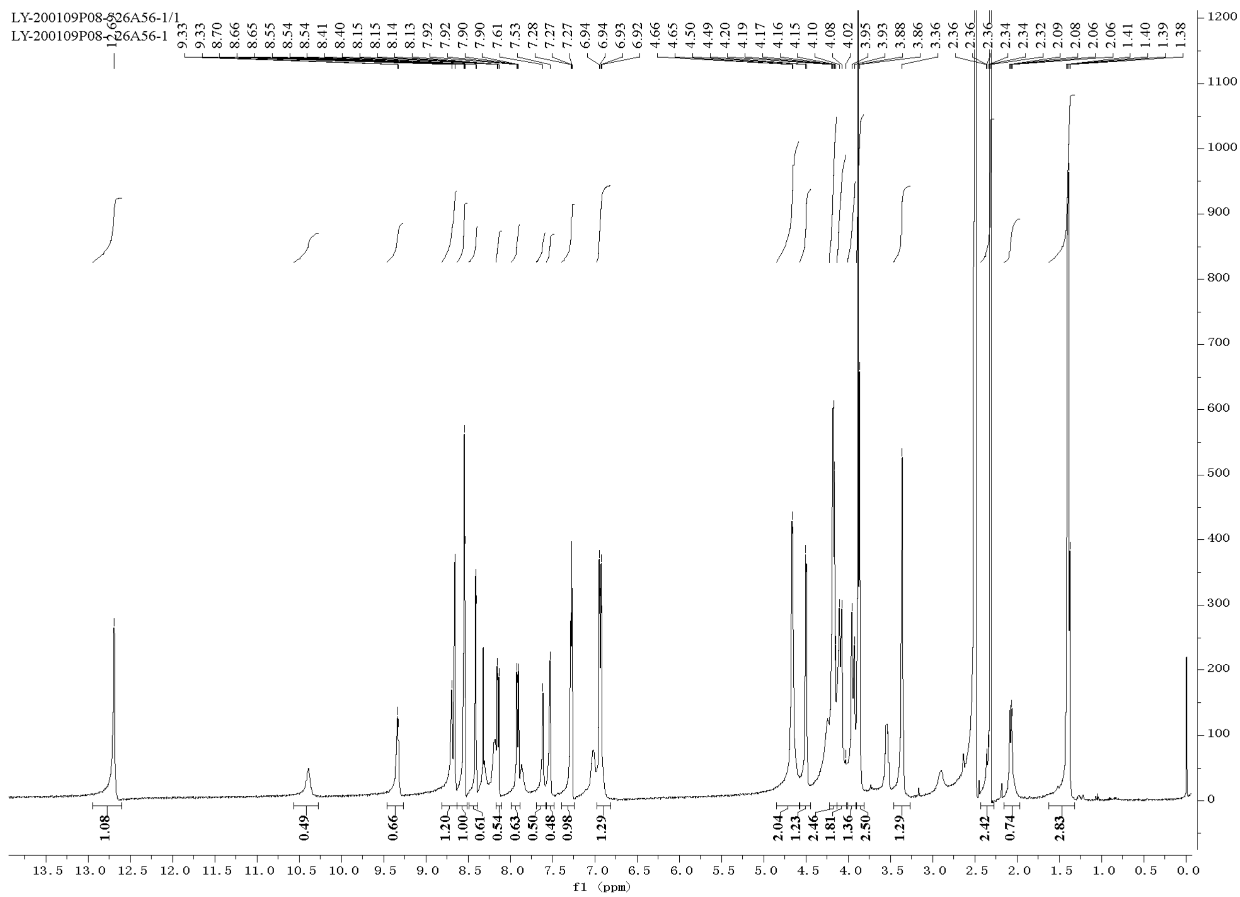
LY-200109P08-526A56-1/1
LY-200109P08-126A56-1
12.66
9.33 9.33 8.70 8.66 8.65 8.55 8.54 8.54 8.41 8.40 8.15 8.15 8.14 8.13 7.92 7.92 7.90 7.90 7.61 7.53 7.28 7.27 7.27 6.94 6.94 6.93 6.92 4.66 4.65 4.50 4.49 4.20 4.19 4.17 4.16 4.15 4.10 4.08 4.02 3.95 3.93 3.88 3.86 3.36 2.36 2.36 2.36 2.34 2.34 2.32 2.09 2.08 2.06 1.41 1.40 1.39 1.38
1.08 0.49 0.66 1.20 1.00 0.61 0.54 0.63 0.50 0.48 0.98 1.29 2.04 1.23 2.46 1.81 1.36 2.50 1.29 2.42 0.74 2.83
f1 (ppm)

FIG. 25B

**INTERNATIONAL SEARCH REPORT**

International application No. **PCT/CN2023/133860**

**A. CLASSIFICATION OF SUBJECT MATTER**

C07D 519/00(2006.01)i; A61K31/444(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNABS DWPI CNTXT ENTXTC STN: RET, 肿瘤, 癌症, 晶型, 志健金瑞, tumor, cancer, crysta+, STN结构式检索, search for structural formula in STN, CAS: 2598870-24-5

**C. DOCUMENTS CONSIDERED TO BE RELEVANT**

| Category* | Citation of document, with indication, where appropriate, of the relevant passages | Relevant to claim No. |
|---|---|---|
| PX | CN 115594678 A (APPLIED PHARMACEUTICAL SCIENCE, INC.) 13 January 2023 (2023-01-13)<br>description, paragraphs [0001]-[0004], and embodiment 3 | 1-12 |
| PX | WO 2023280073 A1 (APPLIED PHARMACEUTICAL SCIENCE, INC.) 12 January 2023 (2023-01-12)<br>description, page 1, embodiment 3 | 1-12 |
| X | WO 2021023209 A1 (APPLIED PHARMACEUTICAL SCIENCE, INC.) 11 February 2021 (2021-02-11)<br>embodiment 9, and claims 98, 101 and 106-107 | 1-12 |
| X | WO 2022166642 A1 (APPLIED PHARMACEUTICAL SCIENCE, INC.) 11 August 2022 (2022-08-11)<br>claims 1, 4 and 9-10 | 1-12 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **26 February 2024** | **01 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/133860**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **12**
because they relate to subject matter not required to be searched by this Authority, namely:
The technical solution of claim 12 relates to a method for treating diseases. This report is provided on the basis of a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/133860**

| Patent document cited in search report | | | Publication date (day/month/year) | Patent family member(s) | | | Publication date (day/month/year) |
|---|---|---|---|---|---|---|---|
| CN | 115594678 | A | 13 January 2023 | None | | | |
| WO | 2023280073 | A1 | 12 January 2023 | TW | 202317569 | A | 01 May 2023 |
| WO | 2021023209 | A1 | 11 February 2021 | None | | | |
| WO | 2022166642 | A1 | 11 August 2022 | KR | 20230136766 | A | 26 September 2023 |
| | | | | TW | 202237607 | A | 01 October 2022 |
| | | | | EP | 4289428 | A1 | 13 December 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202211494114
- CN 2020107049 W


### Non-patent literature cited in the description


- *Cell*, 1985, vol. 42 (2), 581-588
- *Mol Cell Endocrinol*, 2010, vol. 322 (1-2), 2-7
- *J Clin Oncol*, 2012, vol. 30 (2), 200-202
- *Journal of Thoracic Oncology*, 2020, vol. 15 (4), 541-549